# EUROPEAN PATENT APPLICATION

(11) **EP 2 684 468 A1**
(43) Date of publication of application: **15.01.2014**
(21) Application number: 12755389.9
(22) Date of filing: 07.03.2012
(51) Int. Cl.: A23L 1/226

(54) **SALTY TASTE ENHANCER**

(30) Priority: 07.03.2011 JP 2011049469
(71) Applicant: Ajinomoto Co., Inc., Tokyo 104-8315 (JP)
(72) Inventor: TAHARA, Yuki, Kawasaki-shi Kanagawa 210-8681 (JP); AMINO, Yusuke, Kawasaki-shi Kanagawa 210-8681 (JP); MAEKAWA, Takami, Kawasaki-shi Kanagawa 210-8681 (JP); ETO, Yuzuru, Kawasaki-shi Kanagawa 210-8681 (JP); MIYAKI, Takashi, Kawasaki-shi Kanagawa 210-8681 (JP); SAIKAWA, Wakana, Kawasaki-shi Kanagawa 210-8681 (JP); KAI, Yuko, Kawasaki-shi Kanagawa 210-8681 (JP); ISHIWATARI, Yutaka, Kawasaki-shi Kanagawa 210-8681 (JP)
(74) Representative: Nicholls, Kathryn Margaret
(86) International application number: PCT/JP2012/055761
(87) International publication number: WO 2012/121273

(57) **Abstract**

The present invention provides a compound having a salty taste enhance activity, and a salty taste enhancer containing the compound, and the like.

The present invention relates to a salty taste enhancer for a food or drink, which contains a compound represented by the following formula: 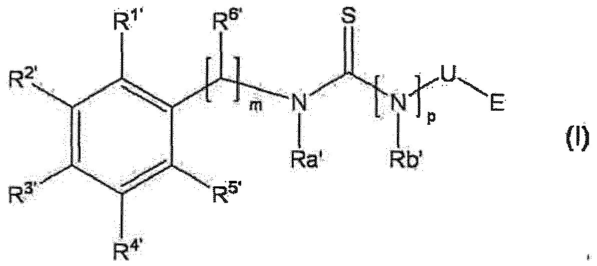 wherein each symbol is as defined in the specification, or an edible salt thereof.

## Description

### Technical Field

The present invention relates to a salty taste enhancer useful as a food material, and a novel compound having a salty taste enhancing effect. Moreover, the present invention relates to a salty taste enhancer, or a salty taste enhancer used for a food or drink, which comprises the compound, and the like.

### Background Art

Excess intake of sodium chloride is one cause of elevation of blood pressure, and is considered to cause cerebral apoplexy and heart disease. To prevent this, reduction of the amount of intake of sodium chloride has been recommended. However, reduced-salt foods using reduced amount of sodium chloride taste plain and markedly reduce taste quality. To improve plain taste due to the reduction of salt, a method including addition of sodium glutamate and spice is known (e.g., non-patent document 1). While sodium glutamate and spice enhance the flavor, they are not sufficient for the effect of enhancing the salty taste itself.

Furthermore, a method of replacing a part of sodium chloride with a sodium chloride alternative such as potassium salt, ammonium salt, basic amino acid, salty taste peptide and the like, has also been reported. Examples thereof include a method of reducing a bitter taste of potassium chloride by using potassium chloride and carrageenan in combination (e.g., patent document 1), a production method of potassium chloride-containing fermentation food (e.g., patent document 2) and the like. Moreover, a substance free of a sodium chloride taste in itself but potentiating a salt taste when co-used with sodium chloride is also known. For example, a method of adding a saturated aliphatic monocarboxylic acid having 3 to 8 carbon atoms to a sodium chloride-containing food or drink at a proportion of 0.01 to 1 wt% based on the weight of sodium chloride and the like is known (e.g., patent document 3). However, they are not satisfactory methods in terms of strength of the salty taste and taste quality.

Therefore, a reduced-salt food superior in taste property, a salty taste enhancer which is superior in the taste quality or strength, or further, a novel compound capable of enhancing the salty taste is still demanded.

On the other hand, various studies of the receptive mechanism of salty taste have been undertaken, though many aspects remain to be clarified.

ENaC (epithelial sodium channel) is a voltage-independent, amiloride-sensitive sodium channel present in the cellular membrane, which is an ion channel that functions when 3 kinds of subunits (α or 5 subunit, β subunit and γ subunit) are bonded. ENaC is known as an influx pathway of sodium ion in many epithelial tissues (non-patent documents 2, 3).

ENaC is one of the proteins particularly studied in relation to the salty taste. Nevertheless, the involvement thereof in the salty taste acceptance of human has not been clarified as yet. While the involvement thereof in the salty taste acceptance of rodents has been acknowledged (non-patent document 4), there are negative opinions on that in the salty taste acceptance of human. In fact, while non-patent document 5 describes that S3969 [N-(2-hydroxyethyl)-4-methyl-2-(4-methyl-1H-indol-3-ylthio)pentanamide] is a stimulant (activator) of ENaC that acts on β subunit, it merely suggests that a salty taste may have a stimulant action on rodents, and is completely silent on how it is actually tasted by human during eating, even a possibility thereof.

### Capsazepine:

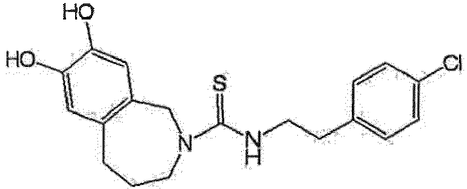

is known as a TRPV1 antagonist, and many thiourea compounds have been synthesized and reported as capsazepine derivatives (e.g., non-patent documents 6 and 7). However, taste property, particularly a salty taste enhancing effect, of these compounds has not been referred to at all.

As for the flavor property of a compound having a thiourea structure, it has long been known that phenylthiocarbamide: 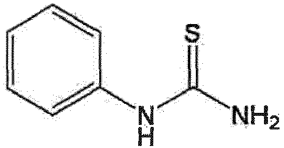 has a strong bitter taste, and the following compound: 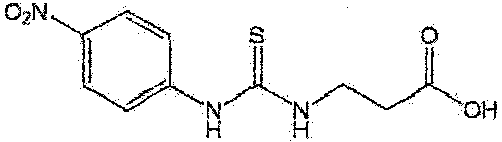 has sweetness (non-patent document 8). Recently, it has been reported that the following structure: 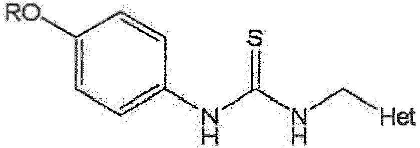 has an agonist activity for T1R2/T1R3 and T1R1/T1R3, which are taste sense receptors (patent document 4). In addition, patent document 5 discloses, as a flavor modulating substance including a salty taste, a compound represented by the following formula: 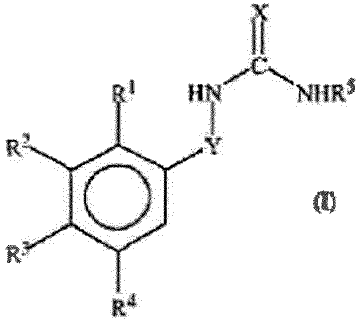 wherein each symbol is as described in patent document 5, and specifically recites N-vanillylthiourea, N,N'-divanillylthiourea, N-octyl-N'-vanillylthiourea, N-hexyl-N'-vanillylthiourea, N-decyl-N'-vanillylthiourea, N-benzyl-N'-vanillylthiourea and the like.

### [Document List]

### [patent documents]

patent document 1: JP-A-4-262758
patent document 2: JP-A-2007-289145
patent document 3: JP-A-5-184326
patent document 4: WO 2006/084184
patent document 5: WO 2007/013811

### [non-patent documents]

non-patent document 1: The Japanese Journal of Taste and Smell Research, vol. 14, No. 3, page 447-450, 2007
non-patent document 2: Palmer LG (1987). "Ion selectivity of epithelial Na channels". J Membr Biol 96: 97-106
non-patent document 3: Lazdunski M, Waldmann R, Champigny G, Bassilana F, Voilley N (1995). "Molecular cloning and functional expression of a novel amiloride-sensitive Na+ channel". J. Biol. Chem. 270 (46): 27411-27414
non-patent document 4: Chandrashekar, J. et al. The cells and peripheral representation of sodium taste in mice. Nature (2010), 464, 297-302
non-patent document 5: Lu, M., et al. Small Molecule Activator of the Human Epithelial Sodium Channel, Journal of Biological Chemistry (2008), 283(18), 11981-11994
non-patent document 6: Harshard K. Rami et al. Bioorganic & Medicinal Chemistry Letters (2004), 14, 3631-3634
non-patent document 7: Michele C. Jetter et al. Bioorganic & Medicinal Chemistry Letters (2004), 14, 3053-3056
non-patent document 8: Shallenberger R. S. "Taste Chemistry" Blackie Academic & Professional (1993)

### SUMMARY OF THE INVENTION

### Problems to be Solved by the Invention

An object of the present invention is to provide a strong salty taste enhancer, a novel compound having a strong salty taste enhancing effect, and a salty taste enhancer comprising the compound, and the like.

### Means of Solving the Problems

The present inventors have conducted intensive studies and found that a compound of the following structure has a strong salty taste enhancing effect, which resulted in the completion of the present invention.

Accordingly, the present invention provides the following.
[1] A salty taste enhancer for a food or drink, which comprises a compound represented by the following formula: 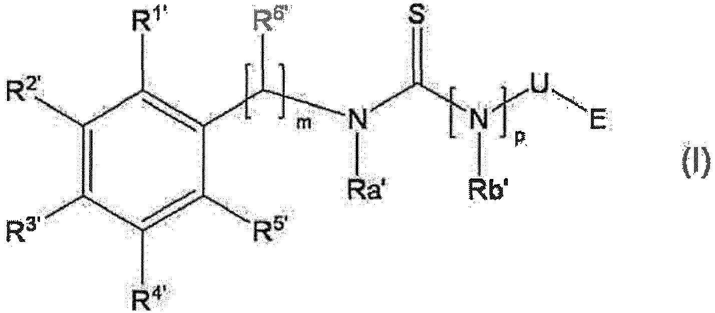 wherein
   R¹' to R⁵' are each independently (i) a hydrogen atom, (ii) a hydroxyl group, (iii) a halogen atom, (iv) a carboxyl group, (v) a C₁₋₄ alkoxy-carbonyl group, (vi) a C₁₋₄ alkoxy group, (vii) an amino group optionally substituted by a C₁₋₄ alkoxy-carbonyl group, (viii) a cyano group, (ix) a nitro group, (x) a C₁₋₄ alkyl group optionally substituted by 1 to 3 halogen atoms, (xi) a C₁₋₄ alkyl-carbonylamino group optionally substituted by an amino group optionally substituted by a C₁₋₄ alkoxy-carbonyl group, (xii) a C₁₋₄ alkylsulfonylamino group, or (xiii) a C₆₋₁₀ arylsulfonylamino group optionally substituted by a C₁₋₄ alkyl group, or
   any two from R¹' to R⁵' are optionally joined to form a C₁₋₄ alkylenedioxy group,
   R⁶' is (i) a hydrogen atom, (ii) a hydroxyl group, (iii) a C₁₋₄ alkyl group optionally substituted by 1 to 3 substituents selected from a hydroxyl group, a C₁₋₄ alkoxy group and a C₇₋₁₄ aralkyloxy group, (iv) a C₁₋₄ alkoxy group, (v) a carboxyl group, (vi) a C₁₋₄ alkoxy-carbonyl group, (vii) a C₆₋₁₀ aryl group, or (viii) a carbamoyl group, or
   R⁶' and R⁵' are optionally joined to form a C₁₋₄ alkylene group, m is an integer of 0 to 2,
   Ra' is a hydrogen atom, a hydroxyl group, a C₁₋₄ alkyl group or a C₇₋₁₄ aralkyl group, or
   Ra' and R⁵' are optionally joined to form a C₁₋₄ alkylene group, Rb' is a hydrogen atom, a hydroxyl group, a C₁₋₄ alkyl group or a C₇₋₁₄ aralkyl group, or
   Rb' and R⁶' are optionally joined to form a carbonyl group, p is 0 or 1,
   U is a single bond, -NH-CH₂-, -N=CR¹²'- wherein R¹²' is a hydrogen atom or a C₁₋₄ alkyl group, or a group represented by the following formula: 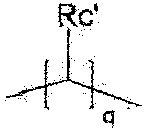
   wherein
   Rc' is (i) a hydrogen atom, (ii) a hydroxyl group, (iii) a C₁₋₄ alkyl group optionally substituted by 1 to 3 substituents selected from a hydroxyl group, a C₁₋₄ alkoxy group and a C₇₋₁₄ aralkyloxy group, (iv) a C₁₋₄ alkoxy group, (v) a carboxyl group, (vi) a C₁₋₄ alkoxy-carbonyl group, (vii) a C₆₋₁₀ aryl group, or (viii) a carbamoyl group, and
   q is an integer of 1 to 3, and
   E is a hydroxyl group, an amino group, a carbamoyl group, or
   a C₃₋₁₀ cycloalkyl group, a C₆₋₁₄ aryl group, or an aromatic heterocyclic group, each of which is optionally substituted by 1 to 3 substituents selected from (a) a hydroxyl group, (b) a halogen atom, (c) a carboxyl group, (d) a C₁₋₄ alkoxy-carbonyl group, (e) a C₁₋₄ alkoxy group, (f) an amino group optionally substituted by a C₁₋₄ alkoxy-carbonyl group, (g) a cyano group, (h) a nitro group, (i) a C₁₋₄ alkyl group optionally substituted by 1 to 3 halogen atoms, (j) a C₁₋₄ alkyl-carbonylamino group optionally substituted by an amino group optionally substituted by a C₁₋₄ alkoxy-carbonyl group, (k) a C₁₋₄ alkylsulfonylamino group, and (1) a C₆₋₁₀ arylsulfonylamino group optionally substituted by a C₁₋₄ alkyl group,
   provided that partial structures: 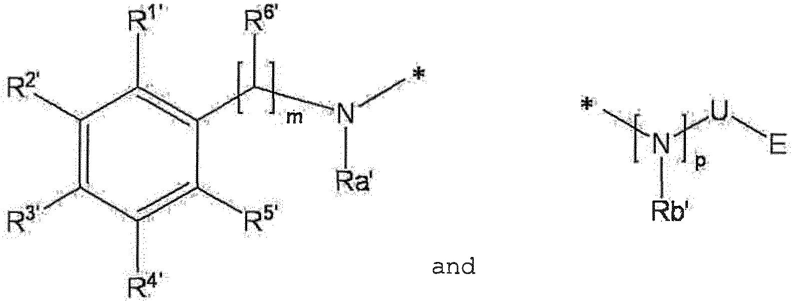 wherein * shows a binding position with a thiocarbonyl group, are not 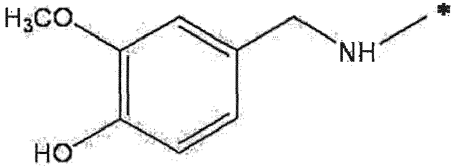 wherein * is as defined above,
   or an edible salt thereof.
[2] The salty taste enhancer for a food or drink of the above-mentioned [1], wherein, in the formula (I),
   R¹' to R⁵' are each independently (i) a hydrogen atom, (ii) a hydroxyl group, (iii) a halogen atom, (iv) a carboxyl group, (v) a C₁₋₄ alkoxy-carbonyl group, (vi) a C₁₋₄ alkoxy group, (vii) an amino group, or (viii) a C₁₋₄ alkyl group,
   R⁶' is (i) a hydrogen atom, (ii) a C₁₋₄ alkyl group optionally substituted by 1 to 3 substituents selected from a hydroxyl group and a C₁₋₄ alkoxy group, (iii) a carboxyl group, or (iv) a C₁₋₄ alkoxy-carbonyl group,
   Ra' is a hydrogen atom, a hydroxyl group or a C₁₋₄ alkyl group, Rb' is a hydrogen atom, a hydroxyl group or a C₁₋₄ alkyl group, p is 1,
   U is a single bond, -NH-CH₂-, -N=CR¹²'- wherein R¹²' is a hydrogen atom or a C₁₋₄ alkyl group, or a group represented by the following formula: 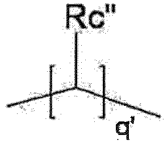 wherein
   Rc" is (i) a hydrogen atom, (ii) a hydroxyl group, (iii) a C₁₋₄ alkyl group optionally substituted by 1 to 3 substituents selected from a hydroxyl group and a C₁₋₄ alkoxy group, (iv) a carboxyl group, or (v) a C₁₋₄ alkoxy-carbonyl group, and q' is 1 or 2, and
   E is a phenyl group or an aromatic heterocyclic group, each of which is optionally substituted by 1 to 3 substituents selected from (a) a hydroxyl group, (b) a halogen atom, (c) a carboxyl group, (d) a C₁₋₄ alkoxy-carbonyl group, (e) a C₁₋₄ alkoxy group, (f) an amino group, and (g) a C₁₋₄ alkyl group.
[3] The salty taste enhancer for a food or drink of the above-mentioned [1], wherein the compound represented by the formula (I) is 1-(2-phenylethyl)-3-((R)-1-phenylethyl)thiourea.
[4] The salty taste enhancer for a food or drink of the above-mentioned [1], wherein the compound represented by the formula (I) is 1-((S)-2-hydroxy-1-phenylethyl)-3-(2-phenylethyl)thiourea.
[5] A method of adjusting salty taste of a food or drink, which comprises mixing a compound represented by the formula (I) of the above-mentioned [1] or an edible salt thereof, with a food or drink.
[6] A method of producing a food or drink, which comprises mixing a compound represented by the formula (I) of the above-mentioned [1] or an edible salt thereof, with a food or drink.
[7] A food or drink having an enhanced salty taste, which comprises not less than an effective amount of a compound represented by the formula (I) of the above-mentioned [1] or an edible salt thereof.
[8] A food or drink having an enhanced salty taste, which comprises effective amounts of a compound represented by the formula (I) of the above-mentioned [1] or an edible salt thereof and potassium chloride.
[9] A compound represented by the following formula: 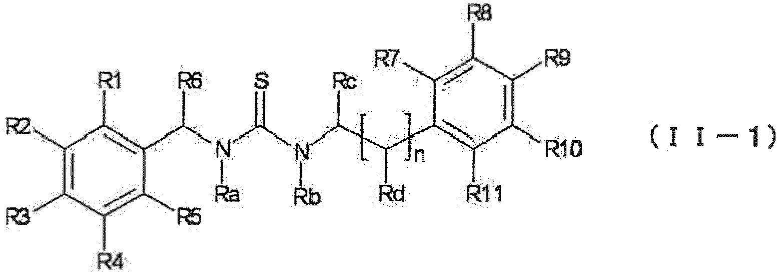 wherein
   R¹ to R⁵ are each independently a hydrogen atom, a hydroxyl group, a C₁₋₄ alkoxy group, a halogen atom or an amino group, R⁶ is (i) a hydrogen atom, (ii) a C₁₋₄ alkyl group optionally substituted by a hydroxyl group or a C₁₋₄ alkoxy group, (iii) a carboxyl group, or (vi) a C₁₋₄ alkoxy-carbonyl group, Ra and Rb are each independently a hydrogen atom, a hydroxyl group or a methyl group,
   Rc is (i) a hydrogen atom, (ii) a carboxyl group, (iii) a C₁₋₄ alkyl group optionally substituted by a hydroxyl group or a C₁₋₄ alkoxy group, or (iv) a C₁₋₄ alkoxy-carbonyl group; Rd is (i) a hydrogen atom, (ii) a hydroxyl group, or (iii) a C₁₋₄ alkyl group optionally substituted by a hydroxyl group or a C₁₋₄ alkoxy group;
   R⁷ to R¹¹ are each independently a hydrogen atom, a hydroxyl group, a carboxyl group, a halogen atom, a C₁₋₄ alkyl group or a C₁₋₄ alkoxy group; and
   n is 0 or 1,
   provided that
   (1) when n is 0, then at least two of R¹ to R⁵ are each a hydroxyl group,
   (2) when n is 1 and R⁶ is an unsubstituted C₁₋₄ alkyl group, then at least one of R¹ to R⁵ and R⁷ to R¹¹ is a hydroxyl group, and
   (3) when n is 1, R⁶ is a hydrogen atom, Ra is a hydrogen atom or a methyl group, Rc is (i) a hydrogen atom, (ii) a carboxyl group, (iii) a C₁₋₄ alkyl group optionally substituted by a C₁₋₄ alkoxy group, or (iv) a C₁₋₄ alkoxy-carbonyl group, and
      Rd is (i) a hydrogen atom, or (ii) a C₁₋₄ alkyl group optionally substituted by a hydroxyl group, then
      (i) at least one of R¹ to R⁵ is a hydroxyl group,
      (ii) when R³ is a hydroxyl group and R⁴ is a hydrogen atom, then R² is not a methoxy group,
      (iii) R⁹ is not a halogen atom or a methoxy group, and
      (iv) when Rb is a methyl group, then Rd is not a hydroxyl group,
   or an edible salt thereof.
[10] A compound represented by the following formula: 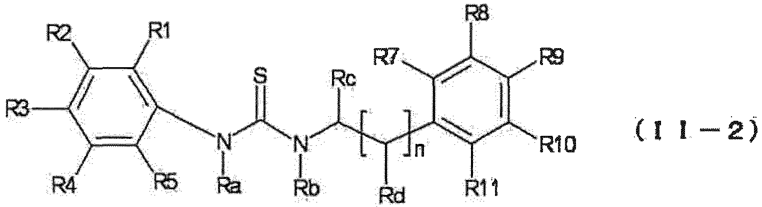 wherein
   R¹ is a hydrogen atom, a hydroxyl group, a carboxyl group or a C₁₋₄ alkoxy-carbonyl group,
   R² to R⁵ are each independently a hydrogen atom, a hydroxyl group, a C₁₋₄ alkoxy group or an amino group,
   Ra and Rb are each independently a hydrogen atom, a hydroxyl group or a C₁₋₄ alkyl group,
   Rc and Rd are each independently a hydrogen atom or a C₁₋₄ alkyl group,
   R⁷ to R¹¹ are each independently a hydrogen atom, a hydroxyl group, a carboxyl group, a halogen atom, a C₁₋₄ alkyl group or a C₁₋₄ alkoxy group, and
   n is 0 or 1,
   provided that
   (1) when n is 0, then
      (i) R¹ is a hydroxyl group or a carboxyl group, and Rc is a C₁₋₄ alkyl group, or
      (ii) at least two of R¹ to R⁵ are each a hydroxyl group, and
   (2) when n is 1, then
      (i) at least two of R¹ to R⁵ are each a hydroxyl group, or
      (ii) when R¹ is a carboxyl group or a C₁₋₄ alkoxy-carbonyl group, then (a) Rb is a C₁₋₄ alkyl group, and (b) at least one of R² to R⁵ and R⁷ to R¹¹ is a hydroxyl group, or an edible salt thereof.
[11] A compound represented by the following formula: 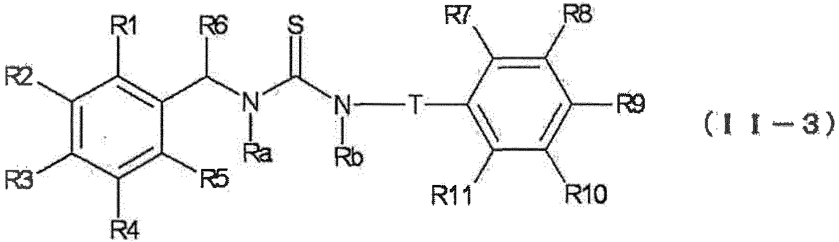 wherein
   R¹ to R⁵ are each independently a hydrogen atom, a hydroxyl group, a C₁₋₄ alkoxy group or an amino group,
   R⁶ is a C₁₋₄ alkyl group,
   Ra and Rb are each independently a hydrogen atom, a hydroxyl group or a C₁₋₄ alkyl group,
   T is -NH-CH₂- or -N=CR¹²- wherein R¹² is a hydrogen atom or a C₁₋₄ alkyl group, and
   R⁷ to R¹¹ are each independently a hydrogen atom, a hydroxyl group, a carboxyl group, a halogen atom, a C₁₋₄ alkyl group, a C₁₋₄ alkoxy group or a C₁₋₄ alkoxy-carbonyl group,
   provided that
   (1) at least one of R⁷ to R¹¹ is not a hydrogen atom, and
   (2) when T is -N=CH- and R⁷ is a hydroxyl group, then R¹¹ is not a hydrogen atom,
   or an edible salt thereof.
[12] A compound represented by the following formula: 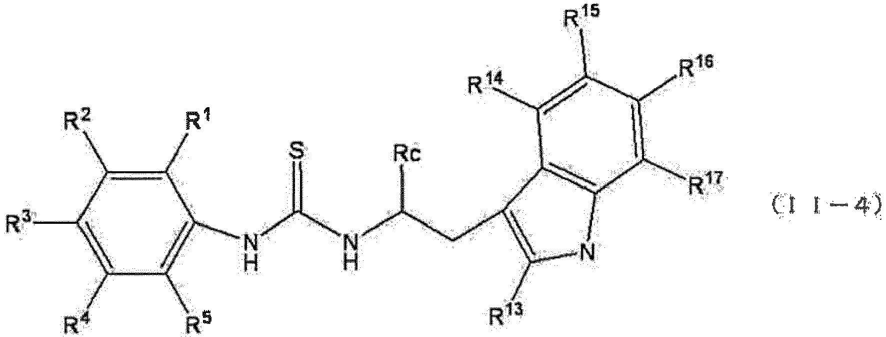 wherein
   R¹ to R⁵ are each independently a hydrogen atom, a hydroxyl group, a halogen atom, a carboxyl group, a C₁₋₄ alkyl group, a C₁₋₄ alkoxy group or a C₁₋₄ alkoxy-carbonyl group,
   Rc is a hydrogen atom, a carboxyl group, a C₁₋₄ alkyl group optionally substituted by 1 to 3 hydroxyl groups, or a C₁₋₄ alkoxy-carbonyl group, and
   R¹³ to R¹⁷ are each independently a hydrogen atom, a hydroxyl group, a halogen atom, a C₁₋₄ alkyl group or a C₁₋₄ alkoxy group,
   provided that
   (i) at least one of R¹ to R⁵ is a hydroxyl group, or
   (ii) when R¹ is a C₁₋₄ alkoxy group, then R⁵ is not a hydrogen atom,
   or an edible salt thereof.
[13] A compound selected from the group consisting of
   1-(3,4-dihydroxybenzyl)-3-(2-phenylethyl)thiourea;
   N-(2-phenylethyl)-3,4-dihydro-6,7-dihydroxyisoquinoline-2(1H)-carbothioamide;
   1-(3,4-dihydroxybenzyl)-1-methyl-3-(2-phenylethyl)thiourea;
   1-(4-hydroxybenzyl)-3-(2-phenylethyl)thiourea;
   1-(3-hydroxybenzyl)-3-(2-phenylethyl)thiourea;
   1-(3,4-dihydroxybenzyl)-3-phenylthiourea;
   1-(3,4-dihydroxybenzyl)-1-isobutyl-3-(2-phenylethyl)thiourea;
   1-((S)-2-hydroxy-1-phenylethyl)-3-(2-phenylethyl)thiourea;
   1-benzyl-3-[(R)-1-(hydroxymethyl)-2-phenylethyl]thiourea;
   1-benzyl-3-(2-hydroxy-2-phenylethyl)thiourea;
   1-benzyl-3-[(S)-1-(hydroxymethyl)-2-phenylethyl]thiourea;
   1-[2-(2-hydroxyphenyl)ethyl]-3-benzylthiourea;
   1-[2-(3-hydroxyphenyl)ethyl]-3-benzylthiourea;
   1-benzyl-1-hydroxy-3-(2-phenylethyl)thiourea;
   1-benzyl-3-((R)-2-hydroxy-2-phenylethyl)thiourea;
   1-benzyl-3-((S)-2-hydroxy-2-phenylethyl)thiourea;
   1-((S)-2-methoxy-1-phenylethyl)-3-(2-phenylethyl)thiourea;
   1-benzyl-3-(2-methoxy-2-phenylethyl)thiourea;
   1-(3-aminobenzyl)-3-(2-phenylethyl)thiourea;
   1-{3-[(2-aminoacetyl)amino]benzyl}-3-(2-phenylethyl)thiourea;
   1-[2-(2-hydroxyphenyl)ethyl]-3-((R)-1-phenylethyl)thiourea;
   1-[3-(mesylamino)benzyl]-3-(2-phenylethyl)thiourea;
   1-[2-(2-hydroxyphenyl)ethyl]-3-((R)-2,3-dihydro-1H-inden-1-yl)thiourea;
   1-[2-(2-hydroxyphenyl)ethyl]-3-[(R)-1-(3-hydroxyphenyl)ethyl]thiourea;
   1-(2-hydroxybenzyl)-4-((R)-1-phenylethyl)thiosemicarbazide;
   1-(2,6-dihydroxyphenyl)-3-((R)-1-phenylethyl)thiourea;
   2-{(E)-[4-((R)-1-phenylethyl)thiosemicarbazido]methylidene}benzoic acid;
   1-[2-(2-hydroxyphenyl)ethyl]-3-((R)-1-phenylpropyl)thiourea;
   1-[2-(2-hydroxyphenyl)ethyl]-3-[(R)-1-(3,4-dimethoxyphenyl)ethyl]thiourea;
   (S)-2-[3-(2-phenylethyl)thioureido]-2-phenylacetic acid;
   (S)-2-{3-[2-(2-hydroxyphenyl)ethyl]thioureido}-2-phenylacetic acid ;
   1-[2-(2-hydroxyphenyl)ethyl]-3-[(R)-1-(3,4-dihydroxyphenyl)ethyl]thiourea;
   1-[2-(2-hydroxyphenyl)-2-hydroxyethyl]-3-[(R)-1-(3-hydroxyphenyl)ethyl]thiourea;
   2-{3-[(R)-1-(3-hydroxyphenyl)ethyl]thioureido}-3-(2-hydroxyphenyl)propanoic acid;
   (R)-2-{3-[(R)-1-(3-hydroxyphenyl)ethyl]thioureido}-3-phenylpropanoic acid;
   (S)-2-{3-[(R)-1-(3-hydroxyphenyl)ethyl]thioureido}-3-phenylpropanoic acid;
   (R)-2-{3-[(R)-1-(3-hydroxyphenyl)ethyl]thioureido}-3-(4-hydroxyphenyl)propanoic acid;
   (S)-2-{3-[(R)-1-(3-hydroxyphenyl)ethyl]thioureido}-3-(4-hydroxyphenyl)propanoic acid;
   1-[2-(2,3-dihydroxyphenyl)-2-hydroxyethyl]-3-[(R)-1-(3-hydroxyphenyl)ethyl]thiourea;
   1-[2-(2,5-dihydroxyphenyl)-2-hydroxyethyl]-3-[(R)-1-(3-hydroxyphenyl)ethyl]thiourea;
   1-[2-(2-hydroxy-5-methylphenyl)-2-hydroxyethyl]-3-[(R)-1-(3-hydroxyphenyl)ethyl]thiourea;
   1-[2-(2,4-dihydroxyphenyl)-2-hydroxyethyl]-3-[(R)-1-(3-hydroxyphenyl)ethyl]thiourea;
   1-[2-(5-chloro-2-hydroxyphenyl)-2-hydroxyethyl]-3-[(R)-1-(3-hydroxyphenyl)ethyl]thiourea;
   1-[2-(5-bromo-2-hydroxyphenyl)-2-hydroxyethyl]-3-[(R)-1-(3-hydroxyphenyl)ethyl]thiourea;
   2-[3-(4-chlorophenyl)thioureido]benzoic acid;
   2-{3-[(R)-1-(3-hydroxyphenyl)ethyl]thioureido}benzoic acid;
   5-hydroxy-2-[3-methyl-3-(2-phenylethyl)thioureido]benzoic acid;
   methyl 2-{3-methyl-3-[2-(2-hydroxyphenyl)ethyl]thioureido}benzoate;
   methyl 2-{3-methyl-3-[2-(2-hydroxyphenyl)ethyl]thioureido}-5-hydroxybenzoate;
   2-{3-methyl-3-[2-(2-hydroxyphenyl)ethyl]thioureido}benzoic acid;
   2-{3-methyl-3-[2-(2-hydroxyphenyl)ethyl]thioureido}-5-hydroxybenzoic acid; and
   2-(2-{3-[(R)-1-(3-hydroxyphenyl)ethyl]thioureido}ethyl)benzoic acid; or an edible salt thereof.
[14] A method of enhancing a salty taste of a food or drink, comprising a step of mixing a compound represented by the formula (I) or an edible salt thereof, with a food or drink.
[15] A method of producing a food or drink with an enhanced salty taste, comprising a step of mixing a compound represented by the formula (I) or an edible salt thereof, with a food or drink.

### Effect of the Invention

The salty taste enhancer and the compound having a salty taste enhancing effect of the present invention are expected to show a strong salty taste enhancing effect, and can be used as salty taste enhancers for a food or drink, and the like. Description of Embodiments

The terms used in the present specification are explained below.

Examples of the "halogen atom" include chlorine atom, bromine atom, fluorine atom and iodine atom.

Examples of the "C₁₋₄ alkyl group" include methyl group, ethyl group, propyl group, isopropyl group, butyl group, sec-butyl group, tert-butyl group and the like.

Examples of the "C₁₋₄ alkoxy group" include methoxy group, ethoxy group, propoxy group, isopropoxy group, butoxy group, sec-butoxy group, tert-butoxy group and the like.

Examples of the "C₃₋₁₀ cycloalkyl group" include cyclopropyl group, cyclobutyl group, cyclopentyl group, cyclohexyl group, cycloheptyl group, cyclooctyl group, cyclononyl group, cyclodecyl group, adamantyl group and the like.

Examples of the "C₁₋₄ alkoxy-carbonyl group" include methoxycarbonyl group, ethoxycarbonyl group, propoxycarbonyl group, isopropoxycarbonyl group, butoxycarbonyl group, sec-butoxycarbonyl group, tert-butoxycarbonyl group and the like.

Examples of the "C₁₋₄ alkyl-carbonylamino group" include acetylamino group, propionylamino group, isopropylcarbonylamino group, butylcarbonylamino group, sec-butylcarbonylamino group, tert-butylcarbonylamino group and the like.

Examples of the "C₁₋₄ alkylsulfonylamino group" include methylsulfonylamino group, ethylsulfonylamino group, propylsulfonylamino group, isopropylsulfonylamino group, butylsulfonylamino group, sec-butylsulfonylamino group, tert-butylsulfonylamino group and the like.

Examples of the "C₆₋₁₀ aryl group" include phenyl group, naphthyl group (e.g., 1-naphthyl group, 2-naphthyl group) and the like.

Examples of the "C₆₋₁₀ arylsulfonylamino group" include phenylsulfonylamino group, naphthylsulfonylamino group (e.g., (naphthyl-1-yl)sulfonylamino group, (naphthyl-2-yl)sulfonylamino group) and the like.

Examples of the "C₇₋₁₄ aralkyl group" include benzyl group, 1-phenylethyl group, 2-phenylethyl group, naphthylmethyl group (1-naphthylmethyl group, 2-naphthylmethyl group), biphenylylmethyl group and the like.

Examples of the "C₇₋₁₄ aralkyloxy group" include benzyloxy group, phenethyloxy group, naphthylmethyloxy group (1-naphthylmethyloxy group, 2-naphthylmethyloxy group), biphenylylmethyloxy group and the like.

Examples of the "C₁₋₄ alkylene group" include methylene group, ethylene group, trimethylene group, tetramethylene group, -CH(CH₃)-, -CH(CH₃)-CH₂-, -CH₂-CH(CH₃)-, -C(CH₃)₂-, -CH(C₂H₅)-,-CH(CH₃)-CH₂-CH₂-, -CH₂-CH(CH₃)-CH₂-, -CH₂-CH₂-CH(CH₃)-, -CH(C₂H₅)-CH₂-, -CH₂-CH(C₂H₅)-, -CH (CH₃)-CH (CH₃)-, -C(CH₃)₂-CH₂-, -CH₂-C(CH₃)₂-, -C(CH₃)(C₂H₅)-, -CH(C₃H₇)-, -CH(CH(CH₃)₂)- and the like.

Examples of the "C₁₋₄ alkylenedioxy group" include methylenedioxy group, ethylenedioxy group, trimethylenedioxy group, tetramethylenedioxy group, -O-CH(CH₃)-O-, -O-CH(CH₃)-CH₂-O-, -O-CH₂-CH(CH₃)-O-, -O-C(CH₃)₂-O-, -O-CH(C₂H₅)-O-, -O-CH(CH₃)-CH₂-CH₂-O-, -O-CH₂-CH(CH₃)-CH₂-O-, -O-CH₂-CH₂-CH(CH₃)-O-, -O-CH(C₂H₅)-CH₂-O-, -O-CH₂-CH(C₂H₅)-O-, -O-CH(CH₃)-CH(CH₃)-O-, -O-C(CH₃)₂-CH₂-O-, -O-CH₂-C(CH₃)₂-O-, -O-C(CH₃)(C₂H₅)-O-, -O-CH(C₃H₇)-O-, -O-CH(CH(CH₃)₂)-O- and the like.

Examples of the "aromatic heterocyclic group" include a 4- to 7-membered (preferably 5- or 6-membered) monocyclic aromatic heterocyclic group containing, as a ring-constituting atom besides carbon atoms, 1 to 4 heteroatoms selected from an oxygen atom, a sulfur atom (said sulfur atom may be oxidized) and a nitrogen atom, and a fused aromatic heterocyclic group. Examples of the fused aromatic heterocyclic group include a group wherein such 4- to 7-membered monocyclic aromatic heterocyclic group, and 1 or 2 rings selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene) and a benzene ring etc. are condensed, and the like.

Preferable examples of aromatic heterocyclic group include
a monocyclic aromatic heterocyclic group such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,5-oxadiazol-3-yl, 1,3,4-oxadiazol-2-yl), thiadiazolyl (e.g., 1,2,3-thiadiazol-4-yl, 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-3-yl, 1,2,4-triazin-5-yl, 1,2,4-triazin-6-yl) and the like;
a fused aromatic heterocyclic group such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl, 4-benzofuranyl, 5-benzofuranyl, 6-benzofuranyl, 7-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl, 6-benzothiazolyl), benzimidazolyl (e.g., benzimidazol-1-yl, benzimidazol-2-yl, benzimidazol-5-yl), benzotriazolyl (e.g., 1H-1,2,3-benzotriazol-1-yl, 1H-1,2,3-benzotriazol-5-yl), indolyl (e.g., indol-1-yl, indol-2-yl, indol-3-yl, indol-5-yl), indazolyl (e.g., 2H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), thienopyrazolyl (e.g., 1H-thieno[2,3-c]pyrazol-5-yl), pyrazolotriazinyl (e.g., pyrazolo[5,1-c][1,2,4]triazin-3-yl), triazolopyrimidinyl (e.g., [1,2,4]triazolo[1,5-a]pyrimidin-2-yl), phthalazinyl and the like;
and the like.

Each moiety in the above-mentioned formula (I) is explained below.

R¹' to R⁵' are each independently (i) a hydrogen atom, (ii) a hydroxyl group, (iii) a halogen atom, (iv) a carboxyl group, (v) a C₁₋₄ alkoxy-carbonyl group, (vi) a C₁₋₄ alkoxy group, (vii) an amino group optionally substituted by a C₁₋₄ alkoxy-carbonyl group, (viii) a cyano group, (ix) a nitro group, (x) a C₁₋₄ alkyl group optionally substituted by 1 to 3 halogen atoms, (xi) a C₁₋₄ alkyl-carbonylamino group optionally substituted by an amino group optionally substituted by a C₁₋₄ alkoxy-carbonyl group, (xii) a C₁₋₄ alkylsulfonylamino group, or (xiii) a C₆₋₁₀ arylsulfonylamino group optionally substituted by a C₁₋₄ alkyl group, or
any two from R¹' to R⁵' are optionally joined to form a C₁₋₄ alkylenedioxy group.

The "halogen atom" for R¹' to R⁵' is preferably chlorine atom or bromine atom.

"C₁₋₄ alkoxy-carbonyl group" for R¹' to R⁵' is preferably methoxycarbonyl group.

The "C₁₋₄ alkoxy group" for R¹' to R⁵' is preferably methoxy group.

The "amino group" of the "amino group optionally substituted by a C₁₋₄ alkoxy-carbonyl group" for R¹' to R⁵' may be mono- or di-substituted (preferably, monosubstituted) by "C₁₋₄ alkoxy-carbonyl group" (preferably, tert-butoxycarbonyl group). When two "C₁₋₄ alkoxy-carbonyl groups" are present, they may be the same or different.

The "amino group optionally substituted by a C₁₋₄ alkoxy-carbonyl group" is preferably an amino group optionally mono-or di-substituted (preferably, monosubstituted) by a tert-butoxycarbonyl group, more preferably, amino group, tert-butoxycarbonylamino group.

The "C₁₋₄ alkyl group" of the "C₁₋₄ alkyl group optionally substituted by 1 to 3 halogen atoms" for R¹' to R⁵' is preferably methyl group. The "C₁₋₄ alkyl group" optionally has 1 to 3 halogen atoms (preferably, fluorine atom) at any substitutable position(s). When two or more halogen atoms are present, they may be the same or different.

The "C₁₋₄ alkyl group optionally substituted by 1 to 3 halogen atoms" is preferably a methyl group optionally substituted by 1 to 3 halogen atoms (preferably, fluorine atom), more preferably, methyl group, trifluoromethyl group.

As the "amino group optionally substituted by a C₁₋₄ alkoxy-carbonyl group" of the "C₁₋₄ alkyl-carbonylamino group optionally substituted by an amino group optionally substituted by a C₁₋₄ alkoxy-carbonyl group" for R¹' to R⁵', those similar to the above-mentioned "amino group optionally substituted by a C₁₋₄ alkoxy-carbonyl group" for R¹' to R⁵' can be mentioned.

The "C₁₋₄ alkyl-carbonylamino group" of the "C₁₋₄ alkyl-carbonylamino group optionally substituted by an amino group optionally substituted by a C₁₋₄ alkoxy-carbonyl group" is preferably acetylamino group. The "C₁₋₄ alkyl-carbonylamino group" optionally has 1 to 3 (preferably, 1) "amino group optionally substituted by a C₁₋₄ alkoxy-carbonyl group" mentioned above at any substitutable position(s). When two or more "amino groups optionally substituted by a C₁₋₄ alkoxy-carbonyl group" are present, they may be the same or different.

The "C₁₋₄ alkyl-carbonylamino group optionally substituted by an amino group optionally substituted by a C₁₋₄ alkoxy-carbonyl group" is preferably a C₁₋₄ alkyl-carbonylamino group (preferably, acetylamino group) optionally substituted by 1 to 3 (preferably, 1) substituents selected from amino group and tert-butoxycarbonylamino group, more preferably, 2-aminoacetylamino group, 2-(tert-butoxycarbonylamino)acetylamino group.

The "C₁₋₄ alkylsulfonylamino group" for R¹' to R⁵' is preferably methylsulfonylamino group.

The "C₆₋₁₀ arylsulfonylamino group" of the "C₆₋₁₀ arylsulfonylamino group optionally substituted by a C₁₋₄ alkyl group" for R¹' to R⁵' is preferably phenylsulfonylamino group. The "C₆₋₁₀ arylsulfonylamino group" optionally has 1 to 3 (preferably, 1) C₁₋₄ alkyl groups (preferably, methyl group) at any substitutable position(s). When two or more "C₁₋₄ alkyl groups" are present, they may be the same or different.

The "C₆₋₁₀ arylsulfonylamino group optionally substituted by a C₁₋₄ alkyl group" is preferably a C₆₋₁₀ arylsulfonylamino group (preferably, phenylsulfonylamino group) optionally substituted by 1 to 3 (preferably, 1) methyl groups, more preferably, 4-methylphenylsulfonylamino group.

The "C₁₋₄ alkylenedioxy group" which may be jointly formed by R¹' to R⁵' is preferably methylenedioxy group.

R¹' to R⁵' is preferably are each independently (i) a hydrogen atom, (ii) a hydroxyl group, (iii) a halogen atom (preferably, chlorine atom, bromine atom), (iv) a carboxyl group, (v) a C₁₋₄ alkoxy-carbonyl group (preferably, methoxycarbonyl group), (vi) a C₁₋₄ alkoxy group (preferably, methoxy group), (vii) an amino group optionally mono- or di-substituted (preferably, monosubstituted) by a tert-butoxycarbonyl group (preferably, amino group, tert-butoxycarbonylamino group), (viii) a cyano group, (ix) a nitro group, (x) a C₁₋₄ alkyl group (preferably, methyl group) optionally substituted by 1 to 3 halogen atoms (preferably, fluorine atom) (preferably, methyl group, trifluoromethyl group), (xi) a C₁₋₄ alkyl-carbonylamino group (preferably, acetylamino group) optionally substituted by 1 to 3 (preferably, 1) substituents selected from an amino group and a tert-butoxycarbonylamino group (preferably, 2-aminoacetylamino group, 2-(tert-butoxycarbonylamino)acetylamino group), (xii) a C₁₋₄ alkylsulfonylamino group (preferably, methylsulfonylamino group), or (xiii) a C₆₋₁₀ arylsulfonylamino group (preferably, phenylsulfonylamino group) optionally substituted by 1 to 3 (preferably, 1) C₁₋₄ alkyl groups (preferably, methyl group) (preferably, 4-methylphenylsulfonylamino group), or any two from R¹' to R⁵' are optionally joined to form a C₁₋₄ alkylenedioxy group (preferably, methylenedioxy group).

More preferably, R¹' to R⁵' are each independently (i) a hydrogen atom, (ii) a hydroxyl group, (iii) a halogen atom (preferably, chlorine atom, bromine atom), (iv) a carboxyl group, (v) a C₁₋₄ alkoxy-carbonyl group (preferably, methoxycarbonyl group), (vi) a C₁₋₄ alkoxy group (preferably, methoxy group), (vii) an amino group, or (viii) a C₁₋₄ alkyl group (preferably, methyl group).

R⁶' is (i) a hydrogen atom, (ii) a hydroxyl group, (iii) a C₁₋₄ alkyl group optionally substituted by 1 to 3 substituents selected from a hydroxyl group, a C₁₋₄ alkoxy group and a C₇₋₁₄ aralkyloxy group, (iv) a C₁₋₄ alkoxy group, (v) a carboxyl group, (vi) a C₁₋₄ alkoxy-carbonyl group, (vii) a C₆₋₁₀ aryl group, or (viii) a carbamoyl group, or
R⁶' and R⁵' are optionally joined to form a C₁₋₄ alkylene group.

The "C₁₋₄ alkyl group" of the "C₁₋₄ alkyl group optionally substituted by 1 to 3 substituents selected from a hydroxyl group, a C₁₋₄ alkoxy group and a C₇₋₁₄ aralkyloxy group" for R⁶' is preferably methyl group, ethyl group, propyl group. The "C₁₋₄ alkyl group" optionally has 1 to 3 (preferably, 1) substituent(s) selected from a hydroxyl group, a C₁₋₄ alkoxy group (preferably, methoxy group) and a C₇₋₁₄ aralkyloxy group (preferably, benzyloxy group) at any substitutable position(s). When two or more substituents are present, they may be the same or different.

The "C₁₋₄ alkyl group optionally substituted by 1 to 3 substituents selected from a hydroxyl group, a C₁₋₄ alkoxy group and a C₇₋₁₄ aralkyloxy group" is preferably a C₁₋₄ alkyl group (preferably, methyl group, ethyl group, propyl group) optionally substituted by 1 to 3 (preferably, 1) substituents selected from a hydroxyl group, a methoxy group and a benzyloxy group, more preferably, methyl group, hydroxymethyl group, methoxymethyl group, benzyloxymethyl group, ethyl group, propyl group.

The "C₁₋₄ alkoxy group" for R⁶' is preferably methoxy group.

The "C₁₋₄ alkoxy-carbonyl group" for R⁶' is preferably methoxycarbonyl group, ethoxycarbonyl group.

The "C₆₋₁₀ aryl group" for R⁶' is preferably phenyl group.

The "C₁₋₄ alkylene group" which may be jointly formed by R⁶' and R⁵' is preferably ethylene group.

R⁶' is preferably (i) a hydrogen atom, (ii) a hydroxyl group, (iii) a C₁₋₄ alkyl group (preferably, methyl group, ethyl group, propyl group) optionally substituted by 1 to 3 (preferably, 1) substituents selected from a hydroxyl group, a methoxy group and a benzyloxy group (preferably, methyl group, hydroxymethyl group, methoxymethyl group, benzyloxymethyl group, ethyl group, propyl group), (iv) a C₁₋₄ alkoxy group (preferably, methoxy group), (v) a carboxyl group, (vi) a C₁₋₄ alkoxy-carbonyl group, (vii) a C₆₋₁₀ aryl group (preferably, phenyl group), or (viii) a carbamoyl group, or
R⁶' and R⁵' are optionally joined to form a C₁₋₄ alkylene group (preferably, ethylene group).

In another embodiment, R⁶' is preferably (i) a hydrogen atom, (ii) a C₁₋₄ alkyl group (preferably, methyl group, ethyl group, propyl group) optionally substituted by 1 to 3 (preferably, 1) substituents selected from a hydroxyl group and a C₁₋₄ alkoxy group (preferably, methoxy group), (iii) a carboxyl group, or (iv) a C₁₋₄ alkoxy-carbonyl group.

m is an integer of 0 to 2.

When m is 2, two R⁶' may be the same or different.

Ra' is a hydrogen atom, a hydroxyl group, a C₁₋₄ alkyl group or a C₇₋₁₄ aralkyl group, or Ra' and R⁵' are optionally joined to form a C₁₋₄ alkylene group.

The "C₁₋₄ alkyl group" for Ra' is preferably methyl group, isobutyl group.

The "C₇₋₁₄ aralkyl group" for Ra' is preferably benzyl group.

The "C₁₋₄ alkylene group" which may be jointly formed by Ra' and R⁵' is preferably methylene group, ethylene group.

Ra' is preferably a hydrogen atom, a hydroxyl group, a C₁₋₄ alkyl group (preferably, methyl group, isobutyl group) or benzyl group, or Ra' and R⁵' are optionally joined to form a C₁₋₄ alkylene group (preferably, methylene group, ethylene group).

Ra' is more preferably a hydrogen atom, a hydroxyl group or a C₁₋₄ alkyl group (preferably, methyl group, isobutyl group).

Rb' is a hydrogen atom, a hydroxyl group, a C₁₋₄ alkyl group or a C₇₋₁₄ aralkyl group, or Rb' and R⁶' are optionally joined to form a carbonyl group.

The "C₁₋₄ alkyl group" for Rb' is preferably methyl group, isobutyl group.

The "C₇₋₁₄ aralkyl group" for Rb' is preferably benzyl group.

Rb' is preferably a hydrogen atom, a hydroxyl group, a C₁₋₄ alkyl group (preferably, methyl group, isobutyl group) or a benzyl group, or Rb' and R⁶' are optionally joined to form a carbonyl group.

Rb' is more preferably a hydrogen atom, a hydroxyl group or a C₁₋₄ alkyl group (preferably, methyl group, isobutyl group).

p is 0 or 1.

p is preferably 1.

U is a single bond, -NH-CH₂-, -N=CR¹²' - wherein R¹²' is a hydrogen atom or a C₁₋₄ alkyl group or a group represented by the following formula: 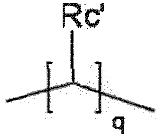 wherein
Rc' is (i) a hydrogen atom, (ii) a hydroxyl group, (iii) a C₁₋₄ alkyl group optionally substituted by 1 to 3 substituents selected from a hydroxyl group, a C₁₋₄ alkoxy group and a C₇₋₁₄ aralkyloxy group, (iv) a C₁₋₄ alkoxy group, (v) a carboxyl group, (vi) a C₁₋₄ alkoxy-carbonyl group, (vii) a C₆₋₁₀ aryl group, or (viii) a carbamoyl group, and
q is an integer of 1 to 3.

R¹²' is preferably a hydrogen atom, a methyl group, more preferably, a hydrogen atom.

The "-N=CR¹²' - wherein R¹²' is a hydrogen atom or a C₁₋₄ alkyl group" for U is preferably -N=C(CH₃)-, -N=CH-, more preferably, -N=CH-.

As the "C₁₋₄ alkyl group optionally substituted by 1 to 3 substituents selected from a hydroxyl group, a C₁₋₄ alkoxy group and a C₇₋₁₄ aralkyloxy group", "C₁₋₄ alkoxy group", "C₁₋₄ alkoxy-carbonyl group" and "C₆₋₁₀ aryl group" for Rc', those similar to the above-mentioned "C₁₋₄ alkyl group optionally substituted by 1 to 3 substituents selected from a hydroxyl group, a C₁₋₄ alkoxy group and a C₇₋₁₄ aralkyloxy group", "C₁₋₄ alkoxy group", "C₁₋₄ alkoxy-carbonyl group" and "C₆₋₁₀ aryl group" for R⁶' can be mentioned.

When q is 2 or 3, plural Rc' may be the same or different.

q is preferably 1 or 2.

A group represented by the following formula: 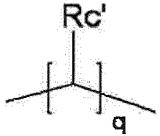 wherein each symbol is as defined above,
is preferably a group represented by the following formula: 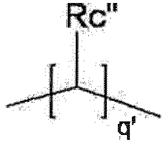 wherein
Rc" is (i) a hydrogen atom, (ii) a hydroxyl group, (iii) a C₁₋₄ alkyl group (preferably, methyl group, ethyl group, propyl group) optionally substituted by 1 to 3 (preferably, 1) substituents selected from a hydroxyl group and a C₁₋₄ alkoxy group (preferably, methoxy group), (iv) a carboxyl group, or (v) a C₁₋₄ alkoxy-carbonyl group, and
q' is 1 or 2.

U is preferably a single bond, -NH-CH₂-, -N=CR¹²'- wherein R¹²' is a hydrogen atom or a C₁₋₄ alkyl group (preferably, - N=CH-), or a group represented by the following formula: 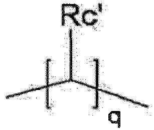 wherein
Rc' is (i) a hydrogen atom, (ii) a hydroxyl group, (iii) a C₁₋₄ alkyl group (preferably, methyl group, ethyl group, propyl group) optionally substituted by 1 to 3 (preferably, 1) substituents selected from hydroxyl group, methoxy group and benzyloxy group (preferably, methyl group, hydroxymethyl group, methoxymethyl group, benzyloxymethyl group, ethyl group, propyl group), (iv) a C₁₋₄ alkoxy group (preferably, methoxy group), (v) a carboxyl group, (vi) a C₁₋₄ alkoxy-carbonyl group, (vii) a C₆₋₁₀ aryl group (preferably, phenyl group), or (viii) a carbamoyl group, and
q is an integer of 1 to 3.

In another embodiment, U is preferably a single bond,-NH-CH₂-, -N=CR¹²' - wherein R¹²' is as defined above (preferably, -N=CH-), or a group represented by the following formula: 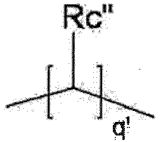 wherein
Rc" is (i) a hydrogen atom, (ii) a hydroxyl group, (iii) a C₁₋₄ alkyl group (preferably, methyl group, ethyl group, propyl group) optionally substituted by 1 to 3 (preferably, 1) substituents selected from a hydroxyl group and a C₁₋₄ alkoxy group (preferably, methoxy group), (iv) a carboxyl group, or (v) a C₁₋₄ alkoxy-carbonyl group, and
q' is 1 or 2.

E is a hydroxyl group, an amino group, a carbamoyl group, or
a C₃₋₁₀ cycloalkyl group, a C₆₋₁₄ aryl group or an aromatic heterocyclic group, each of which is optionally substituted by 1 to 3 substituents selected from (a) a hydroxyl group, (b) a halogen atom, (c) a carboxyl group, (d) a C₁₋₄ alkoxy-carbonyl group, (e) a C₁₋₄ alkoxy group, (f) an amino group optionally substituted by a C₁₋₄ alkoxy-carbonyl group, (g) a cyano group, (h) a nitro group, (i) a C₁₋₄ alkyl group optionally substituted by 1 to 3 halogen atoms, (j) a C₁₋₄ alkyl-carbonylamino group optionally substituted by an amino group optionally substituted by a C₁₋₄ alkoxy-carbonyl group, (k) a C₁₋₄ alkylsulfonylamino group, and (1) a C₆₋₁₀ arylsulfonylamino group optionally substituted by a C₁₋₄ alkyl group.

The "C₃₋₁₀ cycloalkyl group" for E is preferably a cyclohexyl group or an adamantyl group.

The "C₆₋₁₄ aryl group" for E is preferably a phenyl group or a naphthyl group (preferably, 1-naphthyl group).

The "aromatic heterocyclic group" for E is preferably pyridyl (preferably, 3-pyridyl), furyl (preferably, 2-furyl), indolyl (preferably, indol-3-yl) or benzimidazolyl (preferably, benzimidazol-2-yl).

The "C₃₋₁₀ cycloalkyl group", "C₆₋₁₄ aryl group" and "aromatic heterocyclic group" optionally have 1 to 3 substituents selected from (a) a hydroxyl group, (b) a halogen atom, (c) a carboxyl group, (d) a C₁₋₄ alkoxy-carbonyl group, (e) a C₁₋₄ alkoxy group, (f) an amino group optionally substituted by a C₁₋₄ alkoxy-carbonyl group, (g) a cyano group, (h) a nitro group, (i) a C₁₋₄ alkyl group optionally substituted by 1 to 3 halogen atoms, (j) a C₁₋₄ alkyl-carbonylamino group optionally substituted by an amino group optionally substituted by a C₁₋₄ alkoxy-carbonyl group, (k) a C₁₋₄ alkylsulfonylamino group, and (1) a C₆₋₁₀ arylsulfonylamino group optionally substituted by a C₁₋₄ alkyl group at any substitutable position(s).

Examples of the "C₁₋₄ alkoxy-carbonyl group", "C₁₋₄ alkoxy group", "amino group optionally substituted by a C₁₋₄ alkoxy-carbonyl group", "C₁₋₄ alkyl group optionally substituted by 1 to 3 halogen atoms", "C₁₋₄ alkyl-carbonylamino group optionally substituted by an amino group optionally substituted by a C₁₋₄ alkoxy-carbonyl group", "C₁₋₄ alkylsulfonylamino group" and "C₆₋₁₀ arylsulfonylamino group optionally substituted by a C₁₋₄ alkyl group" as such substituents include those similar to the above-mentioned "C₁₋₄ alkoxy-carbonyl group", "C₁₋₄ alkoxy group", "amino group optionally substituted by a C₁₋₄ alkoxy-carbonyl group", "C₁₋₄ alkyl group optionally substituted by 1 to 3 halogen atoms", "C₁₋₄ alkyl-carbonylamino group optionally substituted by an amino group optionally substituted by a C₁₋₄ alkoxy-carbonyl group", "C₁₋₄ alkylsulfonylamino group" and "C₆₋₁₀ arylsulfonylamino group optionally substituted by a C₁₋₄ alkyl group", respectively, for R¹' to R⁵'.

E is preferably a hydroxyl group, an amino group, a carbamoyl group, or
a C₃₋₁₀ cycloalkyl group (preferably, cyclohexyl group, adamantyl group), a C₆₋₁₄ aryl group [preferably, phenyl group, naphthyl group (preferably, 1-naphthyl group)], or an aromatic heterocyclic group [preferably, pyridyl (preferably, 3-pyridyl), furyl (preferably, 2-furyl), indolyl (preferably, indol-3-yl), benzimidazolyl (preferably, benzimidazol-2-yl)], each of which is optionally substituted by 1 to 3 substituents selected from (a) a hydroxyl group, (b) a halogen atom (preferably, chlorine atom, bromine atom), (c) a carboxyl group, (d) a C₁₋₄ alkoxy-carbonyl group (preferably, methoxycarbonyl group), (e) a C₁₋₄ alkoxy group (preferably, methoxy group), (f) an amino group optionally mono- or di-substituted (preferably, monosubstituted) by tert-butoxycarbonyl group (preferably, amino group, tert-butoxycarbonylamino group), (g) a cyano group, (h) a nitro group, (i) a C₁₋₄ alkyl group (preferably, methyl group) optionally substituted by 1 to 3 halogen atoms (preferably, fluorine atom) (preferably, methyl group, trifluoromethyl group), (j) a C₁₋₄ alkyl-carbonylamino group (preferably, acetylamino group) optionally substituted by 1 to 3 (preferably, 1) substituents selected from amino group and tert-butoxycarbonylamino group (preferably, 2-aminoacetylamino group, 2-(tert-butoxycarbonylamino)acetylamino group), (k) a C₁₋₄ alkylsulfonylamino group (preferably, methylsulfonylamino group), and (1) a C₆₋₁₀ arylsulfonylamino group (preferably, phenylsulfonylamino group) optionally substituted by 1 to 3 (preferably, 1) C₁₋₄ alkyl groups (preferably, methyl group) (preferably, 4-methylphenylsulfonylamino group).

In another embodiment, E is preferably a phenyl group or an aromatic heterocyclic group [preferably, pyridyl (preferably, 3-pyridyl), furyl (preferably, 2-furyl), indolyl (preferably, indol-3-yl)], each of which is optionally substituted by 1 to 3 (preferably, 1 or 2) substituents selected from (a) a hydroxyl group, (b) a halogen atom (preferably, chlorine atom, bromine atom), (c) a carboxyl group, (d) a C₁₋₄ alkoxy-carbonyl group (preferably, methoxycarbonyl group), (e) a C₁₋₄ alkoxy group (preferably, methoxy group), (f) an amino group, and (g) a C₁₋₄ alkyl group (preferably, methyl group),
provided that partial structures: 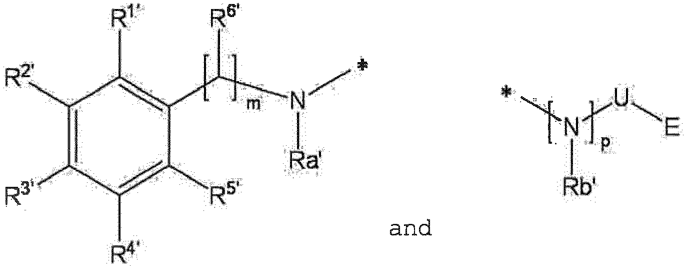 wherein * shows a binding position with a thiocarbonyl group, are not

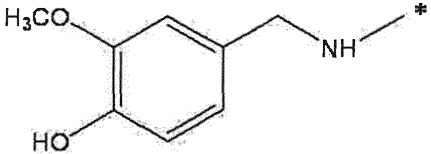

A compound represented by the formula (I) (hereinafter sometimes to be abbreviated as compound (I)) is preferably a compound wherein
R¹' to R⁵' are each independently (i) a hydrogen atom, (ii) a hydroxyl group, (iii) a halogen atom (preferably, chlorine atom, bromine atom), (iv) a carboxyl group, (v) a C₁₋₄ alkoxy-carbonyl group (preferably, methoxycarbonyl group), (vi) a C₁₋₄ alkoxy group (preferably, methoxy group), (vii) an amino group optionally mono- or di-substituted (preferably, monosubstituted) by a C₁₋₄ alkoxy-carbonyl group (preferably, tert-butoxycarbonyl group) (preferably, amino group, tert-butoxycarbonylamino group), (viii) a cyano group, (ix) a nitro group, (x) a C₁₋₄ alkyl group (preferably, methyl group) optionally substituted by 1 to 3 halogen atoms (preferably, fluorine atom) (preferably, methyl group, trifluoromethyl group), (xi) a C₁₋₄ alkyl-carbonylamino group (preferably, acetylamino group) optionally substituted by 1 to 3 (preferably, 1) substituents selected from an amino group optionally mono-or di-substituted (preferably, monosubstituted) by a C₁₋₄ alkoxy-carbonyl group (preferably, tert-butoxycarbonyl group) (preferably, amino group, tert-butoxycarbonylamino group) (preferably, 2-aminoacetylamino group, 2-(tert-butoxycarbonylamino)acetylamino group), (xii) a C₁₋₄ alkylsulfonylamino group (preferably, methylsulfonylamino group), or (xiii) a C₆₋₁₀ arylsulfonylamino group (preferably, phenylsulfonylamino group) optionally substituted by 1 to 3 (preferably, 1) C₁₋₄ alkyl groups (preferably, methyl group) (preferably, 4-methylphenylsulfonylamino group), or
Any two from R¹' to R⁵' are optionally jointed to form a C₁₋₄ alkylenedioxy group (preferably, methylenedioxy group);
R⁶' is (i) a hydrogen atom, (ii) a hydroxyl group, (iii) a C₁₋₄ alkyl group (preferably, methyl group, ethyl group, propyl group) optionally substituted by 1 to 3 (preferably, 1) substituents selected from a hydroxyl group, a C₁₋₄ alkoxy group (preferably, methoxy group) and a C₇₋₁₄ aralkyloxy group (preferably, benzyloxy group) (preferably, methyl group, hydroxymethyl group, methoxymethyl group, benzyloxymethyl group, ethyl group, propyl group), (iv) a C₁₋₄ alkoxy group (preferably, methoxy group), (v) a carboxyl group, (vi) a C₁₋₄ alkoxy-carbonyl group, (vii) a C₆₋₁₀ aryl group (preferably, phenyl group), or (viii) a carbamoyl group, or
R⁶' and R⁵' are optionally jointed to form a C₁₋₄ alkylene group (preferably, ethylene group);
m is an integer of 0 to 2;
Ra' is a hydrogen atom, a hydroxyl group, a C₁₋₄ alkyl group (preferably, methyl group, isobutyl group) or a C₇₋₁₄ aralkyl group (preferably, benzyl group), or
Ra' and R⁵' are optionally jointed to form a C₁₋₄ alkylene group (preferably, methylene group, ethylene group);
Rb' is a hydrogen atom, a hydroxyl group, a C₁₋₄ alkyl group (preferably, methyl group, isobutyl group) or a C₇₋₁₄ aralkyl group (preferably, benzyl group), or
Rb' and R⁶' are optionally jointed to form a carbonyl group;
p is 0 or 1;
U is a single bond, -NH-CH₂-, -N=CR¹²' - wherein R¹²' is a hydrogen atom or a C₁₋₄ alkyl group (preferably, -N=CH-), or a group represented by the following formula: 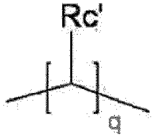 wherein
Rc' is (i) a hydrogen atom, (ii) a hydroxyl group, (iii) a C₁₋₄ alkyl group (preferably, methyl group, ethyl group, propyl group) optionally substituted by 1 to 3 (preferably, 1) substituent selected from a hydroxyl group, a C₁₋₄ alkoxy group (preferably, methoxy group) and a C₇₋₁₄ aralkyloxy group (preferably, benzyloxy group) (preferably, methyl group, hydroxymethyl group, methoxymethyl group, benzyloxymethyl group, ethyl group, propyl group), (iv) a C₁₋₄ alkoxy group (preferably, methoxy group), (v) a carboxyl group, (vi) a C₁₋₄ alkoxy-carbonyl group, (vii) a C₆₋₁₀ aryl group (preferably, phenyl group), or (viii) a carbamoyl group,
q is an integer of 1 to 3; and
E is a hydroxyl group, an amino group, a carbamoyl group, or
a C₃₋₁₀ cycloalkyl group (preferably, cyclohexyl group, adamantyl group), phenyl group, naphthyl group (preferably, 1-naphthyl group), pyridyl (preferably, 3-pyridyl), furyl (preferably, 2-furyl), indolyl (preferably, indol-3-yl), or benzimidazolyl (preferably, benzimidazol-2-yl), each of which is optionally substituted by 1 to 3 substituents selected from (a) a hydroxyl group, (b) a halogen atom (preferably, chlorine atom, bromine atom), (c) a carboxyl group, (d) a C₁₋₄ alkoxy-carbonyl group (preferably, methoxycarbonyl group), (e) a C₁₋₄ alkoxy group (preferably, methoxy group), (f) an amino group optionally mono- or di-substituted (preferably, monosubstituted) by a C₁₋₄ alkoxy-carbonyl group (preferably, tert-butoxycarbonyl group) (preferably, amino group, tert-butoxycarbonylamino group), (g) a cyano group, (h) a nitro group, (i) a C₁₋₄ alkyl group (preferably, methyl group) optionally substituted by 1 to 3 halogen atoms (preferably, fluorine atom) (preferably, methyl group, trifluoromethyl group), (j) a C₁₋₄ alkyl-carbonylamino group (preferably, acetylamino group) optionally substituted by 1 to 3 (preferably, 1) substituents selected from an amino group optionally mono-or di-substituted (preferably, monosubstituted) by a C₁₋₄ alkoxy-carbonyl group (preferably, tert-butoxycarbonyl group) (preferably, amino group, tert-butoxycarbonylamino group) (preferably, 2-aminoacetylamino group, 2-(tert-butoxycarbonylamino)acetylamino group), (k) a C₁₋₄ alkylsulfonylamino group (preferably, methylsulfonylamino group), and (1) a C₆₋₁₀ arylsulfonylamino group (preferably, phenylsulfonylamino group) optionally substituted by 1 to 3 (preferably, 1) C₁₋₄ alkyl groups (preferably, methyl group) (preferably, 4-methylphenylsulfonylamino group).

In another embodiment, the compound represented by the formula (I) is preferably a compound wherein
R¹' to R⁵' are each independently (i) a hydrogen atom, (ii) a hydroxyl group, (iii) a halogen atom (preferably, chlorine atom, bromine atom), (iv) a carboxyl group, (v) a C₁₋₄alkoxy-carbonyl group (preferably, methoxycarbonyl group), (vi) a C₁₋₄alkoxy group (preferably, methoxy group), (vii) an amino group, or (viii) a C₁₋₄ alkyl group (preferably, methyl group);
R⁶' is (i) a hydrogen atom, (ii) a C₁₋₄ alkyl group (preferably, methyl group, ethyl group, propyl group) optionally substituted by 1 to 3 (preferably, 1) substituents selected from a hydroxyl group and a C₁₋₄ alkoxy group (preferably, methoxy group), (iii) a carboxyl group, or (iv) a C₁₋₄ alkoxy-carbonyl group; m is an integer of 0 to 2;
Ra' is a hydrogen atom, a hydroxyl group or a C₁₋₄ alkyl group (preferably, methyl group, isobutyl group);
Rb' is a hydrogen atom, a hydroxyl group or a C₁₋₄ alkyl group (preferably, methyl group, isobutyl group);
p is 1;
U is a single bond, -NH-CH₂-, -N=CR¹²' - wherein R¹²' is as defined above (preferably, -N=CH-), or a group represented by the following formula:

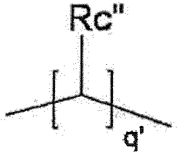

wherein
Rc'' is (i) a hydrogen atom, (ii) a hydroxyl group, (iii) a C₁₋₄ alkyl group (preferably, methyl group, ethyl group, propyl group) optionally substituted by 1 to 3 (preferably, 1) substituents selected from a hydroxyl group and a C₁₋₄ alkoxy group (preferably, methoxy group), (iv) a carboxyl group, or (v) a C₁₋₄ alkoxy-carbonyl group, and
q' is 1 or 2; and
E is a phenyl group or an aromatic heterocyclic group [preferably, pyridyl (preferably, 3-pyridyl), furyl (preferably, 2-furyl), indolyl (preferably, indol-3-yl)], each of which is optionally substituted by 1 to 3 (preferably, 1 or 2) substituents selected from (a) a hydroxyl group, (b) a halogen atom (preferably, chlorine atom, bromine atom), (c) a carboxyl group, (d) a C₁₋₄ alkoxy-carbonyl group (preferably, methoxycarbonyl group), (e) a C₁₋₄ alkoxy group (preferably, methoxy group), (f) an amino group, and (g) a C₁₋₄ alkyl group (preferably, methyl group).

The compound represented by the formula (I) is particularly preferably a compound disclosed in the following Examples.

Furthermore, in another embodiment, the compound represented by the formula (I) is preferably a compound represented by the above-mentioned formula (II-1), (II-2), (II-3) or (II-4). The compound represented by the formula (II-1), (II-2), (II-3) or (II-4) is a novel compound.

The compound represented by the formula (II-1) is preferably a compound wherein
R¹ to R⁵ are each independently a hydrogen atom, a hydroxyl group, or a C₁₋₄ alkoxy group (preferably, methoxy group);
R⁶ is (i) a hydrogen atom, (ii) a C₁₋₄ alkyl group (preferably, methyl group, ethyl group, propyl group) optionally substituted by a hydroxyl group or a C₁₋₄ alkoxy group (preferably, methoxy group), or (iii) a carboxyl group;
Ra and Rb are each independently a hydrogen atom, a hydroxyl group or a methyl group;
Rc is (i) a hydrogen atom, (ii) a carboxyl group, or (iii) a C₁₋₄ alkyl group (preferably, methyl group) optionally substituted by a hydroxyl group;
Rd is (i) a hydrogen atom or (ii) a hydroxyl group;
R⁷ to R¹¹ are each independently a hydrogen atom, a hydroxyl group, a carboxyl group, a halogen atom (preferably, chlorine atom, bromine atom), or a C₁₋₄ alkyl group (preferably, methyl group); and
n is 0 or 1.

The compound represented by the formula (II-2) is preferably a compound wherein
R¹ is a hydrogen atom, a hydroxyl group, a carboxyl group or a C₁₋₄ alkoxy-carbonyl group (preferably, methoxycarbonyl group); R² to R⁵ are each independently a hydrogen atom or a hydroxyl group;
Ra and Rb are each independently a hydrogen atom or a C₁₋₄ alkyl group (preferably, methyl group);
Rc and Rd are each independently a hydrogen atom or a C₁₋₄ alkyl group (preferably, methyl group);
R⁷ to R¹¹ are each independently a hydrogen atom or a hydroxyl group; and
n is 0 or 1.

The compound represented by the formula (II-3) is preferably a compound wherein
R¹ to R⁵ are each a hydrogen atom;
R⁶ is a C₁₋₄ alkyl group (preferably, methyl group);
Ra and Rb are each a hydrogen atom;
T is -NH-CH₂- or -N=CH-; and
R⁷ to R¹¹ are each independently a hydrogen atom, a hydroxyl group, a carboxyl group, a C₁₋₄ alkoxy group (preferably, methoxy group), or a C₁₋₄ alkoxy-carbonyl group (preferably, methoxycarbonyl group).

The compound represented by the formula (II-4) is preferably a compound wherein
R¹ to R⁵ is are each independently a hydrogen atom, a hydroxyl group or a C₁₋₄ alkoxy group (preferably, methoxy group); Rc is a hydrogen atom; and
R²³ to R¹⁷ are each independently a hydrogen atom or a hydroxyl group.

Examples of the edible salt of compound (I) (a compound represented by the formula (I); including compounds represented by (II-1) to (II-4)) include salts with inorganic acid, salts with organic acid, salts with inorganic base, salts with organic base, salts with acidic or basic amino acid, and the like.

Examples of the salt with inorganic acid include hydrochloride, hydrobromide, sulfate, nitrate, phosphate and the like.

Examples of the salt with organic acid include formate, acetate, trifluoroacetate, maleate, tartrate, citrate, fumarate, methanesulfonate, benzenesulfonate, p-toluenesulfonate and the like.

Examples of the salt with inorganic base include sodium salt, potassium salt, calcium salt, magnesium salt, ammonium salt and the like.

Examples of the salt with organic base include salts with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine and the like.

Examples of the salt with acidic or basic amino acid include salts with aspartic acid, glutamic acid, arginine, lysine and ornithine.

When compound (I) or a salt thereof (hereinafter sometimes to be abbreviated as the compound of the present invention) contains an optical isomer, a stereoisomer, a regioisomer or a rotamer, either isomer and a mixture thereof are also encompassed in the compound of the present invention. For example, when the compound of the present invention contains an optical isomer, an optical isomer resolved from racemate is also encompassed in the compound of the present invention. These isomers can be obtained as a single product according to synthesis methods known per se, separation methods known per se (e.g., concentration, solvent extraction, column chromatography, recrystallization, etc.), optical resolution methods known *per se* (e.g., fractional recrystallization, chiral column method, diastereomer method etc.) and the like.

In one embodiment of the present invention, the compound of the present invention can be added to foods or drinks (including various edible solid compositions, liquid compositions, seasonings and the like, hereinafter the same). While the food or drink is not particularly limited, examples thereof include seasonings (e.g., miso, soy sauce, baster, dashi (Japanese soup), dressing, mayonnaise, tomato ketchup etc.), soups (e.g., miso soup, Japanese-style soup *(osuimono),* consomme soup, egg soup, seaweed soup, potage etc.), sauces for soba, wheat noodles (udon), ramen, pasta and the like, sauces, foods of cooked rice (e.g., rice gruel, rice soup, ochazuke etc.), livestock processed products (e.g., ham, sausage, cheese etc.), confectionery and snack foods (e.g., potato chips, Japanese cracker, cookie etc.), cooked foods (e.g., boiled food, fried food, roasted food, curry etc.), drinks and the like.

The amount of the compound of the present invention to be added to a food or drink is not particularly limited as long as the effect thereof can be exhibited. However, since a food or drink is mixtures with various substances, the amount of the compound of the present invention that achieves a salty taste enhancing effect may vary from the amount that shows a salty taste enhancing effect using simple brine and the like. Therefore, the amount of the compound of the present invention to be added to a food or drink can be determined by appropriately examining the optimal amount for each food and drink. For example, 0.000001 to 0.1 wt% is preferable.

The compound of the present invention can be used in combination with a known salty taste alternative. Examples of the salty taste alternative include potassium chloride, organic acid, arginine, argininate, ammonium chloride and the like. These may be used alone or two or more kinds thereof may be used as a mixture.

When the compound of the present invention is added to a food or drink, the food or drink produced may contain a suitable additive to the extent that the salty taste enhancing effect is not prevented. For example, such food or drink can contain, in addition to the compound of the present invention, various additives generally usable for the production of a food or drink, such as protein (milk protein, soybean protein etc.), inorganic salt, acid, amino acids, nucleic acid taste components, saccharides, fats, natural seasonings, spices, excipients, dye components and the like.

Examples of the inorganic salt include potassium chloride, ammonium chloride, magnesium sulfate and the like.

Examples of the acid include carboxylic acids such as ascorbic acid, fumaric acid, malic acid, tartaric acid, citric acid, lactic acid, succinic acid and the like, salts thereof and the like.

Examples of the amino acids include glutamates (e.g., sodium glutamate, potassium glutamate, calcium glutamate, ammonium glutamate, magnesium glutamate etc.), glutamic acid and the like. These have already been used as flavor enhancers for food, and all of them have umami taste derived from glutamic acid and taste property (e.g., sour taste derived from ammonium salt, etc.) characteristic of each cation. In addition, basic amino acids (e.g., lysine, arginine, histidine etc.) and salts thereof can also be used as amino acids.

Examples of the nucleic acid taste component include sodium inosinate, sodium guanylate and the like.

Examples of the saccharide include sucrose, glucose, lactose and the like.

The compound of the present invention preferably contains one or more kinds of additives selected particularly from organic acids, arginine, argininate, ammonium chloride and potassium chloride, which are known to have a salty taste enhancing effect.

The production method of the compound represented by the formula (I) or a salt thereof is not particularly limited, and the compound can be produced by a combination of known methods. Specifically, it can be synthesized according to the following method, which is not to be construed as limitative.

In the following production methods, "equivalent" mean molar equivalent.

The outline of the synthesis method is shown below. In the compound represented by the formula (I), a compound wherein p is 1 and Rb' is a hydrogen atom (hereinafter to be referred to as compound (I-a)) and a compound wherein p is 1 and Ra' is a hydrogen atom (hereinafter to be referred to as compound (I-a')) can be produced by the following production method 1.

### Production method 1

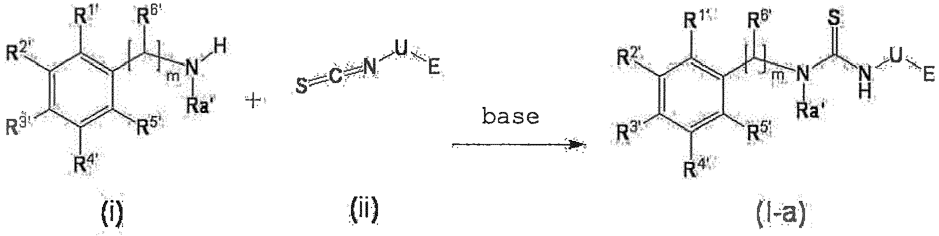

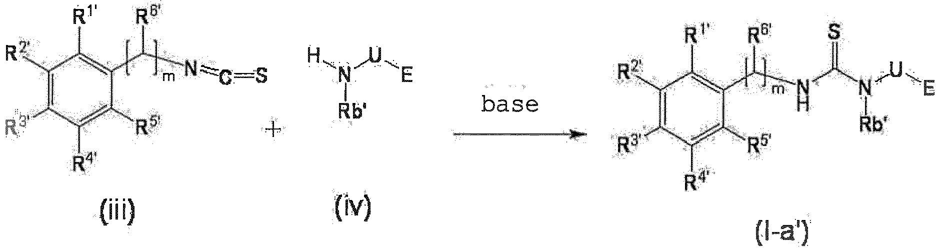

wherein each symbol is as defined above.

Compound (I-a) can be produced by reacting amine compound (i) with isothiocyanate compound (ii), and compound (I-a') can be produced by reacting amine compound (iv) with isothiocyanate compound (iii).

The amine compounds (i) and (iv) may be salts such as hydrochloride, hydrobromide and the like. The amine compound (i) and isothiocyanate compound (ii), or the amine compound (iv) and isothiocyanate compound (iii) may be reacted in the presence of a base such as triethylamine (TEA), sodium hydroxide, potassium hydroxide, N-methylmorpholine, N-methylpiperidine, N,N-diisopropylethylamine and the like. While the ratio of the amine compound (i) and isothiocyanate compound (ii), or the amine compound (iv) and isothiocyanate compound (iii) to be used is not limited, to perform a reaction in good yield, 0.7 to 2.0 equivalents, preferably 0.8 to 1.2 equivalents, of the isothiocyanate compound (ii) or (iii) may be used relative to the amine compound (i) or (iv), respectively. The amount of the base to be used is 1.0 to 5.0 equivalents, preferably 2.0 to 3.0 equivalents, relative to the amine compound (i) or (iv).

The solvent to be used is not particularly limited as long as it does not react with the amine compound (i) or (iv), and the isothiocyanate compound (ii) or (iii) and, for example, N,N-dimethylformamide (DMF), tetrahydrofuran (THF), dichloromethane (DCM), chloroform, dimethyl sulfoxide (DMSO), N-methylpyrrolidone (NMP), a mixed solvent thereof, or a mixed solvent of these and water can be used. Of these, a mixed solvent of dichloromethane, N,N-dimethylformamide, tetrahydrofuran and water is preferable. The amount of the solvent is 1 to 100-fold weight, preferably 10 to 30-fold weight, relative to the amine compound (i) or (iv).

The reaction time is 1 to 50 hr, preferably 3 to 24 hr. This depends on the reaction temperature, and the temperature range is 0 to 50°C, preferably 5 to 35°C.

In the compound represented by the formula (I), a compound wherein p is 1, U is -N=CH-, and Ra' and Rb' are both hydrogen atoms (hereinafter to be referred to as compound (I-b)) can be produced by the following production method 2.

### Production method 2

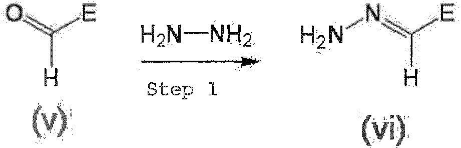

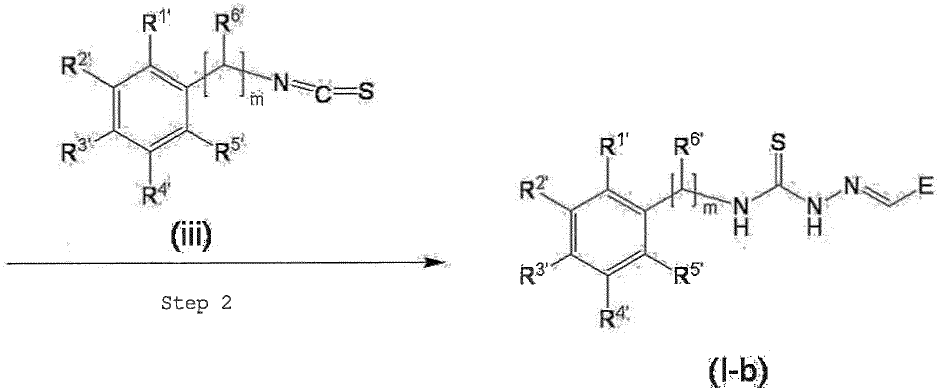

wherein each symbol is as defined above.

### step 1

Hydrazone compound (vi) can be obtained by reacting aldehyde compound (v) with hydrazine. While the ratio of the aldehyde compound (v) and hydrazine is not limited, to perform a reaction in good yield, the amount of the hydrazine to be used is 0.8 to 10.0 equivalents, preferably 3.0 to 5.0 equivalents, relative to the aldehyde compound (v).

The solvent to be used is not particularly limited as long as it does not react with the aldehyde compound (v) and hydrazine and, for example, methanol, ethanol, N,N-dimethylformamide (DMF), dichloromethane (DCM), or a mixed solvent thereof can be used. Of these, methanol or ethanol is preferable. The amount of the solvent is 1- to 100-fold weight, preferably 5- to 20-fold weight, relative to the aldehyde compound (v).

The reaction time is 1 to 50 hr, preferably 3 to 24 hr. This depends on the reaction temperature, and the temperature range is 0 to 50°C, preferably 5 to 35°C.

### step 2

Compound (I-b) can be obtained by reacting hydrazone compound (vi) with isothiocyanate compound (iii).

The hydrazone compound (vi) and isothiocyanate compound (iii) may be reacted in the presence of a base such as triethylamine (TEA), sodium hydroxide, potassium hydroxide, N-methylmorpholine, N-methylpiperidine, N,N-diisopropylethylamine and the like. While the ratio of the hydrazone compound (vi) and isothiocyanate compound (iii) is not limited, to perform a reaction in good yield, 0.8 to 1.2 equivalents of the isothiocyanate compound (iii) may be used relative to the hydrazone compound (vi). The amount of the base to be used is 1.0 to 5.0 equivalents, preferably 2.0 to 3.0 equivalents, relative to the hydrazone compound (vi). Examples of the solvent to be used include those used in the above-mentioned Production Method 1.

The reaction time is 1 to 50 hr, preferably 3 to 24 hr. This depends on the reaction temperature, and the temperature range is 0 to 50°C, preferably 5 to 35°C.

In addition, compound (I-b) can also be produced by the following production method 3.

### Production method 3

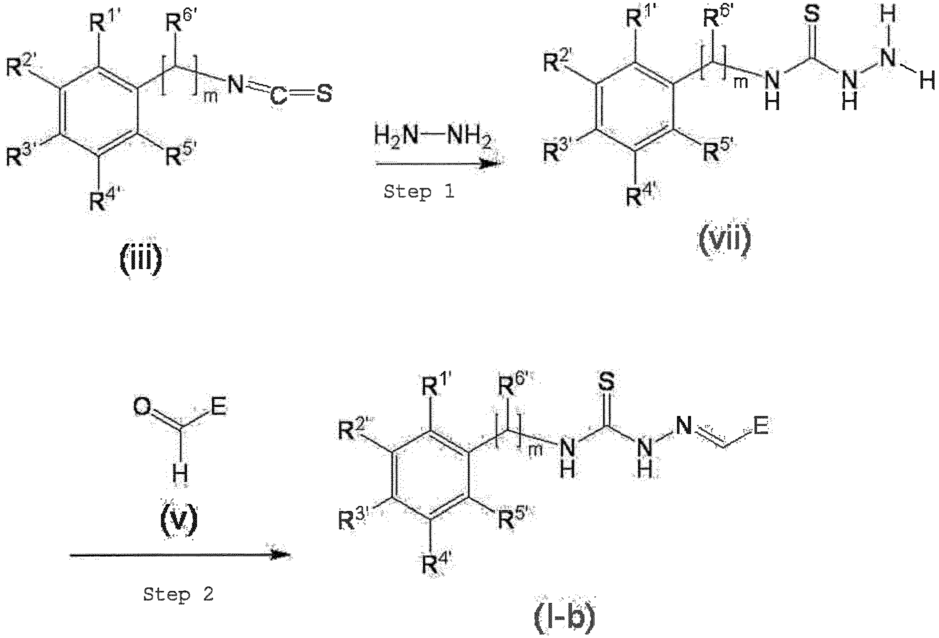

wherein each symbol is as defined above.

### step 1

Thiosemicarbazide compound (vii) can be obtained by reacting isothiocyanate compound (iii) with hydrazine. While the ratio of the isothiocyanate compound (iii) and hydrazine is not limited, to perform a reaction in good yield, the amount of the hydrazine to be used is 0.8 to 5.0 equivalents, preferably 1.1 to 3.0 equivalents, relative to the isothiocyanate compound (iii).

The solvent to be used is not particularly limited as long as it does not react with the isothiocyanate compound (iii) and hydrazine and, for example, methanol, ethanol, N,N-dimethylformamide (DMF), dichloromethane (DCM), or a mixed solvent thereof can be used. Of these, methanol or ethanol is preferable. The amount of the solvent is 1 to 100-fold weight, preferably 3 to 20-fold weight, relative to the isothiocyanate compound (iii).

The reaction time is 1 to 50 hr, preferably 3 to 24 hr.
This depends on the reaction temperature, and the temperature range is 30 to 120°C, preferably 60 to 90°C.

### step 2

Compound (I-b) can be obtained by reacting thiosemicarbazide compound (vii) with aldehyde compound (v). While the ratio of the thiosemicarbazide compound (vii) and aldehyde compound (v) is not limited, to perform a reaction in good yield, the amount of the aldehyde compound (v) to be used is 0.8 to 5.0 equivalents, preferably 1.1 to 3.0 equivalents, relative to the thiosemicarbazide compound (vii).

The solvent to be used is not particularly limited as long as it does not react with the thiosemicarbazide compound (vii) and aldehyde compound (v) and, for example, ethanol, isopropanol, N,N-dimethylformamide (DMF), chloroform, or a mixed solvent thereof can be used. Of these, ethanol or isopropanol is preferable. The amount of the solvent is 1 to 100-fold weight, preferably 3 to 20-fold weight, relative to the thiosemicarbazide compound (vii).

The reaction time is 1 to 50 hr, preferably 3 to 24 hr. This depends on the reaction temperature, and the temperature range is 0 to 50°C, preferably 5 to 35°C.

In addition, a desired compound can be obtained by performing an introduction or conversion reaction of each substituent where necessary by a method known per se, before and after the reactions of the above-mentioned production methods 1 to 3 and the following production methods 4 and 5.

For example, a compound wherein p is 1 and U is -N=CH-(hereinafter to be referred to as compound (I-b')) can be produced by introducing, where necessary, by a method known per se, Ra' and/or Rb' into compound (I-b) obtained in the above-mentioned production method 2 or 3.

In the compound represented by the formula (I), a compound wherein p is 1 and U is -NH-CH₂- (hereinafter to be referred to as compound (I-c)) can be produced by the following production method 4.

### Production method 4

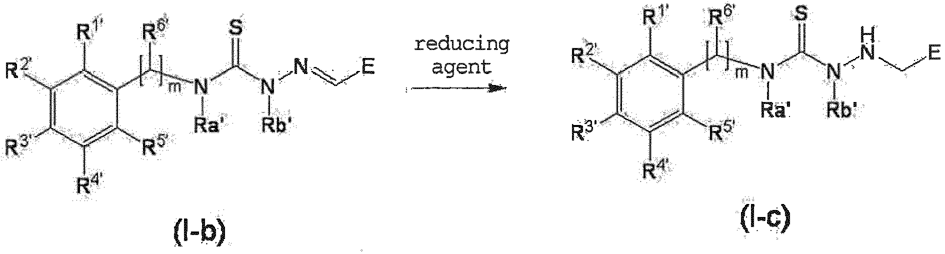

wherein each symbol is as defined above.

Compound (I-c) can be produced by reducing compound (I-b).

As the reducing agent, sodium borohydride, hydrogen-palladium carbon and the like can be used. Of these, sodium borohydride is preferable. While the amount of the reducing agent to be used is not limited, to perform a reaction in good yield, the amount of the reducing agent to be used is 5 to 50 equivalents, preferably 20 to 30 equivalents, relative to the compound (I-b).

The solvent to be used is not particularly limited and, for example, methanol, ethanol, isopropanol, or a mixed solvent thereof can be used. Of these, methanol is preferable. The amount of the solvent is 1 to 100-fold weight, preferably 3 to 20-fold weight, relative to the compound (I-b).

The reaction time is 1 to 50 hr, preferably 3 to 24 hr. This depends on the reaction temperature, and the temperature range is 0 to 50°C, preferably 5 to 35°C.

In the compound represented by the formula (I), a compound wherein p is 0 (hereinafter to be referred to as compound (I-d)) can be produced by the following production method 5.

### Production method 5

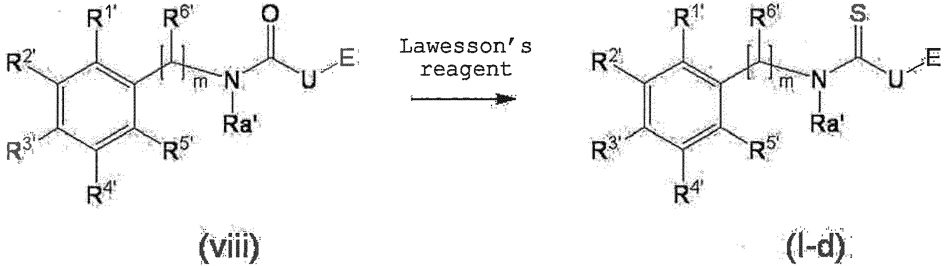

wherein each symbol is as defined above.

Compound (I-d) can be produced by reacting amide compound (viii) with a Lawesson's reagent. The amount of the Lawesson's reagent to be used is 1.1 to 5.0 equivalents, preferably 2.0 to 3.0 equivalents, relative to the amide compound (viii).

Examples of the solvent to be used include hydrocarbons such as benzene, toluene, xylene and the like, halogenated hydrocarbons such as chlorobenzene and the like, ethers such as diethyl ether, diisopropyl ether, dimethoxyethane, dioxane, tetrahydrofuran and the like, and the like, and a mixed solvent thereof.

The reaction time is 1 to 50 hr, preferably 3 to 24 hr. This depends on the reaction temperature, and the temperature range is 0 to 150°C, preferably 50 to 100°C.

In each of the above-mentioned reaction, when the starting compound has an amino group, a carboxyl group, a hydroxy group or a carbonyl group as a substituent, a protecting group generally used in peptide chemistry and the like may be introduced into these groups. By removing the protecting group as necessary after the reaction, the objective compound can be obtained.

The removal of the above-mentioned protecting group can be performed according to a known method, for example, the method described in Protective Groups in Organic Synthesis, John Wiley and Sons (1980) or and the like.

The obtained compound represented by the formula (I) or a salt thereof can be isolated and purified according to a conventional method. For example, when it is purified by crystallization, ethyl acetate, ethanol, methanol, diethyl ether, chloroform, dichloromethane, n-hexane and a mixed solvent thereof can be used as a solvent. Preparative thin layer chromatography (PTLC) or silica gel column chromatography can be employed as purification using chromatography. In this case, solvents exemplified for the above-mentioned crystallization can be used as an eluent.

### Examples

The utility of the present invention is specifically explained below by referring to Examples and Experimental Examples, which is not to be construed as limitative. The compounds of Examples 1 to 9 and 11 to 127 described in Tables 1-1 to 1-24 were produced according to the methods of the representative Examples mentioned below. The structures of the compounds synthesized in the following Examples were determined by nuclear magnetic resonance spectrum (Bruker AVANCE 400) and ESI-MS spectrum.

**[Table 1-1]**

| Ex. No. | structural formula | property values |
|---|---|---|
| 1 | 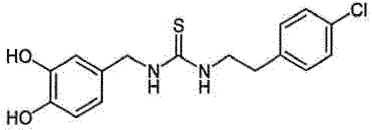 | ¹H NMR (CDCl₃) δ= 2.78 (t, *J*= 6.9 Hz, 2H), 3.69 (bs, 2H), 4.29 (bs, 2H), 5.89 (bs, 1H), 6.42 (bs, 1H), 6.53-6.56 (m, 1H), 6.70 (s, 1H), 6.77 (d, *J*= 8.2 Hz, 2H), 7.02 (d, *J*= 8.2 Hz, 2H), 7.20-7.23 (m, 2H) |
| | | MS(ESI) m/z: 337.1 (M+1) |
| | | yield: 79% |
| 2 | 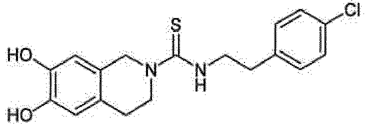 | ¹H NMR (CD₃OD) δ= 2.75 (t, J= 5.9 Hz, 2H), 2.95 (t, *J*= 7.5 Hz, 2H), 3.82-3.86 (m, 2H), 3.91 (t, *J*= 5.9 Hz, 2H), 4.71 (s, 2H), 6.58 (s, 1H), 6.61 (s, 1H), 7.21-7.28 (m, 4H) |
| | | MS(ESI) m/z: 363.1(M+1) |
| | | yield: 82% |
| 3 | 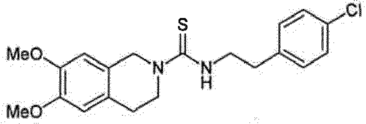 | ¹H NMR (CD₃OD) δ= 2.83 (t, J= 5.9 Hz, 2H), 2.96 (t, *J*= 7.4 Hz, 2H), 3.82 (s, 6H), 3.83-3.86 (m, 2H), 3.96 (t, *J*= 5.9 Hz, 2H), 4.79 (s, 2H), 6.72 (s, 1H), 6.79 (s, 1H), 7.20-7.27 (m, 4H) |
| | | MS (ESI) m/z: 389.0 (M-1) |
| | | yield: 79% |
| 4 | 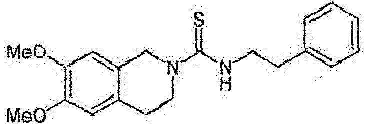 | ¹H NMR (CD₃OD) δ= 2.84 (t, *J*= 5.9 Hz, 2H), 2.97 (t, *J*= 7.6 Hz, 2H), 3.83 (s, 6H), 3.83-3.88 (m, 2H), 3.97 (t, *J*= 5.9 Hz, 2H), 4.81 (s, 2H), 6.74 (s, 1H), 6.79 (s, 1H), 7.19-7.28 (m, 5H) |
| | | MS (ESI) m/z: 356.8 (M+1) |
| | | yield: 88% |
| 5 | 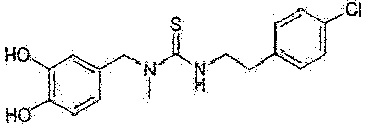 | ¹H NMR (CD₃OD) δ= 2.94 (t, *J*= 7.4 Hz, 2H), 2.99 (s, 3H), 3.83 (t, *J*= 7.4 Hz, 2H), 4.93 (s, 2H), 6.57-6.59 (m, 1H), 6.73-6.75 (m, 2H), 7.21-7.29 (m, 4H) |
| | | MS(ESI) m/z: 350.9 (M+1) |
| | | yield: 47% |

**[Table 1-2]**

| Ex. No. | structural formula | property values |
|---|---|---|
| 6 | 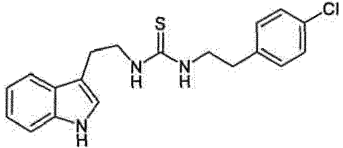 | ¹H NMR (CD₃OD) δ= 2.81 (t, *J*= 7.2 Hz, 2H), 3.02 (t, *J*= 7.0 Hz, 2H), 3.55-3.88 (m, 4H), 7.00-7.36 (m, 8H), 7.61-7.63 (m, 1H) |
| | | MS(ESI) m/z: 358.1(M+1) |
| | | yield: quant. |
| 7 | 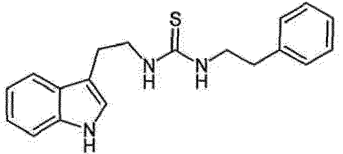 | ¹H NMR (CD₃OD) δ= 2.83 (t, *J*= 7.3 Hz, 2H), 3.02 (t, *J*= 7.1 Hz, 2H), 3.58-3.88 (m, 4H), 7.00-7.36 (m, 9H), 7.61-7.63 (m, 1H) |
| | | MS(ESI) m/z: 324.2(M+1) |
| | | yield: 91% |
| 8 | 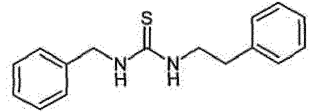 | ¹H NMR (CD₃OD) δ= 2.89 (t, *J*= 7.3 Hz, 2H), 3.75 (bs, 2H), 4.70 (bs, 2H), 7.20-7.33 (m, 10H) |
| | | MS(ESI) m/z: 271.1(M+1) |
| | | yield: 86% |
| 9 | 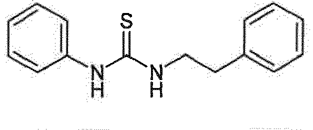 | ¹H NMR (CD₃OD) δ= 2.94 (t, *J*= 7.3 Hz, 2H), 3.79-3.88 (m, 2H), 7.18-7.34 (m, 10H) |
| | | MS(ESI) m/z: 257.1(M+1) |
| | | yield: 76% |
| 11 | 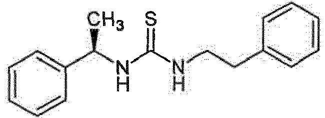 | ¹H NMR (CD₃OD) δ= 1.47 (d, *J*= 6.9 Hz, 3H), 2.82-2.86 (m, 2H), 3.68-3.80 (m, 2H), 5.39 (bs, 1H), 7.18-7.34 (m, 10H) |
| | | MS(ESI) m/z: 284.7(M+1) |
| | | yield: 90% |
| 12 | 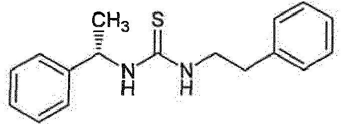 | ¹H NMR (CD₃OD) δ= 1.47 (d, J= 6.9 Hz, 3H), 2.82-2.86 (m, 2H), 3.68-3.80 (m, 2H), 5.39 (bs, 1H), 7.18-7.34 (m, 10H) |
| | | MS(ESI) m/z: 284.7(M+1) |
| | | yield: 94% |

**[Table 1-3]**

| Ex. No. | structural formula | property values |
|---|---|---|
| 13 | 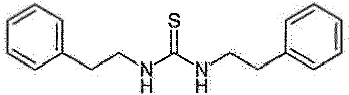 | ¹H NMR (CD₃OD) δ= 2.87 (d, *J*= 7.3 Hz, 4H), 3.68 (bs, 4H), 7.18-7.31 (m, 10H) |
| | | MS (ESI) m/z: 285.0(M+1) |
| | | yield: quant. |
| 14 | 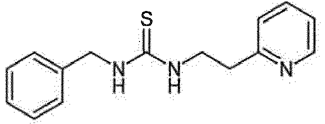 | ¹H NMR (CD₃OD) δ= 3.06 (t, *J*= 7.0 Hz, 2H), 3.86 (bs, 2H), 4.70 (bs, 2H), 7.22-7.33 (m, 7H), 7.70-7.74 (m, 1H), 8.42-8.43 (m, 1H) |
| | | MS(ESI) m/z: 272.4(M+1) |
| | | yield: quant. |
| 15 | 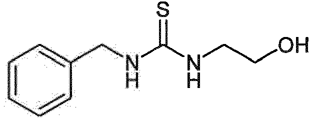 | ¹H NMR (CD₃OD) δ= 3.63 (bs, 4H), 4.73 (bs, 2H), 7.24-7.34 (m, 5H) |
| | | MS(ESI) m/z: 210.8(M+1) |
| | | yield: quant. |
| 16 | 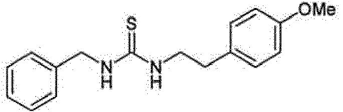 | ¹H NMR (CD₃OD) δ= 2.82 (t, *J*= 7.2 Hz, 2H), 3.70 (bs, 2H), 3.76 (s, 3H), 4.69 (bs, 2H), 6.84 (d, *J*= 8.6 Hz, 2H), 7.14 (d, *J*= 8.6 Hz, 2H), 7.25-7.32 (m, 5H) |
| | | MS (ESI) m/z: 301.1(M+1) |
| | | yield: quant. |
| 17 | 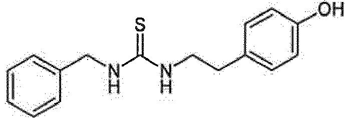 | ¹H NMR (CD₃OD) δ= 2.79 (t, *J*= 7.2 Hz, 2H), 3.70 (bs, 2H), 4.69 (bs, 2H), 6.72 (d, *J*= 8.4 Hz, 2H), 7.05 (d, *J*= 8.4 Hz, 2H), 7.25-7.33 (m, 5H) |
| | | MS(ESI) m/z: 286.9(M+1) |
| | | yield: 60% |
| 18 | 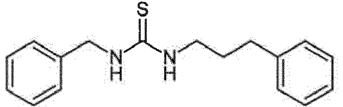 | ¹H NMR (CD₃OD) δ= 1.86-1.90 (m, 2H), 2.60-2.64 (m, 2H), 3.49 (bs, 2H), 4.70 (bs, 2H), 7.14-7.33 (m, 10H) |
| | | MS(ESI) m/z: 284.7(M+1) |
| | | yield: 99% |

**[Table 1-4]**

| Ex. No. | structural formula | property values |
|---|---|---|
| 19 | 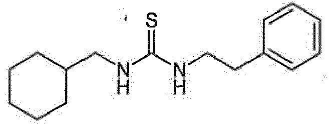 | ¹H NMR (CD₃OD) δ= 0.93-0.96 (m, 2H), 1.21-1.29 (m, 3H), 1.59 (bs, 1H), 1.70-1.76 (m, 5H), 2.88-2.92 (m, 2H), 3.30 (bs, 2H), 3.72 (bs, 2H), 7.20-7.32 (m, 5H) |
| | | MS(ESI) m/z: 276.6(M+1) |
| | | yield: quant. |
| 20 | 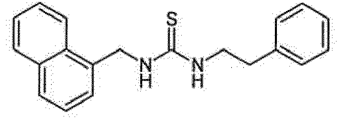 | ¹H NMR (CD₃OD) δ= 2.89 (t, *J*= 7.2 Hz, 2H), 3.78 (bs, 2H), 5.15 (bs, 2H), 7.17-7.25 (m, 5H), 7.44-7.45 (m, 2H), 7.52-7.56 (m, 2H), 7.80-7.93 (m, 2H), 8.06-8.11 (m, 1H) |
| | | MS(ESI) m/z: 320.6(M+1) |
| | | yield: 68% |
| 21 | 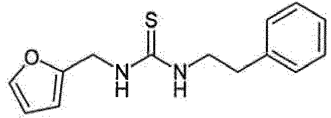 | ¹H NMR (CD₃OD) δ= 2.88 (t, J= 7.3 Hz, 2H), 3.74 (bs, 2H), 4.69 (bs, 2H), 6.26-6.27 (m, 1H), 6.33-6.34 (m, 1H), 7.18-7.31 (m, 5H), 7.39-7.40 (m, 1H) |
| | | MS(ESI) m/z: 261.1(M+1) |
| | | yield: quant. |
| 22 | 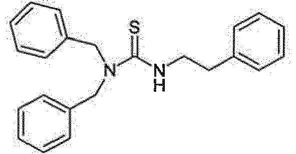 | ¹H NMR (CD₃OD) δ= 2.88-2.90 (m, 2H), 3.85-3.89 (m, 2H), 4.91 (s, 3H), 7.17-7.33 (m, 15H) |
| | | MS (ESI) m/z: 360.8(M+1) |
| | | yield: quant. |
| 23 | 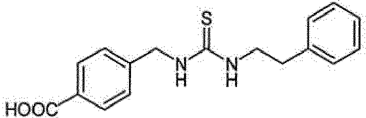 | ¹H NMR (CD₃OD) δ= 2.90 (t, *J*= 7.2 Hz, 2H), 3.75 (bs, 2H), 4.79 (bs, 2H), 7.20-7.30 (m, 5H), 7.39 (d, *J*= 8.2 Hz, 2H), 7.98 (d, *J*= 8.2 Hz, 2H) |
| | | MS(ESI) m/z: 314.9(M+1) |
| | | yield: 60% |

**[Table 1-5]**

| Ex. No. | structural formula | property values |
|---|---|---|
| 24 | 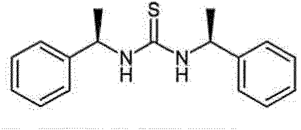 | ¹H NMR (CDCl₃) δ= 1.30 (d, *J*= 6.5 Hz, 6H), 5.05 (bs, 2H), 6.29 (bs, 2H), 7.23-7.35 (m, 10H) |
| | | MS(ESI) m/z: 284.6(M+1) |
| | | yield: 87% |
| 25 | 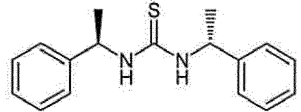 | ¹H NMR (CDCl₃) δ= 1.46 (d, *J*= 6.8 Hz, 6H), 5.07 (bs, 2H), 6.03 (bs, 2H), 7.01 (bs, 4H), 7.22 (bs, 6H) |
| | | MS(ESI) m/z: 285.5(M+1) yield: 60% |
| 26 | 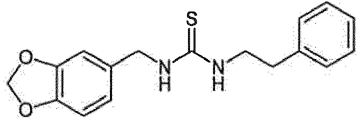 | ¹H NMR (CD₃OD) δ= 2.88 (t, *J*= 7.3 Hz, 2H), 3.73 (bs, 2H), 4.58 (bs, 2H), 5.91 (s, 2H), 6.75 (s, 2H), 6.82 (s, 1H), 7.17-7.30 (m, 5H) |
| | | MS(ESI) m/z: 314.7(M+1) |
| | | yield: quant. |
| 27 | 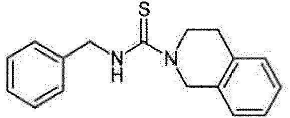 | ¹H NMR (CD₃OD) δ= 2.95 (t, *J*= 6.0 Hz, 2H), 4.04 (t, *J*= 6.0 Hz, 2H), 4.95 (s, 2H), 4.98 (s, 2H), 7.18-7.36 (m, 9H) |
| | | MS(ESI) m/z: 283.1(M+1) |
| | | yield: 76% |
| 28 | 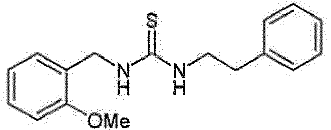 | ¹H NMR (CD₃OD) δ= 2.88 (t, *J*= 7.3 Hz, 2H), 3.74 (bs, 2H), 3.84 (s, 3H), 4.65 (bs, 2H), 6.91-6.97 (m, 2H), 7.19-7.28 (m, 7H) |
| | | MS(ESI) m/z: 300.6(M+1) |
| | | yield: 90% |
| 29 | 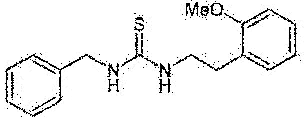 | ¹H NMR (CD₃OD) δ= 2.89 (t, *J*= 7.1 Hz, 2H), 3.69 (bs, 2H), 3.79 (s, 3H), 4.70 (bs, 2H), 6.85-6.93 (m, 2H), 7.12-7.33 (m, 7H) |
| | | MS(ESI) m/z: 300.6(M+1) |
| | | yield: quant. |

**[Table 1-6]**

| Ex. No. | structural formula | property values |
|---|---|---|
| 30 | 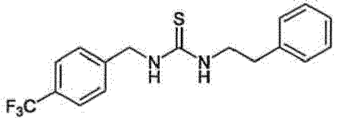 | ¹H NMR (CD₃OD) δ= 2.89-2.92 (m, 2H), 3.79 (bs, 2H), 4.81 (bs, 2H), 7.20-7.29 (m, 5H), 7.46-7.48 (m, 2H), 7.61-7.63 (m, 2H) |
| | | MS(ESI) m/z: 338.8(M+1) |
| | | yield: 54.0% |
| 31 | 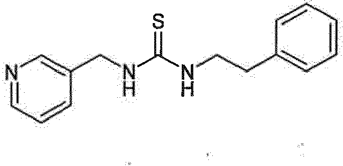 | ¹H NMR (CD₃OD) δ= 2.90 (t, *J*= 7.3 Hz, 2H), 3.73 (bs, 2H), 4.79 (s, 2H), 7.18-7.31 (m, 5H), 7.38-7.41 (m, 1H), 7.79-7.81 (m, 1H), 8.42-8.43 (m, 1H), 8.51 (s, 1H) |
| | | MS (ESI) m/z: 270.0 (M-1) |
| | | yield: quant. |
| 32 | 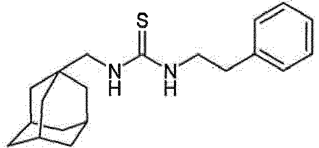 | ¹H NMR (CD₃OD) δ= 1.55 (bs, 7H), 1.68-1.71 (m, 3H), 1.76-1. 79 (m, 3H), 1.98 (bs, 3H), 2.89-2.93 (m, 2H), 3.13-3.31 (m, 2H), 3.72-3.80 (m, 2H), 7.21-7.32 (m, 1H) |
| | | MS(ESI) m/z: 329.1(M+1) |
| | | yield: 92% |
| 33 | 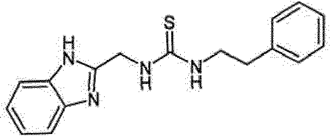 | ¹H NMR (CDCl₃) δ= 2.84 (d, *J*= 7.1 Hz, 6H), 3.73 (bs, 2H), 4.92 (bs, 2H), 7.13-7.57 (m, 9H) |
| | | MS(ESI) m/z: 311.2(M+1) |
| | | yield: 27% |
| 34 | 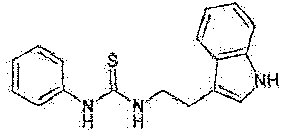 | ¹H NMR (CD₃OD) δ= 3.08 (t, *J*= 7.1 Hz, 2H), 3.88-3.91 (m, 2H), 7.02-7.66 (m, 10H) |
| | | MS (ESI) m/z: 296.0(M+1) |
| | | yield: 95% |

**[Table 1-7]**

| Ex. No. | structural formula | property values |
|---|---|---|
| 35 | 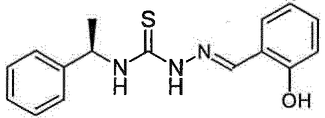 | ¹H NMR ((CD₃)₂SO) δ= 1.56 (d, *J*= 7.0 Hz, 3H), 5.72-5.76 (m, 1H), 6.83-6.89 (m, 2H), 7.21-7.44 (m, 6H), 7.95-7.97 (m, 1H), 8.43 (s, 1H), 8.53-8.56 (m, 1H), 9.94 (bs, 1H), 11.5 (bs, 1H) |
| | | MS (ESI) m/z: 299.9(M+1) |
| | | yield: 59% |
| 36 | 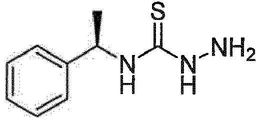 | ¹H NMR ((CD₃)₂SO) δ= 1.46 (d, *J*= 7.0 Hz, 3H), 4.54 (s, 2H), 5.50-5.63 (m, 1H), 7.22-7.38 (m, 5H), 8.00-8.02 (m, 1H), 8.72 (bs, 1H) |
| | | MS (ESI) m/z: 194.2(M-1) |
| | | yield: quant. |
| 37 | 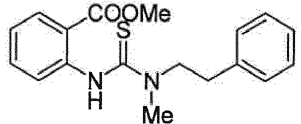 | ¹H NMR (CDCl₃) δ= 3.09-3.13 (m, 2H), 3.33 (s, 3H), 3.94 (s, 3H), 4.12-4.16 (m, 2H), 7.09-7.58 (m, 6H), 7.99-8.02 (m, 1H), 8.84-8.86 (m, 1H), 10.9 (bs, 1H) |
| | | MS(ESI) m/z: 329.1 (M+1) |
| | | yield: quant. |
| 38 | 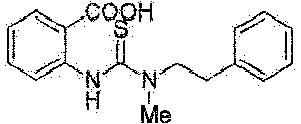 | ¹H NMR (CD₃OD) δ= 3. 07 (t, *J*= 7.8 Hz, 2H), 3.28 (s, 3H), 4.08-4.12 (m, 2H), 7.11-7.54 (m, 7H), 8.04-8.06 (m, 1H), 8.68-8.70 (m, 1H) |
| | | MS (ESI) m/z: 315.2 (M+1) |
| | | vield: 87% |
| 39 | 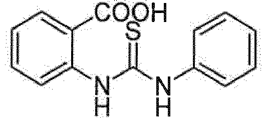 | ¹H NMR ((CD₃)₂SO) δ= 7.16-7.57 (m, 7H), 7.89-7.91 (m, 1H), 8.46-8.48 (m, 1H), 10.51 (bs, 1H), 10.8 (bs, 1H) |
| | | MS (ESI) m/z: 273.0 (M+1) |
| | | yield: 14% |

**[Table 1-8]**

| Ex. No. | structural formula | property values |
|---|---|---|
| 40 | 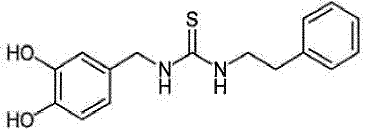 | ¹H NMR (CDCl₃) δ= 2.73-2.77 (m, 2H), 3.63 (bs, 2H), 4.22 (bs, 2H), 6.45-7.26 (m, 8H) |
| | | MS(ESI) m/z: 303.1(M+1) |
| | | yield: 81% |
| 41 | 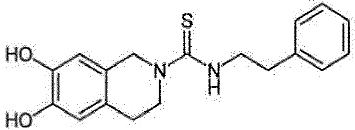 | ¹H NMR (CD₃OD) δ= 2.73-2.76 (m, 2H), 2.94-2.97 (m, 2H), 3.83-3.93 (m, 2H), 4.71 (s, 2H), 6.57 (s, 1H), 6.61 (s, 1H), 7.19-7.30 (m, 4H) |
| | | MS(ESI) m/z: 329.1(M+1) |
| | | yield: 89% |
| 42 | 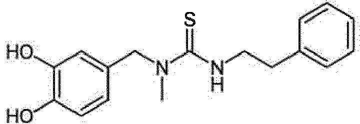 | ¹H NMR (CD₃OD) δ= 2.93-2.97 (m, 2H), 2.98 (s, 3H), 3.82-3.86 (m, 2H), 4.93 (s, 2H), 6.57-6.60 (m, 1H), 6.72-6.75 (m, 2H), 7.19-7.30 (m, 5H) |
| | | MS(ESI) m/z: 316.9(M+1) |
| | | yield: 38% |
| 43 | 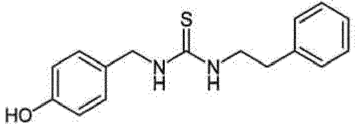 | ¹H NMR (CD₃OD) δ= 2.88 (t, *J*= 7.3 Hz, 2H), 3.73 (bs, 2H), 4.55 (bs, 2H), 6.74-6.77 (m, 2H), 7.12-7.30 (m, 7H) |
| | | MS(ESI) m/z: 287.2(M+1) |
| | | yield: 89% |
| 44 | 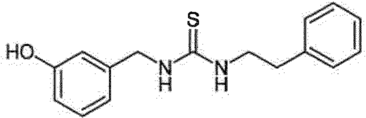 | ¹H NMR (CD₃OD) δ= 2.89 (t, *J*= 7.3 Hz, 2H), 3.74 (bs, 2H), 4.61 (bs, 2H), 6.68-6.77 (m, 3H), 7.12-7.31 (m, 6H) |
| | | MS(ESI) m/z: 287.1(M+1) |
| | | yield: 87% |
| 45 | 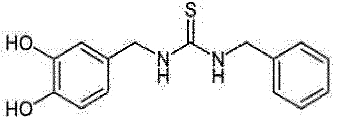 | ¹H NMR (CD₃OD) δ= 4.55 (bs, 2H), 4.74 (bs, 2H), 6.63-6.79 (m, 3H), 7.24-7.31 (m, 5H) |
| | | MS (ESI) m/z: 289.1(M+1) |
| | | yield: 81% |

**[Table 1-9]**

| Ex. No. | structural formula | property values |
|---|---|---|
| 46 | 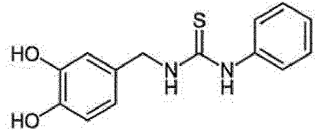 | ¹H NMR (CD₃OD) δ= 4.87 2H), 6.67-6.84 (m, 3H), 7.18-7.22 (m, 1H), 7.33-7.38 (m, 4H) |
| | | MS(ESI) m/z: 275.1(M+1) |
| | | yield: 97% |
| 47 | 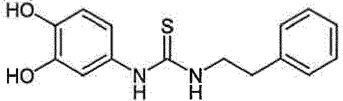 | ¹H NMR (CD₃OD) δ= 2.88 (t, *J*= 7.3 Hz, 2H), 3.77 (t, *J*= 7.3 Hz, 2H), 6.42-6.45 (m, 1H), 6.63-6.64 (m, 1H), 6.72-6.74 (m, 1H), 7.20-7.30 (m, 5H) |
| | | MS (ESI) m/z: 289.1(M+1) |
| | | yield: 58% |
| 48 | 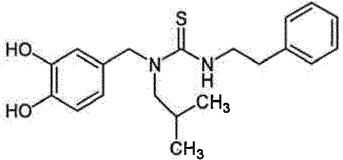 | ¹H NMR (CD₃OD) δ= 0.88 (d, *J*= 6.7 Hz, 6H), 2.01-2.12 (m, 1H), 2.88 (t, *J*= 7.3 Hz, 2H), 3.37-3.39 (m, 2H), 3.82-3.86 (m, 2H), 4.80-4.98 (m, 2H), 6.53-6.54 (m, 1H), 6.69-6.73 (m, 2H), 7.18-7.26 (m, 5H) |
| | | MS(ESI) m/z: 358.9(M+1) |
| | | yield: 81% |
| 49 | 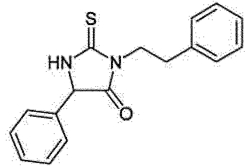 | ¹H NMR (CDCl₃) δ= 3.00-3.04 (m, 2H), 4.05-4.09 (m, 2H), 5.02 (s, 1H), 7.18-7.39 (m, 10H) |
| | | MS(ESI) m/z: 295.1(M-1) |
| | | yield: 21% |
| 50 | 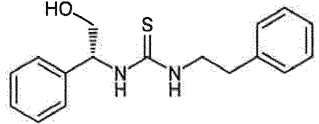 | ¹H NMR (CD₃OD) δ= 2.83-2.87 (m, 2H), 3.71-3.80 (m, 4H), 5.38 (bs, 1H), 7.17-7.37 (m, 10H) |
| | | MS(ESI) m/z: 301.1(M+1) |
| | | yield: quant. |
| 51 | 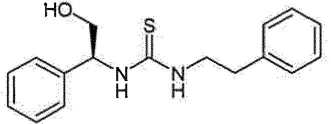 | ¹H NMR (CD₃OD) δ= 2.83-2.87 (m, 2H), 3.73-3.80 (m, 4H), 5.38 (bs, 1H), 7.19-7.37 (m, 10H) |
| | | MS(ESI) m/z: 301.1(M+1) |
| | | yield: 92% |

**[Table 1-10]**

| Ex. No. | structural formula | property values |
|---|---|---|
| 52 | 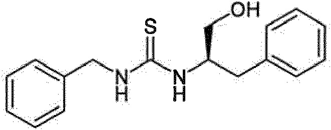 | ¹H NMR (CD₃OD) δ= 2.91-2.93 (m, 2H), 3.57-3.59 (m, 2H), 4.50-4.78 (m, 3H), 7.19-7.33 (m, 10H) |
| | | MS(ESI) m/z: 300.8(M+1) |
| | | yield: 97% |
| 53 | 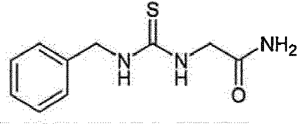 | ¹H NMR (CD₃OD) δ= 4.24 (s, 2H), 4.73 (bs, 2H), 7.26-7.35 (m, 5H) |
| | | MS(ESI) m/z: 224.1(M+1) |
| | | yield: 43% |
| 54 | 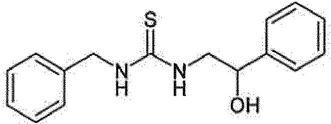 | ¹H NMR (CD₃OD) δ= 3.32-3.34 (m, 1H), 3.90 (bs, 1H), 4.73 (bs, 2H), 4.89-4.98 (m, 1H), 7.26-7.42 (m, 10H) |
| | | MS(ESI) m/z: 286.9(M+1) |
| | | yield: quant. |
| 55 | 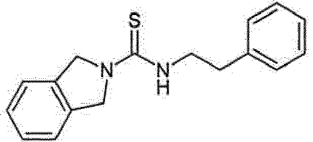 | ¹H NMR (CD₃OD) δ= 2.99 (t, *J*= 7.6 Hz, 2H), 3.86 (t, *J*= 7.6 Hz, 2H), 4.85-4.92 (m, 4H), 7.19-7.35 (m, 9H) |
| | | MS (ESI) m/z: 283.1(M+1) |
| | | yield: 71% |
| 56 | 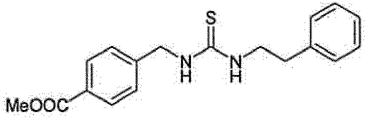 | ¹H NMR (CD₃OD) δ= 2.88-2.92 (m, 2H), 3.74 (bs, 2H), 3.90 (s, 3H), 4.80 (bs, 2H), 7.20-7.40 (m, 7H), 7.96-7.98 (m, 10H) |
| | | MS(ESI) m/z: 329.0(M+1) |
| | | yield: 88% |
| 57 | 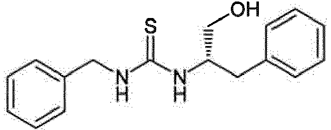 | ¹H NMR (CD₃OD) δ= 2.91-2.93 (m, 2H), 3.57-3.59 (m, 2H), 4.50-4.78 (m, 3H), 7.19-7.33 (m, 10H) |
| | | MS (ESI) m/z: 300.8(M+1) |
| | | yield: 72% |

**[Table 1-11]**

| Ex. No. | structural formula | property values |
|---|---|---|
| 58 | 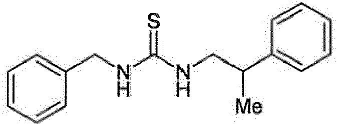 | ¹H NMR (CD₃OD) δ= 1.25 (d, *J*= 7.0 Hz, 3H), 3.08-3.20 (m, 1H), 3.58-3.89 (m, 2H), 4.69 (bs, 2H), 7.20-7.33 (m, 10H) |
| | | MS (ESI) m/z: 282.5(M-1) |
| | | yield: quant. |
| 59 | 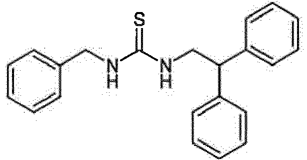 | ¹H NMR (CD₃OD) δ= 4.17 (m, 2H), 4.48-4.66 (m, 3H), 7.22-7.30 (m, 15H) |
| | | MS (ESI) m/z: 346.8(M+1) |
| | | yield: 94% |
| 60 | 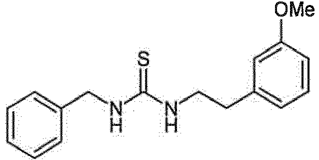 | ¹H NMR (CD₃OD) δ= 2.86 (t, *J*= 7.2 Hz, 2H), 3.69-3.80 (m, 2H), 3.77 (s, 3H), 4.70 (bs, 2H), 6.80-6.82 (m, 3H), 7.17-7.32 (m, 6H) |
| | | MS(ESI) m/z: 300.7(M+1) |
| | | yield: quant. |
| 61 | 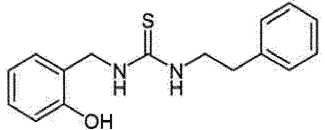 | ¹H NMR (CD₃OD) δ= 2.87-2.91 (m, 2H), 3.73 (bs, 4H), 6.59-7.05 (m, 2H), 7.21-7.33 (m, 7H) |
| | | MS(ESI) m/z: 284.8(M-1) |
| | | yield: 21% |
| 62 | 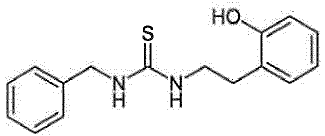 | ¹H NMR (CD₃OD) δ= 2.87-2.90 (m, 2H), 2.68 (bs, 2H), 4.81 (bs, 2H), 6.75-6.79 (m, 2H), 7.03-7.09 (m, 2H), 7.25-7.32 (m, 5H) |
| | | MS(ESI) m/z: 287.1(M+1) |
| | | yield: 8% |
| 63 | 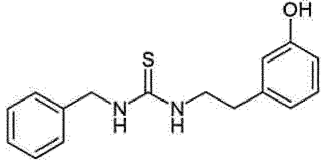 | ¹H NMR (CD₃OD) δ= 2.81 (t, *J*= 7.3 Hz, 2H), 3.72 (bs, 2H), 4.69 (bs, 2H), 6.64-6.71 (m, 3H), 7.08-7.12 (m, 1H), 7.24-7.32 (m, 5H) |
| | | MS(ESI) m/z: 286.9(M+1) |
| | | yield: 9% |

**[Table 1-12]**

| Ex. No. | structural formula | property values |
|---|---|---|
| 64 | 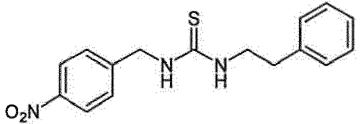 | ¹H NMR (CD₃OD) δ= 2.88-2.92 (m, 2H), 3.74 (bs, 2H), 4.85 (bs, 2H), 7.19-7.29 (m, 5H), 7.46-7.48 (m, 2H), 8.13-8.15 (m, 2H) |
| | | MS(ESI) m/z: 316.1(M+1) |
| | | yield: 93% |
| 65 | 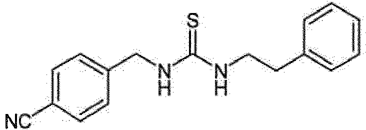 | ¹H NMR (CD₃OD) δ= 2.90 (t, *J*= 7.3 Hz, 2H), 3.78 (bs, 2H), 4.81 (bs, 2H), 7.21-7.29 (m, 5H), 7.43-7.46 (m, 2H), 7.65-7.67 (m, 2H) |
| | | MS (ESI) m/z: 295.8(M+1) |
| | | yield: 94% |
| 66 | 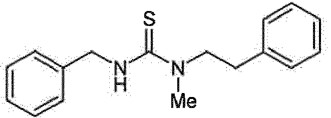 | ¹H NMR (CD₃OD) 5= 2.97 (t, *J*= 7.5 Hz, 2H), 3.05 (s, 3H), 4.05 (t, *J*= 7.5 Hz, 2H), 4.87 (s, 2H), 7.22-7.32 (m, 10H) |
| | | MS(ESI) m/z: 285.1(M+1) |
| | | yield: 75% |
| 67 | 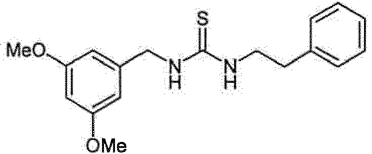 | ¹H NMR (CD₃OD) δ= 2.88 (t, *J*= 7.2 Hz, 2H), 3.76 (s, 8H), 4.62 (bs, 2H), 6.37-6.38 (m, 1H), 6.48-6.49 (m, 2H), 7.19-7.27 (m, 5H) |
| | | MS (ESI) m/z: 331.2(M+1) |
| | | yield: 92% |
| 68 | 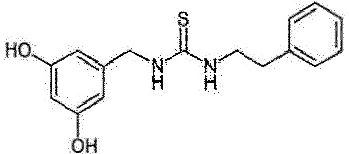 | ¹H NMR (CD₃OD) δ= 2.87 (t, *J*= 7.3 Hz, 2H), 3.73 (bs, 2H), 4.54 (bs, 2H), 6.21-6.22 (m, 1H), 6.28-6.29 (m, 2H), 7.17-7.30 (m, 5H) |
| | | MS (ESI) m/z: 301.1(M-1) |
| | | yield: 43% |
| 69 | 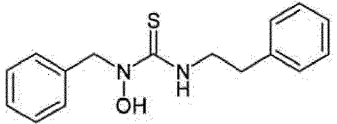 | ¹H NMR (CD₃OD) δ= 2.87-2.91 (m, 2H), 3.74 (bs, 2H), 4.69 (bs, 2H), 7.20-7.34 (m, 10H) |
| | | MS(ESI) m/z: 284.8(M-1) |
| | | yield: 36% |

**[Table 1-13]**

| Ex. No. | structural formula | property values |
|---|---|---|
| 70 | 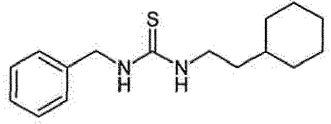 | ¹H NMR (CD₃OD) δ= 0.93-0.98 (m, 2H), 1.21-1.32 (m, 4H), 1.43-1.49 (m, 2H), 1.66-1.77 (m, 5H), 3.51 (bs, 2H), 4.71 (bs, 2H), 7.24-7.33 (m, 5H) |
| | | MS(ESI) m/z: 277.3(M+1) |
| | | yield: 77% |
| 71 | 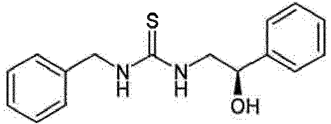 | ¹H NMR (CD₃OD) δ= 3.32-3.34 (m, 1H), 3.90 (bs, 1H), 4.73 (bs, 2H), 4.92-4.94 (m, 1H), 7.26-7.42 (m, 10H) |
| | | MS (ESI) m/z: 287.1(M+1) |
| | | yield: 61% |
| 72 | 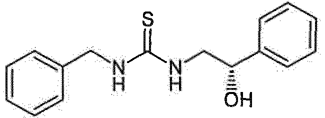 | ¹H NMR (CD₃OD) δ= 3.32-3.34 (m, 1H), 3.90 (bs, 1H), 4.73 (bs, 2H), 4.89-4.98 (m, 1H), 7.26-7.42 (m, 10H) |
| | | MS(ESI) m/z: 287.3(M+1) |
| | | yield: 54% |
| 73 | 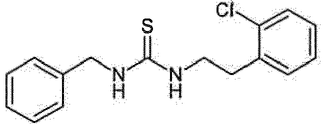 | ¹H NMR (CD₃OD) δ= 3.06 (t, *J*= 7.2 Hz, 2H), 3.78 (bs, 2H), 4.70 (bs, 2H), 7.10-7.14 (m, 1H), 7.25-7.35 (m, 7H), 7.54-7.56 (m, 1H) |
| | | MS (ESI) m/z: 305.3(M+1) |
| | | yield: quant. |
| 74 | 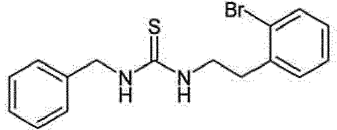 | ¹H NMR (CD₃OD) δ= 3.06 (t, *J*= 7.2 Hz, 2H), 3.78 (bs, 2H), 4.70 (bs, 2H), 7.20-7.38 (m, 9H) |
| | | MS(ESI) m/z: 348.9(M+1) |
| | | yield: quant. |
| 75 | 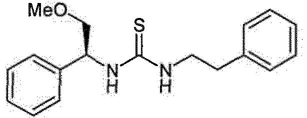 | ¹H NMR (CD₃OD) δ= 2.86-2.88 (m, 2H), 3.35 (s, 3H), 3.64-3.75 (m, 4H), 5.52 (bs, 1H), 7.20-7.33 (m, 10H) |
| | | MS(ESI) m/z: 315.0(M+1) |
| | | yield: 74% |

**[Table 1-14]**

| Ex. No. | structural formula | property values |
|---|---|---|
| 76 | 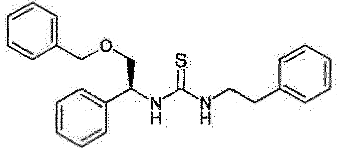 | ¹H NMR (CD₃OD) δ= 2.83-2.86 (m, 2H), 3.68-3.82 (m, 4H), 4.47-4.57 (m, 2H), 5.60 (bs, 1H), 7.18-7.33 (m, 15H) |
| | | MS(ESI) m/z: 391.1(M+1) |
| | | yield: 96% |
| 77 | 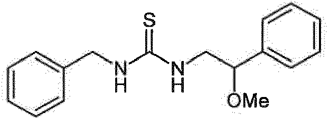 | ¹H NMR ((CD₃)₂SO) δ= 3.16 (s, 3H), 3.43-3.48 (m, 1H), 3.88 (bs, 1H), 4.41-4.44 (m, 2H), 4.68 (bs, 2H), 7.24-7.42 (m, 10H), 7.59-7.62 (m, 1H), 7.98 (bs, 1H) |
| | | MS(ESI) m/z: 298.9(M-1) |
| | | yield: 95% |
| 78 | 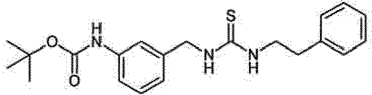 | ¹H NMR (CD₃OD) δ= 1.53 (s, 9H), 2.90 (t, *J*= 7.3 Hz, 2H), 3.76 (bs, 2H), 4.68 (bs, 2H), 6.93-6.99 (m, 1H), 7.19-7.37 (m, 8H) |
| | | MS (ESI) m/z: 386.1(M+1) |
| | | yield: 81% |
| 79 | 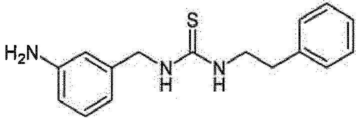 | ¹H NMR (CD₃OD) δ= 2.88 (t, *J*= 7.3 Hz, 2H), 3.74 (bs, 2H), 4.56 (bs, 2H), 6.62-6.68 (m, 3H), 7.04-7.31 (m, 6H) |
| | | MS(ESI) m/z: 286.4(M+1) yield: quant. |
| 80 | 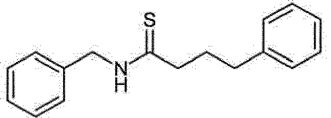 | ¹H NMR (CD₃OD) δ= 2.84-2.88 (m, 4H), 3.02-3.06 (m, 2H), 3.75-3.79 (m, 2H), 7.18-7.30 (m, 10H) |
| | | MS(ESI) m/z: 269.9(M+1) |
| | | yield: 66% |
| 81 | 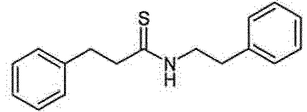 | ¹H NMR (CD₃OD) δ= 2.05-2.12 (m, 4H), 2.62-2.71 (m, 4H), 4.87 (s, 2H), 7.14-7.36 (m, 10H) |
| | | MS (ESI) m/z: 270.1(M+1) |
| | | yield: 48% |

**[Table 1-15]**

| Ex. No. | structural formula | property values |
|---|---|---|
| 82 | 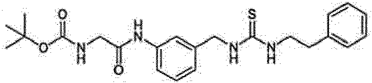 | ¹H NMR (CD₃OD) δ= 1.47-1.50 (m, 9H), 2.87-2.90 (m, 2H), 3.69-4.90 (m, 4H), 4.68 (bs, 2H), 7.03-7.59 (m, 9H) |
| | | MS(ESI) m/z: 443.0(M+1) |
| | | yield: quant. |
| 83 | 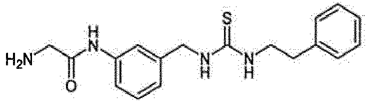 | ¹H NMR (CD₃OD) δ= 2.88 (t, J*=* 7.3 Hz, 2H), 3.42 (s, 2H), 3.73 (bs, 2H), 4.68 (bs, 2H), 7.04-7.06 (m, 1H), 7.17-7.29 (m, 6H), 7.49-7.53 (m, 2H) |
| | | MS (ESI) m/z: 340.9(M+1) |
| | | yield: 17% |
| 84 | 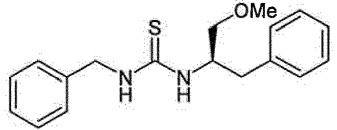 | ¹H NMR (CD₃OD) δ= 2.89-3.00 (m, 2H), 3.33 (s, 3H), 3.35-3.36 (m, 2H), 4.70 (bs, 2H), 7.21-7.33 (m, 9H) |
| | | MS(ESI) m/z: 315.2(M+1) |
| | | yield: 52% |
| 85 | 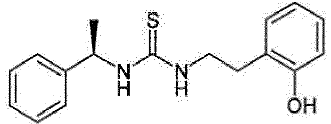 | ¹H NMR (CD₃OD) δ= 1.48 (d, *J*= 6.4 Hz, 3H), 2.85 (t, *J*= 7.1 Hz, 2H), 3.68 (bs, 2H), 5.40 (bs, 1H), 6.74-6.82 (m, 2H), 7.03-7.07 (m, 2H), 7.20-7.34 (m, 5H) |
| | | MS(ESI) m/z: 301.1(M+1) |
| | | yield: quant. |
| 86 | 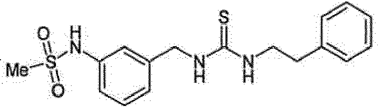 | ¹H NMR (CD₃OD) δ= 2.90 (t, *J*= 7.3 Hz, 2H), 2.96 (s, 3H), 3.75 (bs, 2H), 4.80 (bs, 2H), 7.08-7.32 (m, 9H) |
| | | MS(ESI) m/z: 364.2(M+1) |
| | | yield: 78% |
| 87 | 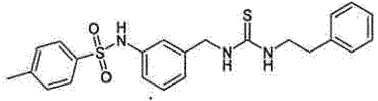 | ¹H NMR (CD₃OD) δ= 2.35 (s, 3H), 2.88 (t, *J*= 7.3 Hz, 2H), 3.74 (bs, 2H), 4.60 (bs, 2H), 6.96-6.98 (m, 2H), 7.11-7.30 (m, 9H), 7.66-7.68 (m, 2H) |
| | | MS(ESI) m/z: 440.0(M+1) |
| | | yield: 88% |

**[Table 1-16]**

| Ex. No. | structural formula | property values |
|---|---|---|
| 88 | 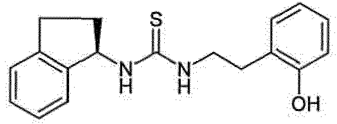 | ¹H NMR (CD₃OD) δ= 1.84 (bs, 1H), 2.55-2.59 (m, 1H), 2.79-3.00 (m, 4H), 3.74 (bs, 2H), 5.90 (bs, 1H), 6.74-6.77 (m, 2H), 7.01-7.29 (m, 6H) |
| | | MS(ESI) m/z: 313.2(M+1) |
| | | yield: 98% |
| 89 | 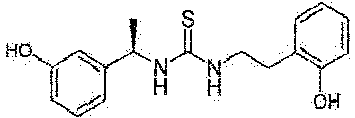 | ¹H NMR (CD₃OD) 5= 1.47 (d, *J*= 6.8 Hz, 3H), 2.85 (t, *J*= 7.1 Hz, 2H), 3.65 (bs, 2H), 5.32 (bs, 1H), 6.67-6.82 (m, 5H), 7.03-7.17 (m, 3H) |
| | | MS(ESI) m/z: 317.1(M+1) |
| | | yield: 40% |
| 90 | 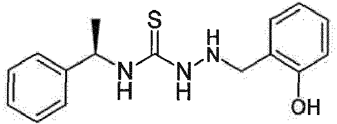 | ¹H NMR (CD₃OD) δ= 1.43 (d, *J*= 7.0 Hz, 3H), 3.87-3.95 (m, 2H), 5.46-5.50 (m, 1H), 6.77-6.85 (m, 2H), 7.13-7.31 (m, 7H) |
| | | MS(ESI) m/z: 302.0(M+1) |
| | | yield: 46% |
| 91 | 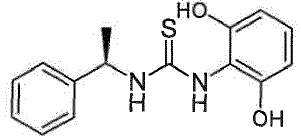 | ¹H NMR (CD₃OD) δ= 1.49 (d, *J*= 7.0 Hz, 3H), 5.59 (bs, 1H), 6.42 (d, *J*= 8.2 Hz, 2H), 6.94-6.98 (m, 1H), 7.21-7.34 (m, 5H) |
| | | MS(ESI) m/z: 289.0(M+1) |
| | | yield: 31% |
| 92 | 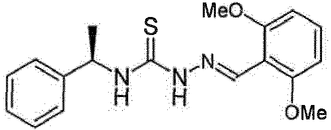 | ¹H NMR ((CD₃)₂SO) δ= 1.54 (d, *J*= 6.9 Hz, 3H), 3.77 (s, 6H), 5.49-5.57 (m, 1H), 6.71 (d, *J*= 8.5 Hz, 2H), 7.31-7.39 (m, 6H), 8.03-8.06 (m, 1H), 8.35 (s, 1H), 11.6 (s, 1H) |
| | | MS(ESI) m/z: 344.2(M+1) |
| | | yield: 85% |

**[Table 1-17]**

| Ex. No. | structural formula | property values |
|---|---|---|
| 93 | 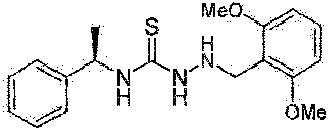 | ¹H NMR ((CD₃)₂SO) δ= 1.38 (d, *J*= 6.9 Hz, 3H), 3.71 (s, 6H), 3.84-3.90 (m, 2H), 4.85 (bs, 1H), 5.39-5.50 (m, 1H), 6.65 (d, *J*= 8.4 Hz, 2H), 7.23-7.35 (m, 6H), 7.90-7.92 (m, 1H), 8.88 (s, 1H) |
| | | MS(ESI) m/z: 346.2(M+1) |
| | | yield: 62% |
| 94 | 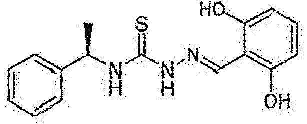 | ¹H NMR ((CD₃)₂SO) δ= 1. 52 (d, *J*= 7.0 Hz, 3H), 5.60-5.72 (m, 1H), 6.36 (d, *J*= 8.2 Hz, 2H), 7.06-7.10 (m, 1H), 7.23-7.26 (m, 1H), 7.32-7.43 (m, 5H), 8.61 (s, 1H), 8.67-8.69 (m, 1H), 10.1 (bs, 1H), 11.4 (s, 1H) |
| | | MS (ESI) m/z: 316.2(M+1) |
| | | yield: 94% |
| 95 | 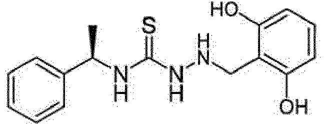 | ¹H NMR ((CD₃)₂SO) δ= 1.42 (d, *J*= 7.0 Hz, 3H), 3.83 (d, *J*= 6.7 Hz, 2H), 4.71 (bs, 1H), 5.46-5.50 (m, 1H), 6.34 (d, *J*= 8.1 Hz, 2H), 6.87-6.91 (m, 1H), 7.21-7.23 (m, 1H), 7.31-7.32 (m, 4H), 8.36-8.38 (m, 1H), 8.86 (s, 1H), 9.46 (s, 2H) |
| | | MS(ESI) m/z: 318.0(M+1) |
| | | yield: 71% |
| 96 | 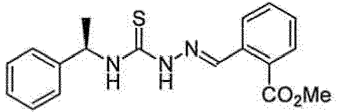 | ¹H NMR ((CD₃)₂SO) δ= 1. 60 (d, *J*= 7.0 Hz, 3H), 3.88 (s, 3H), 5.68-5.80 (m, 1H), 7.21-8.75 (m, 10H), 10.8 (s, 1H) |
| | | MS(ESI) m/z: 339.9(M-1) |
| | | yield: 90% |
| 97 | 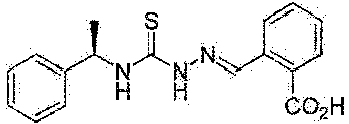 | ¹H NMR ((CD₃)₂SO) 5= 1.60 (d, *J*= 7.0 Hz, 3H), 3.88 (s, 3H), 5.68-5.80 (m, 1H), 7.21-8.75 (m, 10H), 10.8 (s, 1H) |
| | | MS(ESI) m/z: 339.9(M-1) |
| | | yield: 71% |

**[Table 1-18]**

| Ex. No. | structural formula | property values |
|---|---|---|
| 98 | 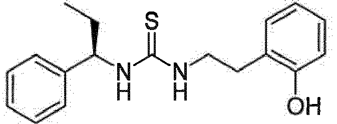 | ¹H NMR (CD₃OD) δ= 0.90-0.95 (m, 3H), 2.11 (bs, 2H), 2.82-2.86 (m, 2H), 3.65 (bs, 2H), 5.27 (bs, 1H), 6.74-6.81 (m, 2H), 7.03-7.07 (m, 2H), 7.22-7.32 (m, 5H) |
| | | MS(ESI) m/z: 312.9(M-1) |
| | | yield: 99% |
| 99 | 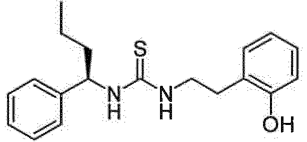 | ¹H NMR (CD₃OD) δ= 0.89-0.96 (m, 3H), 1.29-1.41 (m, 2H), 1.68-1.92 (m, 2H), 2.82-2.86 (m, 2H), 3.66 (bs, 2H), 5.60 (bs, 1H), 6.74-6.84 (m, 2H), 7.02-7.07 (m, 2H), 7.19-7.31 (m, 5H) |
| | | MS(ESI) m/z: 329.3(M+1) |
| | | yield: quant. |
| 100 | 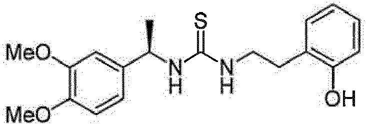 | ¹H NMR (CD₃OD) δ= 1.48 (d, *J*= 6.6 Hz, 3H), 2.81-2.85 (m, 2H), 3.69 (bs, 2H), 3.81 (s, 6H), 5.40 (bs, 1H), 6.72-7.06 (m, 7H) |
| | | MS(ESI) m/z: 359.1(M-1) |
| | | yield: 92% |
| 101 | 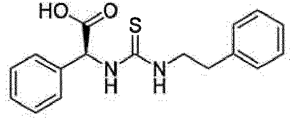 | ¹H NMR ((CD₃)₂SO) δ= 2.79-2.83 (m, 2H), 3.62-3.67 (m, 2H), 5.92-5.94 (m, 1H), 7.20-7.41 (m, 10H), 7.72 (bs, 1H), 8.16 (bs, 1H) |
| | | MS(ESI) m/z: 315.2(M+1) |
| | | yield: 26% |
| 102 | 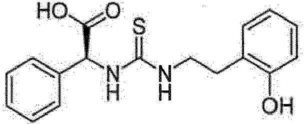 | ¹H NMR ((CD₃)₂SO) δ= 2.72 (bs, 2H), 3.35-3.63 (m, 3H), 5.48 (bs, 1H), 6.69-7.35 (m, 11H), 8.16 (bs, 2H), 9.92 (bs, 1H) |
| | | MS(ESI) m/z: 328.8(M-1) |
| | | yield: 37% |

**[Table 1-19]**

| Ex. No. | structural formula | property values |
|---|---|---|
| 103 | 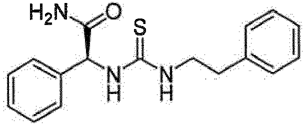 | ¹H NMR ((CD₃)₂SO) δ= 2.88-2.98 (m, 2H), 3.06 (s, 2H), 3.80-3.99 (m, 2H), 7.16-7.24 (m, 5H), 7.37 (s, 5H), 10.8 (bs, 1H) |
| | | MS(ESI) m/z: 311.9(M-1) |
| | | yield: 16% |
| 104 | 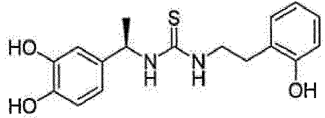 | ¹H NMR ((CD₃)₂SO) δ= 1. 33 (d, *J*= 6.82 Hz, 3H), 2.73 (t, *J*= 7.4 Hz, 2H), 3.54 (bs, 2H), 5.29 (bs, 1H), 6.56-6.81 (m, 5H), 7.01-7.06 (m, 1H), 7.20 (bs, 1H), 7.61 (bs, 1H), 8.77-8.82 (m, 2H), 9.37 (bs, 1H) |
| | | MS (ESI) m/z: 333.2(M+1) |
| | | yield: 61% |
| 105 | 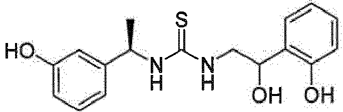 | ¹H NMR ((CD₃)₂SO) δ= 1.16-1.38 (m, 3H), 3.18 (bs, 1H), 3.82 (bs, 1H), 4.95-4.97 (m, 1H), 5.38-5.48 (m, 2H), 6.61-6.83 (m, 5H), 7.05-7.13 (m, 2H), 7.34-7.36 (m, 2H), 8.01 (bs, 1H), 9.35-9.60 (m, 2H) |
| | | MS(ESI) m/z: 333.1(M+1) |
| | | yield: 76% |
| 106 | 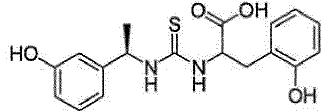 | ¹H NMR ((CD₃)₂SO) δ= 1.34 (bs, 3H), 2.97-3.13 (m, 2H), 4.58 (bs, 1H), 5.37 (bs, 1H), 5.75 (bs, 1H), 6.64-6.72 (m, 5H), 6.98-7.11 (m, 3H), 7.39 (bs, 1H), 8.21 (bs, 1H), 9.39 (bs, 1H) |
| | | MS(ESI) m/z: 358.9 (M-1) |
| | | yield: 74% |

**[Table 1-20]**

| Ex. No. | structural formula | property values |
|---|---|---|
| 107 | 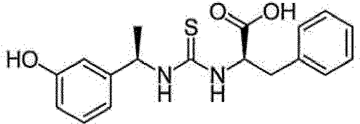 | ¹H NMR ((CD₃)₂SO) δ= 1.35 (d, *J*= 6.9 Hz, 3H), 2.98-3.03 (m, 1H), 3.15-3.17 (m, 1H), 5.07-5.08 (m, 1H), 5.34 (bs, 1H), 6.62-6.72 (m, 3H), 7.09-7.40 (m, 7H), 8.14 (bs, 1H), 9.37 (bs, 1H) |
| | | MS(ESI) m/z: 345.1(M+1) |
| | | yield: 31% |
| 108 | 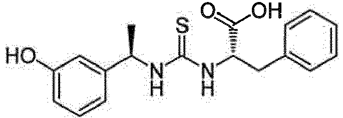 | ¹H NMR ((CD₃)₂SO) δ= 1.34 (d, *J*= 6.9 Hz, 3H), 2.97-3.02 (m, 1H), 3.16-3.21 (m, 1H), 5.06-5.08 (m, 1H), 5.33 (bs, 1H), 6.63-6.70 (m, 3H), 7.10-7.38 (m, 7H), 8.13-8.15 (m, 1H), 9.36 (s, 1H) |
| | | MS(ESI) m/z: 344.9(M+1) |
| | | yield: 51% |
| 109 | 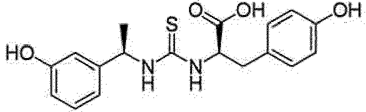 | ¹H NMR ((CD₃)₂SO) δ= 1.35 (d, *J*= 6.9 Hz, 3H), 2.89-2.91 (m, 1H), 2.99-3.08 (m, 1H), 4.98-4.99 (m, 1H), 5.35 (bs, 1H), 6.62-6.72 (m, 5H), 6.94 (bs, 2H), 7.09-7.13 (m, 1H), 7.35 (bs, 1H), 8.13 (bs, 1H), 9.24 (bs, 1H), 9.36 (bs, 1H) |
| | | MS(ESI) m/z: 361.2(M+1) |
| | | yield: 74% |
| 110 | 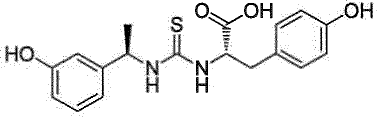 | ¹H NMR ((CD₃)₂SO) δ= 1.34 (d, *J*= 6.9 Hz, 3H), 2.85-2.90 (m, 1H), 3.03-3.08 (m, 1H), 4.98-5.00 (m, 1H), 5.34 (bs, 1H), 6.63-6.70 (m, 5H), 6.90-6.92 (m, 2H), 7.11-7.15 (m, 1H), 7.30 (bs, 1H), 8.13-8.16 (m, 1H), 9.23 (bs, 1H), 9.36 (bs, 1H) |
| | | MS(ESI) m/z: 361.0(M+1) |
| | | yield: 79% |

**[Table 1-21]**

| Ex. No. | structural formula | property values |
|---|---|---|
| 111 | 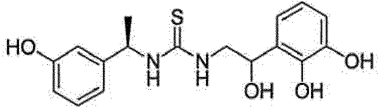 | ¹H NMR ((CD₃)₂SO) 5= 1.35-1.37 (m, 3H), 3.18 (bs, 1H), 3.79 (bs, 1H), 4.96-4.98 (m, 1H), 5.37-5.47 (m, 2H), 6.61-6.83 (m, 6H), 7.09-7.14 (m, 1H), 7.35 (bs, 1H), 8.00 (bs, 1H), 8.38 (bs, 1H), 9.23 (bs, 1H), 9.33 (bs, 1H) |
| | | MS(ESI) m/z: 346.8(M-1) |
| | | yield: quant. |
| 112 | 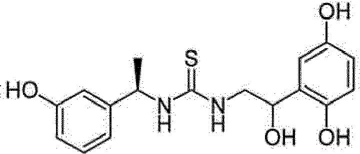 | ¹H NMR ((CD₃)₂SO) δ= 1.36 (bs, 3H), 3.11 (bs, 1H), 3.80 (bs, 1H), 4.88 (bs, 1H), 5.28-5.50 (m, 2H), 6.46-6.84 (m, 7H), 7.38 (bs, 1H), 8.01 (bs, 1H), 8.38 (bs, 1H), 8.66-8.84 (m, 2H), 9.33-9.35 (m, 1H) |
| | | MS(ESI) m/z: 346.9 (M-1) |
| | | yield: 8% |
| 113 | 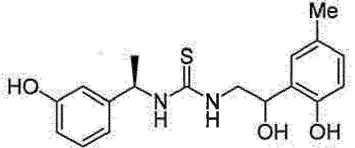 | ¹H NMR ((CD₃)₂SO) δ= 1.34-1.46 (m, 3H), 2.20 (bs, 3H), 3.19-3.52 (m, 4H), 6.61-6.88 (m, 5H), 7.09-7.16 (m, 2H), 7.92-8.14 (m, 1H), 9.02-9.41 (m, 2H) |
| | | MS(ESI) m/z: 345.0 (M-1) |
| | | yield: 29% |
| 114 | 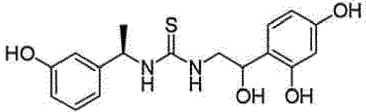 | ¹H NMR ((CD₃)₂SO) δ= 1.42-1.43 (m, 3H), 2.79-3.95 (m, 3H), 5.39-5.51 (m, 1H), 6.18-9.41 (m, 9H) |
| | | MS(ESI) m/z: 345.0 (M-1) |
| | | yield: 10% |

**[Table 1-22]**

| Ex. No. | structural formula | property values |
|---|---|---|
| 115 | 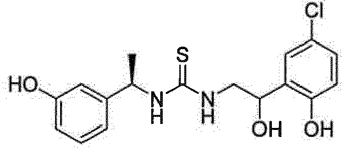 | ¹H NMR ((CD₃)₂SO) δ= 1.43-1.46 (m, 3H), 3.74-3.83 (m, 1H), 4.45-4.53 (m, 1H), 5.07 (bs, 1H), 5.27-5.30 (m, 1H), 5.48-5.52 (m, 1H), 6.62-6.84 (m, 5H), 7.05-7.13 (m, 2H), 7.34-7.37 (m, 1H), 8.12-8.15 (m, 1H), 9.32-9.90 (m, 3H) |
| | | yield: 7% |
| 116 | 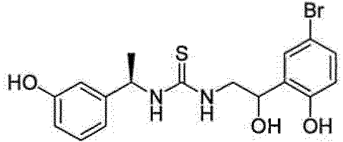 | ¹H NMR ((CD₃)₂SO) δ= 1.42-1.46 (m, 3H), 3.77-3.83 (m, 1H), 4.43-4.51 (m, 1H), 5.25-5.29 (m, 1H), 5.47-5.51 (m, 1H), 6.62-6.83 (m, 6H), 7.07-7.13 (m, 2H), 7.33-7.36 (m, 1H), 8.10-8.13 (m, 1H), 9.39 (bs, 2H) |
| | | yield: 7% |
| 117 | 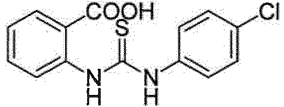 | ¹H NMR ((CD₃)₂SO) δ= 7.15-7.19 (m, 1H), 7.41-7.44 (m, 2H), 7.51-7.55 (m, 3H), 7.90-7.93 (m, 1H), 8.41-8.43 (m, 1H), 10.56 (s, 1H), 11.19 (bs, 2H) |
| | | MS(ESI) m/z: 304.8 (M-1) |
| | | yield: 5% |
| 118 | 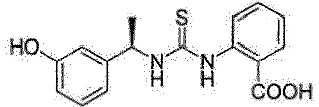 | ¹H NMR (CD₃OD) δ= 1.54 (d, *J*= 6.9 Hz, 3H), 5.60 (bs, 1H), 6.68-6.70 (m, 1H), 6.85-6.89 (m, 2H), 7.11-7.18 (m, 2H), 7.46-7.51 (m, 1H), 8.00-8.03 (m, 1H), 8.39 (bs, 1H) |
| | | MS(ESI) m/z: 317.2 (M+1) |
| | | yield: 7% |
| 119 | 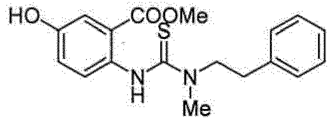 | ¹H NMR (CDCl₃) δ= 3.08-3.12 (m, 2H), 3.30 (s, 3H), 3.92 (s, 2H), 4.12 (bs, 2H), 6.25 (bs, 1H), 6.95-6.98 (m, 1H), 7.25-7.37 (m, 6H), 8.31 (d, *J*= 9.0 Hz, 1H), 10.2 (bs, 1H) |
| | | MS(ESI) m/z: 345.1 (M+1) |
| | | yield: 93% |

**[Table 1-23]**

| Ex. No. | structural formula | property values |
|---|---|---|
| 120 | 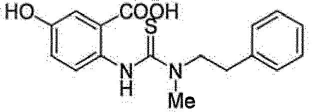 | ¹H NMR ((CD₃)₂SO) δ= 2.93-2.97 (m, 2H), 3.21 (s, 1H), 3.97-4.01 (m, 2H), 6.94-6.97 (m, 1H), 7.21-7.24 (m, 1H), 7.29-7.32 (m, 5H), 7.94 (d, *J*= 8.9 Hz, 1H), 9.60 (s, 1H), 9.96 (s, 1H) |
| | | MS(ESI) m/z: 331.1 (M+1) |
| | | yield: 64% |
| 121 | 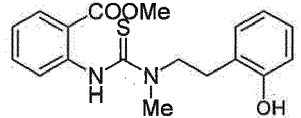 | ¹H NMR (CD₃OD) δ= 3.04-3.08 (m, 2H), 3.30 (s, 3H), 3.92 (s, 3H), 4.04 (bs, 2H), 6.75-6.79 (m, 2H), 7.04-7.06 (m, 1H), 7.14-7.18 (m, 2H), 7.49-7.51 (m, 1H), 7.96-7.99 (m, 1H), 8.36-8.38 (m, 1H) |
| | | MS(ESI) m/z: 345.0 (M+1) |
| | | yield: 84% |
| 122 | 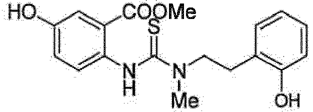 | ¹H NMR (CD₃OD) δ= 3.03-3.07 (m, 2H), 3.30 (s, 3H), 3.88 (s, 3H), 3.98-4.07 (m, 2H), 6.76-6.80 (m, 2H), 6.97-7.00 (m, 1H), 7.04-7.09 (m, 1H), 7.15-7.17 (m, 1H), 7.36-7.37 (m, 1H), 7.79-7.82 (m, 1H) |
| | | MS(ESI) m/z: 361.1 (M+1) |
| | | yield: 52% |
| 123 | 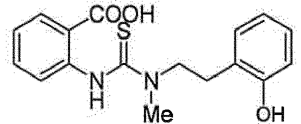 | ¹H NMR (CD₃OD) δ= 3.03-3.07 (m, 2H), 3.30 (s, 3H), 3.88 (s, 3H), 3.98-4.07 (m, 2H), 6.76-6.80 (m, 2H), 6.97-7.00 (m, 1H), 7.04-7.09 (m, 1H), 7.15-7.17 (m, 1H), 7.36-7.37 (m, 1H), 7.79-7.82 (m, 1H) |
| | | MS(ESI) m/z: 361.1 (M+1) |
| | | yield: 38% |

**[Table 1-24]**

| Ex. No. | structural formula | property values |
|---|---|---|
| 124 | 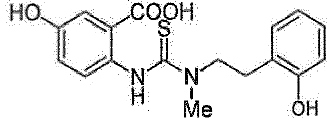 | ¹H NMR (CD₃OD) δ= 2.99-3.03 (m, 2H), 3.24 (s, 3H), 3.99 (bs, 2H), 6.71-6.75 (m, 2H), 6.93-6.96 (m, 1H), 7.00-7.04 (m, 1H), 7.12-7.14 (m, 1H), 7.39-7.40 (m, 1H), 8.06-8.08 (m, 1H) |
| | | MS(ESI) m/z: 347.1 (M+1) |
| | | yield: 66% |
| 125 | 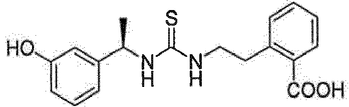 | ¹H NMR (CD₃OD) δ= 1.47 (bs, 3H), 3.19-3.23 (m, 2H), 3.70 (bs, 2H), 5.40 (bs, 1H), 6.64-6.67 (m, 1H), 6.78 (bs, 2H), 7.10-7.14 (m, 1H), 7.28-7.32 (m, 2H), 7.45 (bs, 1H), 7.93 (bs, 1H) |
| | | MS(ESI) m/z: 342.9 (M-1) |
| | | yield: 62% |
| 126 | 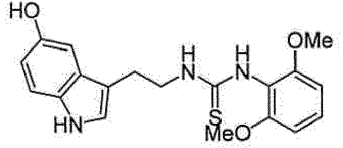 | ¹H NMR ((CD₃)₂SO) δ= 2.80 (t, *J*= 7.4 Hz, 2H), 3.59-3.70 (m, 2H), 3.72 (s, 6H), 6.58-6.61 (m, 1H), 6.68 (d *J*= 8.4 Hz, 2H), 6.92-6.98 (m, 3H), 7.12 (d *J*= 8.6 Hz, 1H), 7.22-7.26 (m, 1H), 8.38 (s, 1H), 8.57 (s, 1H), 10.47 (s, 1H) |
| | | MS(ESI) m/z: 369.8 (M-1) |
| | | yield: 99% |
| 127 | 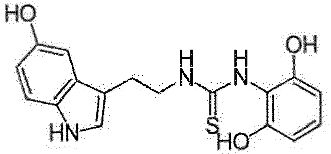 | ¹H NMR ((CD₃)₂SO) δ= 2.78-2.89 (m, 2H), 3.67 (bs, 2H), 6.38 (d *J*= 8.2 Hz, 2H), 6.58-6.61 (m, 1H), 6.88-6.92 (m, 2H), 7.01 (s, 1H), 7.12 (d *J*= 8.6 Hz, 1H), 7.33 (bs, 1H), 8.39 (bs, 1H), 8.58 (bs, 1H), 9.29 (bs, 2H), 10.48 (s, 1H) |
| | | MS(ESI) m/z: 342.0 (M-1) |
| | | yield: 24% |

The representative production methods of the compounds described in the above-mentioned Tables 1-1 to 1-24 are described in the following. The compound of the present invention can be synthesized according to any of the following methods.

### Example 40

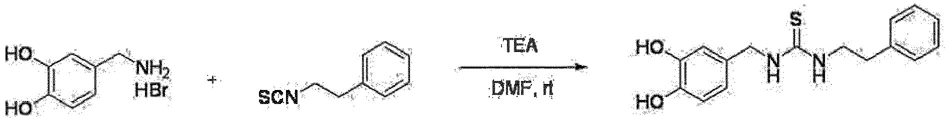

3,4-Dihydroxybenzylamine hydrobromide (220.1 mg, 1.0 mmol) was dissolved in N,N-dimethylformamide (3 mL), triethylamine (416 µL, 3.0 mmol) was added, and the mixture was stirred at room temperature for 15 min. Thereafter, phenethyl isothiocyanate (195 µL, 1.2 mmol) was added, and the mixture was stirred at room temperature overnight. After completion of the reaction, the mixture was diluted with ethyl acetate, and washed with water and saturated brine. The organic layer was dried over magnesium sulfate and filtered, and the solvent was evaporated. The obtained crude product was purified by silica gel column chromatography to give 1-(3,4-dihydroxybenzyl)-3-(2-phenylethyl)thiourea (245.7 mg, 0.8 mmol).

### Example 41

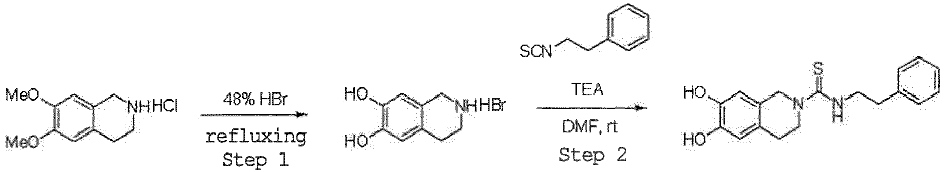

### step 1

To 6,7-dimethoxy-1,2,3,4-tetrahydroisoquinoline hydrochloride (2.297 g, 10.0 mmol) was added 48% aqueous hydrobromic acid solution (6.8 mL, 60.0 mmol), and the mixture was refluxed. After completion of the reaction, the mixture was concentrated, and the residue was washed with ethyl acetate and filtered. The obtained solid was dried to give 6,7-dihydroxy-1,2,3,4-tetrahydroisoquinolinehydrobromide (2.326 g, 9.5 mmol).

### step 2

Under conditions similar to those in Example 40, 6,7-dihydroxy-1,2,3,4-tetrahydroisoquinoline hydrobromide was reacted with phenethyl isothiocyanate to give N-(2-phenylethyl)-3,4-dihydro-6,7-dihydroxyisoquinoline-2(1H)-carbothioamide.

### Example 47

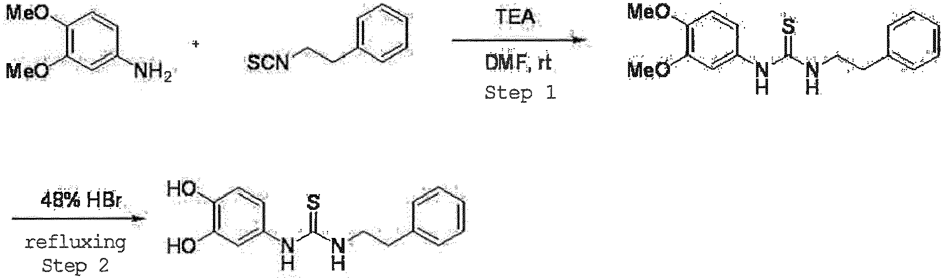

### step 1

Under conditions similar to those in Example 40, 3,4-dimethoxyaniline was reacted with phenethyl isothiocyanate to give 1-(3,4-dimethoxyphenyl)-3-(2-phenylethyl)thiourea.

### step 2

Under conditions similar to those in Example 41, step 1, 1-(3,4-dimethoxyphenyl)-3-(2-phenylethyl)thiourea was reacted in 48% aqueous hydrobromic acid solution to give 1-(3,4-dihydroxyphenyl)-3-(2-phenylethyl)thiourea.

### Example 42

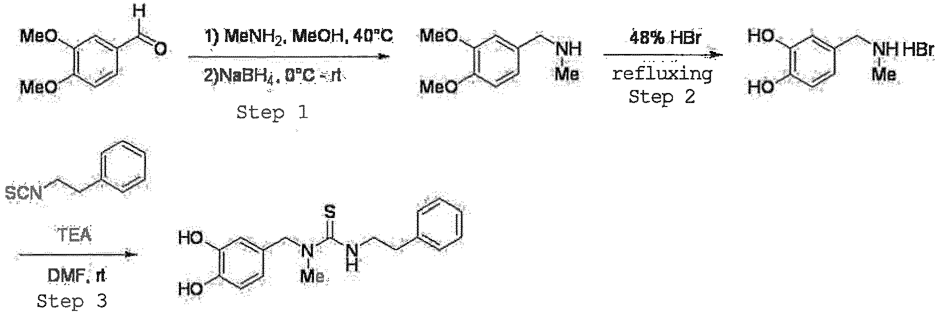

### step 1

To 3,4-dimethoxybenzaldehyde (3.323 g, 20.0 mmol) was added 9.8M methylamine-methanol solution (20.5 mL, 200.0 mmol), and the mixture was stirred at 40°C for 5 hr. Thereafter, sodium borohydride (3.783 g, 100.0 mmol) was added in an ice bath, and the mixture was reacted at room temperature. After completion of the reaction, the mixture was adjusted to pH about 2 by adding 2M hydrochloric acid, concentrated, and adjusted to pH about 11 by adding 2M aqueous sodium hydroxide solution. After extraction with dichloromethane, the organic layer was dried over magnesium sulfate and filtered, and the solvent was evaporated. The obtained crude product was purified by silica gel column chromatography to give 3,4-dimethoxy-N-methylbenzylamine (3.806 g, 21.0 mmol).

### step 2

Under conditions similar to those in Example 41, step 1, 3,4-dimethoxy-N-methyl-benzylamine was reacted in 48% aqueous hydrobromic acid solution to give 3,4-dihydroxy-N-methylbenzylamine hydrobromide.

### step 3

Under conditions similar to those in Example 40, 3,4-dihydroxy-N-methylbenzylamine hydrobromide was reacted with phenethyl isothiocyanate to give 1-(3,4-dihydroxybenzyl)-1-methyl-3-(2-phenylethyl)thiourea.

### Example 78

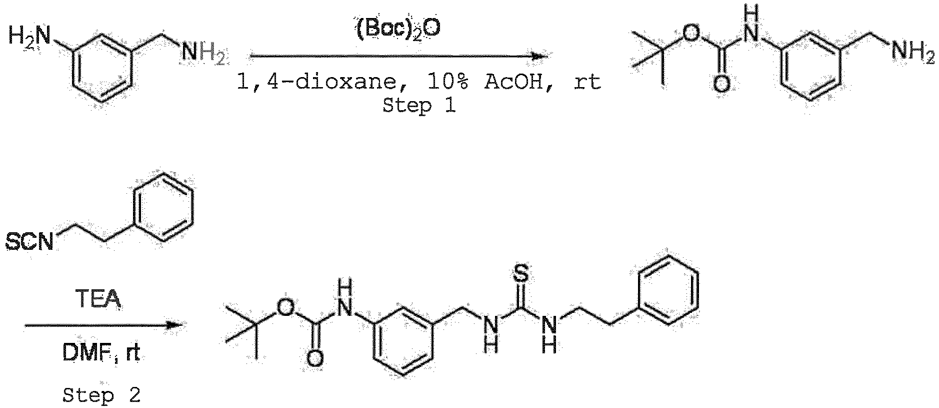

### step 1

3-Aminobenzylamine (1.405 g, 11.5 mmol) was dissolved in 10% aqueous acetic acid solution (100 mL), thereto was added a solution of di-tert-butyl dicarbonate (2.619 g, 12.0 mmol) in 1,4-dioxane (100 mL), and the mixture was stirred at room temperature. After completion of the reaction, water was added, the mixture was extracted three times with diethylether, and the aqueous layer was adjusted to pH about 14 by adding 2M aqueous sodium hydroxide solution. Thereafter, the aqueous layer was extracted three times with diethylether, and washed twice with water. The organic layer was dried over magnesium sulfate and filtered, and the solvent was evaporated. The obtained crude product was crystallized from ethyl acetate-hexane mixed solution to give tert-butyl N-(3-(aminomethyl)phenyl)carbamate (0.969 g, 4.36 mmol).

### step 2

Under conditions similar to those in Example 40, tert-butyl N-(3-(aminomethyl)phenyl)carbamate was reacted with phenethyl isothiocyanate to give tert-butyl 3-{[3-(2-phenylethyl)thioureido]methyl}phenylcarbamate.

### Example 79

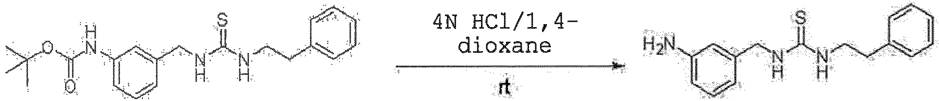

To tert-butyl 3-{[3-(2-phenylethyl)thioureido]methyl}phenylcarbamate (0.386 g, 1.0 mmol) obtained in Example 78 was added 4N hydrogen chloride/1,4-dioxane solution, and the mixture was stirred at room temperature. After completion of the reaction, the mixture was adjusted to pH about 14 by adding 1M sodium hydroxide, and extracted with ethyl acetate. The organic layer was dried over magnesium sulfate and filtered, and the solvent was evaporated. The obtained crude product was purified by silica gel column chromatography to quantitatively give 1-(3-aminobenzyl)-3-(2-phenylethyl)thiourea.

### Example 80

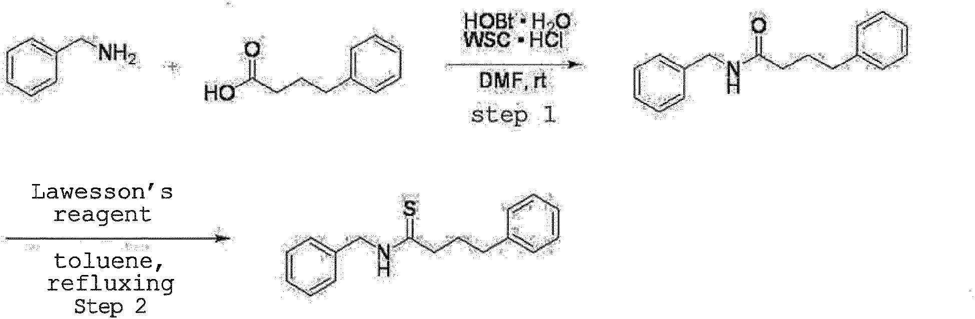

### step 1

4-Phenylbutanoic acid (1.642 g, 10.0 mmol) was dissolved in N,N-dimethylformamide (30 mL), and 1-hydroxybenzotriazole monohydrate (1.838 g, 12.0 mmol) and 1-ethyl-3-(3-dimethylaminopropyl)carbodiimide hydrochloride (2.300 g, 12.0 mmol) were added. Thereafter, benzylamine (1.3 mL, 12.0 mmol) was added, and the mixture was stirred at room temperature overnight. After completion of the reaction, the solvent was evaporated, and the residue was diluted with ethyl acetate. The mixture was washed successively with 5% aqueous citric acid solution (twice), saturated brine (once), 10% saturated aqueous sodium hydrogen carbonate (twice) and saturated brine (once). The organic layer was dried over magnesium sulfate and filtered, and the solvent was evaporated. The obtained crude product was purified by silica gel column chromatography to give N-benzyl-4-phenylbutanamide (2.390 g, 9.43 mmol).

### step 2

N-Benzyl-4-phenylbutanamide (0.760 g, 3.0 mmol) was dissolved in toluene (20 mL), a Lawesson's reagent (2.427 g, 6.0 mmol) was added, and the mixture was refluxed. After completion of the reaction, the solvent was evaporated, and the residue was diluted with ethyl acetate. The organic layer was washed three times with saturated brine, dried over magnesium sulfate and filtered, and the solvent was evaporated. The obtained crude product was purified by silica gel column chromatography to give N-benzyl-4-phenylbutanethioamide (0.533 g, 1.98 mmol).

### Example 82

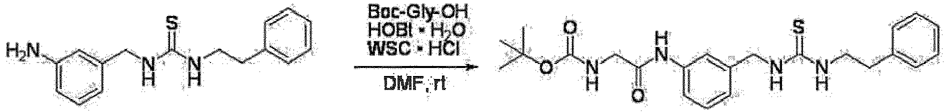

Under conditions similar to those in Example 80, step 1, 1-(3-aminobenzyl)-3-(2-phenylethyl)thiourea obtained in Example 79 was reacted with N-(tert-butoxycarbonyl)glycine (Boc-Gly-OH) to give tert-butyl (3-{[3-(2-phenylethyl)thioureido]methyl}phenylcarbamoyl)methylcarbamate.

### Example 83

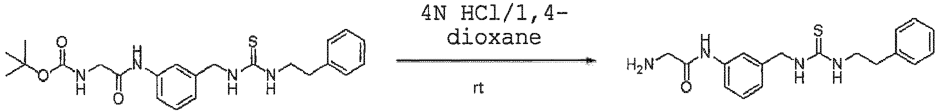

Under conditions similar to those in Example 79, tert-butyl (3-{[3-(2-phenylethyl)thioureido]methyl}phenylcarbamoyl)methylcarbamate was deprotected with 4N hydrogen chloride/1,4-dioxane solution to give 1-{3-[(2-aminoacetyl)amino]benzyl}-3-(2-phenylethyl)thiourea.

### Example 86

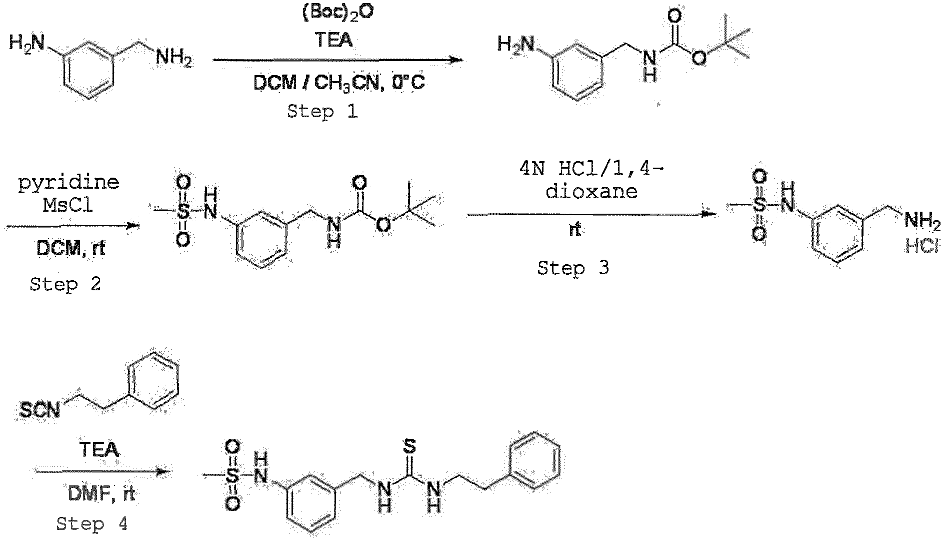

### step 1

3-Aminobenzylamine (1.222 g, 10.0 mmol) was dissolved in dichloromethane (25 mL) and acetonitrile (10 mL), triethylamine (3.1 mL, 22.0 mmol) was added, and the mixture was stirred in an ice bath. Thereto was slowly added a solution of di-tert-butyl dicarbonate (2.183 g, 10.0 mmol) in dichloromethane (3.5 mL), and the mixture was further stirred. After completion of the reaction, the solvent was evaporated to quantitatively give a crude product of tert-butyl 3-aminobenzylcarbamate.

### step 2

tert-Butyl 3-aminobenzylcarbamate (1.227 g, 5.5 mmol) was dissolved in dichloromethane (20 mL), pyridine (2.8 mL, 33.0 mmol) and methanesulfonyl chloride (0.5 mL, 6.6 mmol) were added, and the mixture was stirred at room temperature. After completion of the reaction, the organic layer was washed with 1M hydrochloric acid, dried over magnesium sulfate and filtered, and the solvent was evaporated to quantitatively give a crude product of tert-butyl N-[3-(methanesulfonamido)benzyl]carbamate.

### step 3

Under conditions similar to those in Example 79, tert-butyl N-[3-(methanesulfonamido)benzyl]carbamate was deprotected with 4N hydrogen chloride/1,4-dioxane solution to give N-[3-(aminomethyl)phenyl]methanesulfonamide hydrochloride.

### step 4

Under conditions similar to those in Example 40, N-[3-(aminomethyl)phenyl]methanesulfonamide hydrochloride was reacted with phenethyl isothiocyanate to give 1-[3-(mesylamino)benzyl]-3-(2-phenylethyl)thiourea.

### Example 89

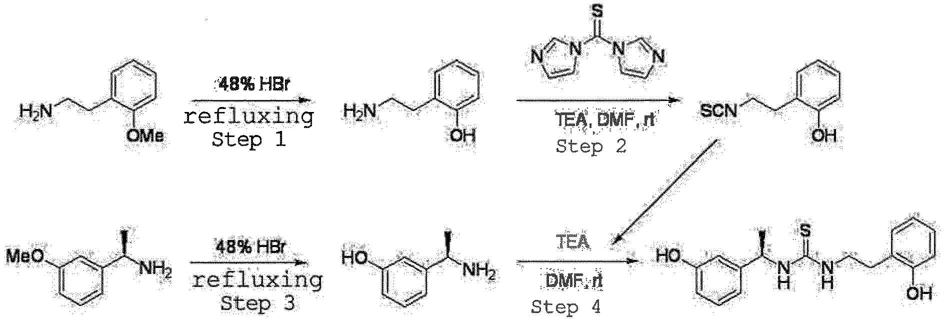

### step 1

Under conditions similar to those in Example 41, step 1, 2-(2-methoxyphenyl)ethylamine was reacted in 48% aqueous hydrobromic acid solution to give 2-(2-hydroxyphenyl)ethylamine.

### step 2

1,1-Thiocarbonyldiimidazole (1.283 g, 7.2 mmol) was dissolved in N,N-dimethylformamide (15 mL), and the mixture was stirred at 50°C. Thereto was added a solution of 2-(2-hydroxyphenyl)ethylamine (0.823 g, 6.0 mmol) and triethylamine (1.0 mL, 7.2 mmol) in N,N-dimethylformamide (15 mL), and the mixture was stirred at room temperature for 2 hr. After completion of the reaction, the solvent was evaporated, and the residue was diluted with ethyl acetate. The mixture was washed with water and saturated brine, the organic layer was dried over magnesium sulfate and filtered, and the solvent was evaporated. The obtained crude product was purified by silica gel column chromatography to give 2-(2-hydroxyphenyl)ethyl isothiocyanate (0.592 g, 3.3 mmol).

### step 3

Under conditions similar to those in Example 41, step 1, (R)-1-(3-methoxyphenyl)ethylamine was reacted in 48% aqueous hydrobromic acid solution to give (R)-1-(3-hydroxyphenyl)ethylamine.

### step 4

Under conditions similar to those in Example 40, (R)-1-(3-hydroxyphenyl)ethylamine was reacted with 2-(2-hydroxyphenyl)ethyl isothiocyanate to give 1-[2-(2-hydroxyphenyl)ethyl]-3-[(R)-1-(3-hydroxyphenyl)ethyl]thiourea.

### Example 92

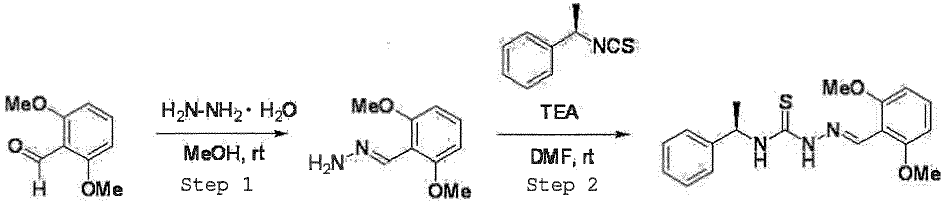

### step 1

Hydrazine monohydrate (3.0 mL, 60.0 mmol) was dissolved in methanol (4 mL), 2,6-dimethoxybenzaldehyde (2.493 g, 15.0 mmol) was added, and the mixture was stirred at room temperature. After completion of the reaction, the solvent was evaporated, and the residue was crystallized from ethyl acetate-hexane mixed solvent to give 2,6-dimethoxybenzaldehyde hydrazone (1.045 g, 5.8 mmol).

### step 2

Under conditions similar to those in Example 40, 2,6-dimethoxybenzaldehyde hydrazone was reacted with (R)-1-phenylethyl isothiocyanate to give (E)-1-(2,6-dimethoxybenzylidene)-4-((R)-1-phenylethyl)thiosemicarbazide.

### Example 93

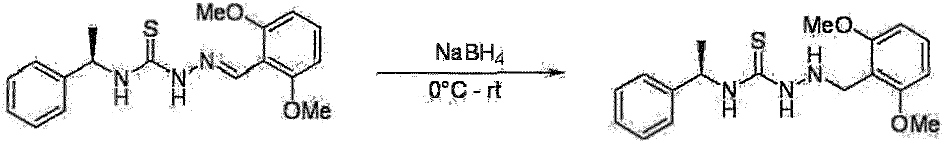

(E)-1-(2,6-dimethoxybenzylidene)-4-((R)-1-phenylethyl)thiosemicarbazide (1.596 g, 4.65 mmol) obtained in Example 92 was dissolved in methanol (45 mL) and, in an ice bath, sodium borohydride (5.275 g, 139.4 mmol) was added, and the mixture was reacted at room temperature. After completion of the reaction, the mixture was adjusted to pH about 2 by adding 2M hydrochloric acid, concentrated, and adjusted to pH about 11 by adding 2M aqueous sodium hydroxide solution. The mixture was extracted with ethyl acetate, the organic layer was dried over magnesium sulfate and filtered, and the solvent was evaporated. The obtained crude product was purified by silica gel column chromatography to give 1-(2,6-dimethoxybenzyl)-4-((R)-1-phenylethyl)thiosemicarbazide (0.999 g, 2.9 mmol).

### Example 96

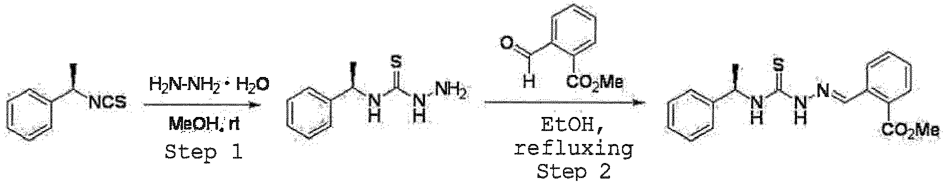

### step 1

To hydrazone monohydrate (1.101 g, 22.0 mmol) was added methanol (30 mL), a solution of ((R)-1-phenylethyl)isothiocyanate (3.265 g, 20.0 mmol) in methanol (30 mL) was further added, and the mixture was reacted at room temperature. After completion of the reaction, the solvent was evaporated to quantitatively give a crude product of 4-((R)-1-phenylethyl)thiosemicarbazide.

### step 2

Methyl 2-formylbenzoate (0.903 g, 5.5 mmol) was dissolved in ethanol (5 mL), a solution of 4-((R)-1-phenylethyl)thiosemicarbazide (0.976 g, 5.0 mmol) in ethanol (10 mL) was added, and the mixture was refluxed. After completion of the reaction, the solvent was evaporated, and the obtained crude product was purified by silica gel column chromatography to give methyl 2-{(E)-[4-((R)-1-phenylethyl)thiosemicarbazono]methyl}benzoate (1.539 g, 4.5 mmol).

### Example 97

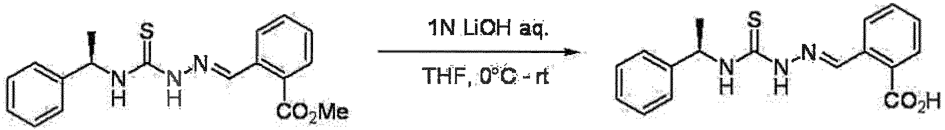

Methyl 2-{(E)-[4-((R)-1-phenylethyl)thiosemicarbazido]methyl}benzoate (0.410 g, 1.2 mmol) was dissolved in tetrahydrofuran (7 mL), 1N aqueous lithium hydroxide solution (2.4 mL) was added, and the mixture was allowed to naturally warm from 0°C to room temperature, and reacted for 2 hr. After completion of the reaction, the mixture was adjusted to pH about 2 with 1M hydrochloric acid, and extracted with ethyl acetate. The obtained organic layer was washed with saturated brine, dried over magnesium sulfate and filtered, and the solvent was evaporated. The obtained crude product was purified by silica gel column chromatography to give 2-{(E)-[4-((R)-1-phenylethyl)thiosemicarbazono]methyl}benzoic acid (0.280 g, 0.86 mmol).

### Example 102

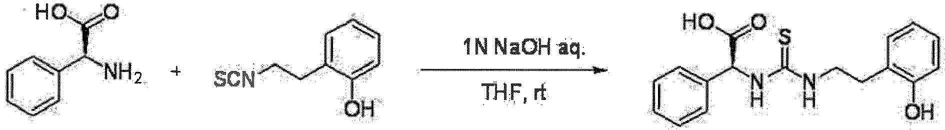

(L)-phenylglycine (0.151 g, 1.0 mmol) and 2-(2-hydroxyphenyl)ethyl isothiocyanate (0.179 g, 1.0 mmol) were dissolved in tetrahydrofuran (2 mL), 1N aqueous sodium hydroxide solution (1.1 mL) was added, and the mixture was reacted at room temperature for 2 hr. After completion of the reaction, the mixture was adjusted to pH about 2 by adding 1M hydrochloric acid, and extracted with ethyl acetate. The obtained organic layer was washed with saturated brine, dried over magnesium sulfate and filtered, and the solvent was evaporated. The obtained crude product was purified by silica gel column chromatography to give (S)-2-{3-[2-(2-hydroxyphenyl)ethyl]thioureido}-2-phenylacetic acid (0.123 g, 0.37 mmol).

### Example 105

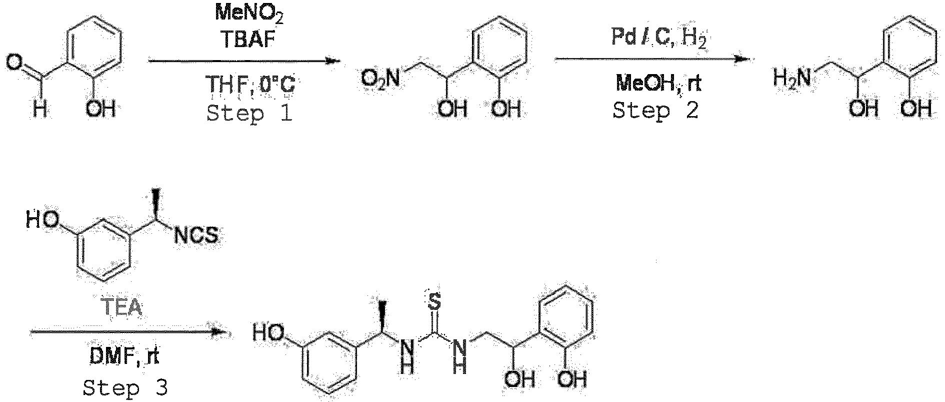

### step 1

Salicylaldehyde (4.885 g, 40.0 mmol) was dissolved in THF (25 mL) and nitromethane (11.2 mL, 208.0 mmol) was added in an ice bath. Furthermore, 1M tetrabutylammonium fluoride-THF solution (44 mL) was added, and the mixture was stirred for 1 hr. After completion of the reaction, the mixture was diluted with ethyl acetate, and washed with water and saturated brine. The organic layer was dried over magnesium sulfate and filtered, and the solvent was evaporated to give a crude product 1-(2-hydroxyphenyl)-2-nitroethanol containing a residual starting material by 6% (confirmed by ¹H-NMR).

### step 2

1-(2-Hydroxyphenyl)-2-nitroethanol was dissolved in methanol (200 mL), 5% palladium/carbon (4 g) was added, and the mixture was stirred at room temperature overnight under a hydrogen atmosphere. After completion of the reaction, the mixture was filtered, and the solvent was evaporated. The obtained crude product was purified by silica gel column chromatography to give 2-amino-1-(2-hydroxyphenyl)ethanol (3.42 g, 22.3 mmol).

### step 3

Under conditions similar to those in Example 40, 2-amino-1-(2-hydroxyphenyl)ethanol was reacted with (R)-1-(3-hydroxyphenyl)ethyl isothiocyanate to give 1-[2-(2-hydroxyphenyl)-2-hydroxyethyl]-3-[(R)-1-(3-hydroxyphenyl)ethyl]thiourea.

### Example 106

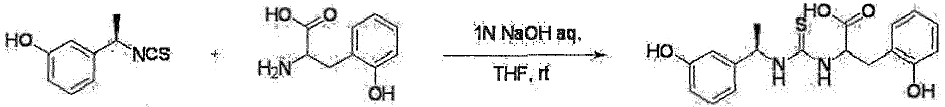

(R)-1-(3-hydroxyphenyl)ethyl isothiocyanate (0.122 g, 0.68 mmol) and 2-hydroxyphenylalanine (0.123 g, 0.68 mmol) were dissolved in tetrahydrofuran (2 mL), 1N aqueous sodium hydroxide solution (0.8 mL) was added, and the mixture was reacted at room temperature for 2 hr. After completion of the reaction, the mixture was adjusted to pH about 2 by adding 1M hydrochloric acid, and extracted with ethyl acetate. The obtained organic layer was washed with saturated brine, dried over magnesium sulfate and filtered, and the solvent was evaporated. The obtained crude product was purified by silica gel column chromatography to give 2-{3-[(R)-1-(3-hydroxyphenyl)ethyl]thioureido}-3-(2-hydroxyphenyl)propanoic acid (0.180 g, 0.50 mmol).

### Example 121

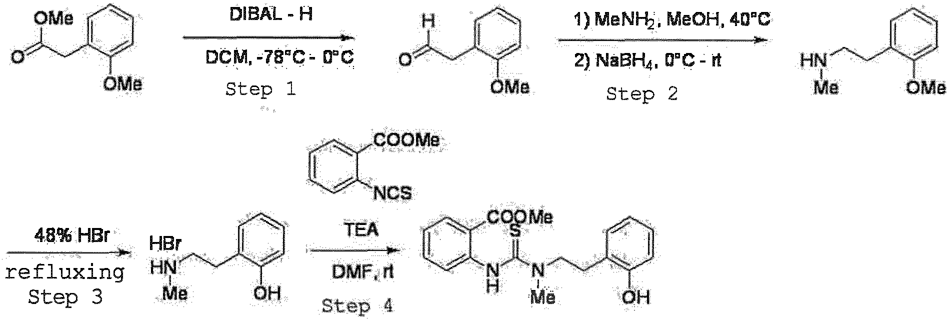

### step 1

(2-Methoxyphenyl)methyl acetate (5.406 g, 30.0 mmol) was dissolved in dichloromethane (150 mL), and the mixture was cooled to -78°C. 1.0M Diisobutylaluminum hydride-hexane solution (36 mL) was added, and the mixture was stirred at 0°C for 1 hr. After completion of the reaction, 1M hydrochloric acid was added, and the mixture was further stirred for 1 hr. Thereafter, the mixture was diluted with dichloromethane, the organic layer was washed with water and saturated brine, dried over magnesium sulfate and filtered, and the solvent was evaporated. The obtained crude product was purified by silica gel column chromatography to give (2-methoxyphenyl)acetaldehyde (2.935 g, 19.5 mmol).

### step 2

(2-Methoxyphenyl)acetaldehyde (1.502 g, 10.0 mmol) was dissolved in methanol (30 mL), 9.8M methylamine-methanol solution (1.53 mL, 15.0 mmol) was added, and the mixture was stirred at 40°C for 2 hr. Thereafter, in an ice bath, sodium borohydride (0.757 g, 20.0 mmol) was added, and the mixture was reacted at room temperature. After 1 hr, saturated aqueous sodium hydrogen carbonate solution was added to discontinue the reaction, and the reaction mixture was diluted with ethyl acetate. The organic layer was washed with saturated brine, dried over magnesium sulfate and filtered, and the solvent was evaporated. The obtained crude product was purified by silica gel column chromatography to give N-methyl-(2-methoxyphenyl)ethylamine (0.929 g, 5.6 mmol).

### step 3

Under conditions similar to those in Example 41, step 1, N-methyl-(2-methoxyphenyl)ethylamine was reacted in 48% aqueous hydrobromic acid solution to give N-methyl-(2-hydroxyphenyl)ethylamine hydrobromide.

### step 4

Under conditions similar to those in Example 40, methyl 2-isothiocyanatobenzoate was reacted with N-methyl-(2-hydroxyphenyl)ethylamine hydrobromide to give methyl 2-{3-methyl-3-[2-(2-hydroxyphenyl)ethyl]thioureido}benzoate.

### Example 123

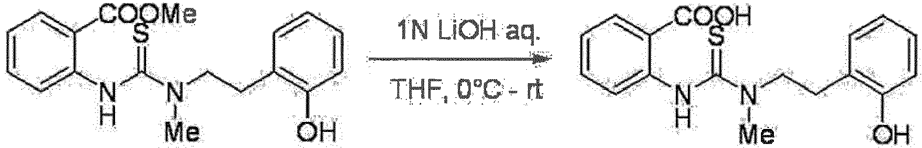

Under conditions similar to those in Example 97, methyl 2-{3-methyl-3-[2-(2-hydroxyphenyl)ethyl]thioureido}benzoate obtained in Example 121 was hydrolyzed with lithium hydroxide to give 2-{3-methyl-3-[2-(2-hydroxyphenyl)ethyl]thioureido}benzoic acid.

### Example 125

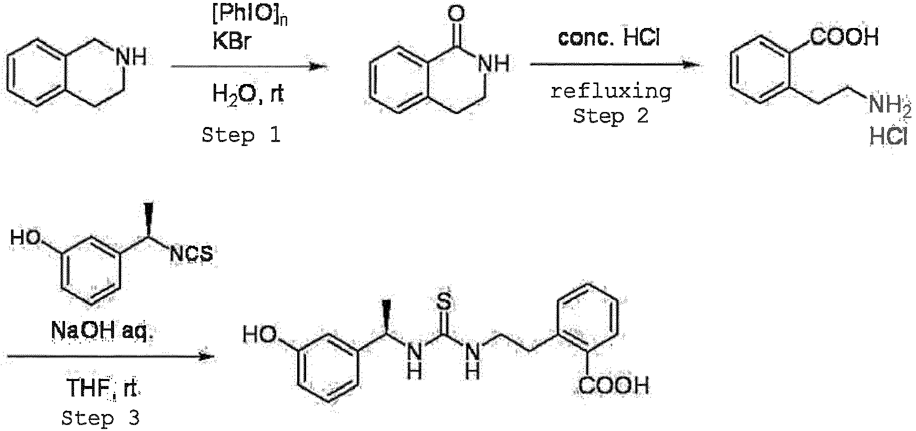

### step 1

1,2,3,4-Tetrahydroisoquinoline (1.0 g, 7.6 mol) was dissolved in water (40 mL), iodosobenzene (5.0 g, 22.7 mmol) and potassium bromide (0.902 g, 7.58 mmol) were added, and the mixture was reacted at room temperature overnight. After completion of the reaction, saturated aqueous sodium hydrogen carbonate solution was added, and the mixture was extracted with dichloromethane. The organic layer was dried over magnesium sulfate and filtered, and the solvent was evaporated. The obtained crude product was purified by silica gel column chromatography to give 3,4-dihydroisoquinoline-1(2H)-one (0.715 g, 4.9 mmol).

### step 2

To 3,4-dihydroisoquinoline-1(2H)-one (0.715 g, 4.9 mmol) was added concentrated hydrochloric acid (10 mL), and the mixture was refluxed for 2 days. Thereafter, the mixture was concentrated and dried to give 2-(2-aminoethyl)benzoic acid hydrochloride (0.688 g, 3.4 mmol).

### step 3

Under conditions similar to those in Example 106, 2-(2-aminoethyl)benzoic acid hydrochloride was reacted with (R)-1-(3-hydroxyphenyl)ethyl isothiocyanate to give 2-(2-{3-[(R)-1-(3-hydroxyphenyl)ethyl]thioureido}ethyl)benzoic acid.

### Experimental Example 1 Measurement of ENaC activation potency (stimulation activity) of the compound of the present invention

ENaC activation current value, namely, the inward current in ENaC-expressing oocyte was measured as follows.

According to the method described in Motonao Nakamura, Takao Shimizu: Experiment of African clawed frog oocyte, Experimental Medicine Vol. 11, No. 3, 224-232 (1993), oocyte was collected from African clawed frog, microinjected into cRNA of oocyte, and the current value was measured by a Two Electrode Voltage Clamp technique. In the present invention, ND96 (96 mM NaCl, 2 mM KCl, 1 mM MgCl₂, 5 mM Hepes, 1.8 mM CaCl₂, pH 7.6) was used instead of MBS buffer in the above-mentioned document. Injector NANOLITER 2000 manufactured by World Precision Instruments was used for microinjection, OpusXpress 6000A manufactured by Molecular Devices was used for current value measurement by a Two Electrode Voltage Clamp technique, and Clampfit 10.2 software manufactured by Molecular Devices was used for data analysis.

Human ENaC δ, β and γ subunit genes shown by SEQ ID NOs: 1, 2 and 3, respectively, in the Sequence Listing were each cloned into a plasmid vector, and used as a template DNA for cRNA synthesis. cRNA was synthesized using MEGAscript kit manufactured by Ambion and according to the method of the manual of the manufacturer.

A mixture of the same amount of ENaC δ subunit cRNA (0.4 to 0.8 µg/µL), and β and γ subunits cRNA (0.4 µg/µL) was injected into the oocyte of African clawed frog at 27.6 nL per oocyte, and the oocyte was cultured for 16 hr-72 hr. Thereafter, the oocyte was set on a measuring apparatus OpusXpress 6000A, the electrodes were inserted into the oocyte, the voltage was clamped at a level lower by -30 mV than the resting membrane potential by a Two Electrode Voltage Clamp technique, and the current value was measured.

To measure the ENaC activation potency, oocyte was stimulated with each test compound, and the minimum effective dose (hereinafter to be abbreviated as MED) of the compound at the time point when the electric current started to increase was measured. In this case, whichever smaller value of the minimum effective dose of the stimulation compound that increased the current value by not less than 10%, and the minimum effective concentration of the compound that increased, by not less than 25%, the electric current increase value due to the stimulation by 5 µM capsazepine (CAS NO.: 138977-28-3), as compared to the current value before addition of the compound and without stimulation, was taken as MED. A smaller MED means higher ENaC activation potency.

Each test compound was directly dissolved in ND96, or first dissolved in dimethyl sulfoxide (DMSO) to 100 mM, and then prepared by diluting the solution with ND96 to a concentration used for the evaluation. The concentration of the test compound in ND96 was prepared to various concentrations within the range of 0.1 nM to 300 µm. DMSO at a concentration contained in the prepared liquid does not influence the current value of ENaC.

ND96 as a perfusion fluid was flown at a rate of 0.5 mL/min through the measuring apparatus OpusXpress 6000A on which ENaC-expressing oocyte was set, the perfusion fluid was temporarily stopped every 3 minutes to add a test compound solution (0.25 mL) dissolved in ND96 at a rate of 0.5 mL/min. In this way, the test compound was contacted with ENaC-expressing oocyte, and whether ENaC is activated by the compound was examined by measuring the current value. After addition of the test compound, feeding of the perfusion fluid was resumed to wash away the compound to be ready for the next addition of the compound.

The compound was added successively from a low concentration to a high concentration to stimulate ENaC-expressing oocyte. The concentrations actually added was, for example, 1 nM, 3 nM, 10 nM, 30 nM, 100 nM, 300 nM; 1 µM, 3 µM, 10 µm, 30 µm, 100 µm, 300 µm; 1.2 µM, 6 µM, 30 µm, 150 µm of one test compound. The compound was added from low concentrations to high concentrations at 3 min intervals. The first compound was added from a low concentration to a high concentration, then the second and the third compounds were similarly added successively to the same oocyte, and changes in the current value were measured. The current value was continuously measured from the start to the end of the experiment.

The measurement results of MED are shown in the following Tables 2-1 to 2-5.

**[Table 2-1]**

| compound | MED (µM) |
|---|---|
| capsazepine | 3 |
| Example 1 | 1 |
| Example 2 | 3 |
| Example 3 | 30 |
| Example 4 | 30 |
| Example 5 | 1 |
| Example 6 | 30 |
| Example 7 | 5 |
| Example 8 | 1 |
| Example 9 | 3 |
| Example 11 | 0.3 |
| Example 12 | 10 |
| Example 13 | 10 |
| Example 14 | 3 |
| Example 15 | 30 |
| Example 16 | 3 |
| Example 17 | 3 |
| Example 18 | 10 |
| Example 19 | 3 |
| Example 20 | 3 |
| Example 21 | 1 |
| Example 22 | 30 |
| Example 23 | 10 |
| Example 24 | 3.3 |
| Example 25 | 10 |
| Example 26 | 3.3 |
| Example 27 | 10 |
| Example 28 | 1-3 |

**[Table 2-2]**

| compound | MED (µM) |
|---|---|
| Example 29 | 3.3 |
| Example 30 | 30 |
| Example 31 | 1 |
| Example 32 | 10 |
| Example 33 | 3 |
| Example 34 | 10 |
| Example 35 | 3 |
| Example 36 | 30 |
| Example 37 | 3 |
| Example 38 | 0.1 |
| Example 39 | 1 |
| Example 40 | 1 |
| Example 41 | 3 |
| Example 42 | 1 |
| Example 43 | 1 |
| Example 44 | 1 |
| Example 45 | 3 |
| Example 46 | 3 |
| Example 47 | 5 |
| Example 48 | 1 |
| Example 49 | 30 |
| Example 50 | 3 |
| Example 51 | 0.3 |
| Example 52 | 1 |
| Example 53 | 30 |
| Example 54 | 1 |
| Example 55 | 30 |
| Example 56 | 10 |

**[Table 2-3]**

| compound | MED (µM) |
|---|---|
| Example 57 | 1 |
| Example 58 | 3.3 |
| Example 59 | 10 |
| Example 60 | 10 |
| Example 61 | 30 |
| Example 62 | 1.1 |
| Example 63 | 3.3 |
| Example 64 | 30 |
| Example 65 | 3.3 |
| Example 66 | 3-10 |
| Example 67 | 10 |
| Example 68 | 3 |
| Example 69 | 1 |
| Example 70 | 10 |
| Example 71 | 1 |
| Example 72 | 1 |
| Example 73 | 3-10 |
| Example 74 | 3-10 |
| Example 75 | 1 |
| Example 76 | 10 |
| Example 77 | 1 |
| Example 78 | 10 |
| Example 79 | 1 |
| Example 80 | 10 |
| Example 81 | 10 |
| Example 82 | 3 |
| Example 83 | 1 |
| Example 84 | 10 |

**[Table 2-4]**

| compound | MED (µM) |
|---|---|
| Example 85 | 0.6 |
| Example 86 | 1 |
| Example 87 | 10 |
| Example 88 | 0.6-1 |
| Example 89 | 0.06 |
| Example 90 | 1 |
| Example 91 | 1 |
| Example 92 | 30 |
| Example 93 | 30 |
| Example 94 | 3-10 |
| Example 95 | 3-10 |
| Example 96 | 30 |
| Example 97 | 1 |
| Example 98 | 1 |
| Example 99 | 10 |
| Example 100 | 1 |
| Example 101 | 1 |
| Example 102 | 1 |
| Example 103 | 3 |
| Example 104 | 0.06-0.1 |
| Example 105 | 0.06-0.1 |
| Example 106 | 0.1-0.3 |
| Example 107 | 0.3 |
| Example 108 | 0.3 |
| Example 109 | 0.3 |
| Example 110 | 0.3 |
| Example 111 | 0.06-0.1 |

**[Table 2-5]**

| compound | MED (µM) |
|---|---|
| Example 112 | 0.1 |
| Example 113 | 0.3 |
| Example 114 | 0.1 |
| Example 115 | 0.06-0.1 |
| Example 116 | 0.1 |
| Example 117 | 0.3 |
| Example 118 | 0.1 |
| Example 119 | 3 |
| Example 120 | 0.1-0.3 |
| Example 123 | 0.06 |
| Example 124 | 0.3 |
| Example 125 | 0.06-0.1 |
| Example 126 | 10 |

Thus, the compound of the present invention was confirmed to have an ENaC activating action.

### Experimental Example 2 Sensory evaluation of salty taste enhancing activity of capsazepine in human

The presence or absence of a salty taste enhancing activity in human was examined by a quantitative sensory evaluation test for capsazepine having an ENaC activation action.

The quantitative sensory evaluation test was performed as follows. Distilled water containing sodium chloride (0.8 g/dl) was mixed with capsazepine as a sample at 0.0001 to 0.001 g/dl, and the level of a salty taste enhancing activity was measured. Distilled water containing 0.8 g/dl of sodium chloride was used as a control, and distilled water containing 0.9 g/dl or 1.0 g/dl of sodium chloride was used as a comparison target. Using a linear measurement method, a straight line showing 1.0-fold (0.8 g/dl), 1.13-fold (0.9 g/dl), 1.25-fold (1.0 g/dl) positions of sodium chloride concentration was drawn and the position showing the strength of the corresponding salty taste was put down thereon. As for the sensory evaluation, an average of the positions put down by the panelists was determined, and an increase in the level of salty taste relative to the level of salty taste in the control was indicated as a salty taste enhancing rate (fold). The panelists were those who had an experience of developing flavoring of foodstuffs for a total of one year or more, and capable of distinguishing sodium chloride solutions having different concentrations of 0.8 g/dl, 0.9 g/dl, 1.0 g/dl (periodically confirmed). The "initial taste" means the evaluation of the strength of the salty taste for 2 seconds after placing in the mouth, and the "middle and after taste" means a combination of middle taste and after taste, which is the evaluation of the strength of the salty taste after 2 seconds from placing in the mouth. The results are shown in the following Table 3.

From Table 3, it was found that capsazepine having an ENaC activation action has a salty taste enhancing activity in human.

**Table 3**

| sample | concentration (g/dl) | strength of salty taste | | evaluation comments |
|---|---|---|---|---|
| | | initial taste | middle and after taste | |
| control | - | 1 | 1 | - |
| capsazepine | 0.0001 | 1.04 | 1.05 | salty taste becomes slightly stronger |
| | 0.0005 | 1.11 | 1.08 | salty taste is strengthened from initial taste but bitter taste also accompanies |
| | 0.001 | 1.11 | 1.11 | salty taste is strengthened but bitter taste is considerably strong |

### Experimental Example 3 Sensory evaluation of salty taste enhancing activity of compound of the present invention in human

The intensity of a salty taste enhancing activity and the strength of a bitter taste were examined by a quantitative sensory evaluation test for the compound of the present invention (compounds of Examples 11 and 51).

The quantitative sensory evaluation test was performed as follows. Distilled water containing sodium chloride (0.8 g/dl) was mixed with capsazepine as a sample at 0.001 g/dl, or the compound of Example 11 or Example 51 at 0.0001 to 0.001 g/dl, and the level of a salty taste enhancing activity was measured. Distilled water containing 0.8 g/dl of sodium chloride was used as a control, and distilled water containing 0.9 g/dl or 1.0 g/dl of sodium chloride was used as a comparison target. Using a linear measurement method, a straight line showing 1.0-fold (0.8 g/dl), 1.13-fold (0.9 g/dl), 1.25-fold (1.0 g/dl) positions of sodium chloride concentration was drawn and the position showing the strength of the corresponding salty taste was put down thereon. As for the strength of a bitter taste, using the bitter taste of a solution obtained by mixing distilled water containing sodium chloride (0.8 g/dl) with capsazepine at 0.0001 to 0.001 g/dl as the standard, the strength of the bitter taste of the solution obtained by mixing the compound of Example 11 or Example 51 at 0.0001 to 0.001 g/dl was relatively evaluated by comparison. The points were marked based on 0 point for no bitter taste, and 5.0 points for the strength of the bitter taste of a solution containing 0.001 g/dl capsazepine. The test was performed at n=5. The panelists were those who had an experience of developing flavoring of foodstuffs for a total of one year or more, and capable of distinguishing sodium chloride solutions having different concentrations of 0.8 g/dl, 0.9 g/dl, 1.0 g/dl (periodically confirmed). The "initial taste" means the evaluation of the strength of the salty taste for 2 seconds after placing in the mouth, and the "middle and after taste" means a combination of middle taste and after taste, which is the evaluation of the strength of the salty taste after 2 seconds from placing in the mouth.

The results are shown in the following Table 4, the results of a bitter taste evaluation are shown in the following Table 5.

**[Table 4]**

| sample | concentration (g/dl) | salty taste enhancing rate (fold) | |
|---|---|---|---|
| | | initial taste | middle and after taste |
| control | - | 1 | 1 |
| capsazepine | 0.0001 | 1.11 | 1.11 |
| compound of Example 11 | 0.0001 | 1.10 | 1.10 |
| | 0.0005 | 1.15 | 1.14 |
| | 0.001 | 1.13 | 1.14 |
| compound of Example 51 | 0.0001 | 1.09 | 1.11 |
| | 0.0005 | 1.13 | 1,17 |
| | 0.001 | 1.17 | 1.17 |

**[Table 5]**

| sample | concentration (g/dl) | strength of bitter taste |
|---|---|---|
| control | - | 0 |
| capsazepine | 0.0001 | 0.8 |
| | 0.0005 | 2.6 |
| | 0.001 | 5.0 |
| compound of Example 11 | 0.0001 | 0.09 |
| | 0.0005 | 0.12 |
| | 0.001 | 0.16 |
| compound of Example 51 | 0.0001 | 0.01 |
| | 0.0005 | 0.11 |
| | 0.001 | 0.40 |

From the above results, it was clarified that the compound of the present invention has an equivalent or stronger salty taste enhancing effect as compared to capsazepine, and reduces a bitter taste more than capsazepine. Therefore, the compound of the present invention has extremely superior properties as a salty taste enhancer.

### Industrial Applicability

According to the present invention, a compound having a strong salty taste enhancing effect and a less bitter taste can be provided. Since the salty taste enhancer of the present invention can be used for salt reduction, it is useful for various foods or drinks (particularly, food or drink or seasoning for subjects who desire to limit the ingestion dose of sodium chloride).

This application is based on patent application No. 2011-049469 filed in Japan, the contents of which are encompassed in full herein.

## Claims

1. A salty taste enhancer for a food or drink, which comprises a compound represented by the following formula: 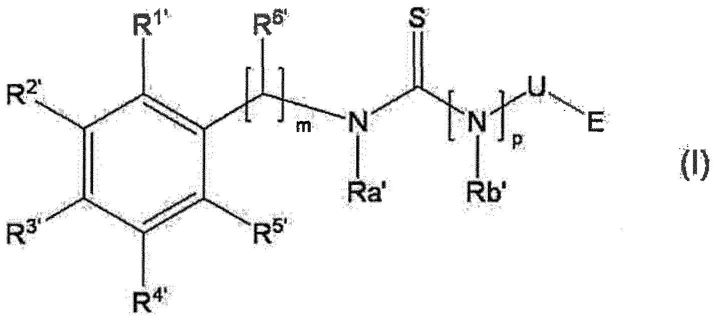 wherein
R¹' to R⁵' are each independently (i) a hydrogen atom, (ii) a hydroxyl group, (iii) a halogen atom, (iv) a carboxyl group, (v) a C₁₋₄ alkoxy-carbonyl group, (vi) a C₁₋₄ alkoxy group, (vii) an amino group optionally substituted by a C₁₋₄ alkoxy-carbonyl group, (viii) a cyano group, (ix) a nitro group, (x) a C₁₋₄ alkyl group optionally substituted by 1 to 3 halogen atoms, (xi) a C₁₋₄ alkyl-carbonylamino group optionally substituted by an amino group optionally substituted by a C₁₋₄ alkoxy-carbonyl group, (xii) a C₁₋₄ alkylsulfonylamino group, or (xiii) a C₆₋₁₀ arylsulfonylamino group optionally substituted by a C₁₋₄ alkyl group, or
any two from R¹' to R⁵' are optionally joined to form a C₁₋₄ alkylenedioxy group,
R⁶' is (i) a hydrogen atom, (ii) a hydroxyl group, (iii) a C₁₋₄ alkyl group optionally substituted by 1 to 3 substituents selected from a hydroxyl group, a C₁₋₄ alkoxy group and a C₇₋₁₄ aralkyloxy group, (iv) a C₁₋₄ alkoxy group, (v) a carboxyl group, (vi) a C₁₋₄ alkoxy-carbonyl group, (vii) a C₆₋₁₀ aryl group, or (viii) a carbamoyl group, or
R⁶' and R⁵' are optionally joined to form a C₁₋₄ alkylene group, m is an integer of 0 to 2,
Ra' is a hydrogen atom, a hydroxyl group, a C₁₋₄ alkyl group or a C₇₋₁₄ aralkyl group, or
Ra' and R⁵' are optionally joined to form a C₁₋₄ alkylene group,
Rb' is a hydrogen atom, a hydroxyl group, a C₁₋₄ alkyl group or a C₇₋₁₄ aralkyl group, or
Rb' and R⁶' are optionally joined to form a carbonyl group,
p is 0 or 1,
U is a single bond, -NH-CH₂-, -N=CR¹²' - wherein R¹²' is a hydrogen atom or a C₁₋₄ alkyl group, or a group represented by the following formula: 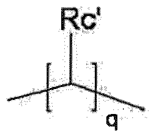
wherein
Rc' is (i) a hydrogen atom, (ii) a hydroxyl group, (iii) a C₁₋₄ alkyl group optionally substituted by 1 to 3 substituents selected from a hydroxyl group, a C₁₋₄ alkoxy group and a C₇₋₁₄ aralkyloxy group, (iv) a C₁₋₄ alkoxy group, (v) a carboxyl group, (vi) a C₁₋₄ alkoxy-carbonyl group, (vii) a C₆₋₁₀ aryl group, or (viii) a carbamoyl group, and
q is an integer of 1 to 3, and
E is a hydroxyl group, an amino group, a carbamoyl group, or a C₃₋₁₀ cycloalkyl group, a C₆₋₁₄ aryl group, or an aromatic heterocyclic group, each of which is optionally substituted by 1 to 3 substituents selected from (a) a hydroxyl group, (b) a halogen atom, (c) a carboxyl group, (d) a C₁₋₄ alkoxy-carbonyl group, (e) a C₁₋₄ alkoxy group, (f) an amino group optionally substituted by a C₁₋₄ alkoxy-carbonyl group, (g) a cyano group, (h) a nitro group, (i) a C₁₋₄ alkyl group optionally substituted by 1 to 3 halogen atoms, (j) a C₁₋₄ alkyl-carbonylamino group optionally substituted by an amino group optionally substituted by a C₁₋₄ alkoxy-carbonyl group, (k) a C₁₋₄ alkylsulfonylamino group, and (l) a C₆₋₁₀ arylsulfonylamino group optionally substituted by a C₁₋₄ alkyl group,
provided that partial structures: 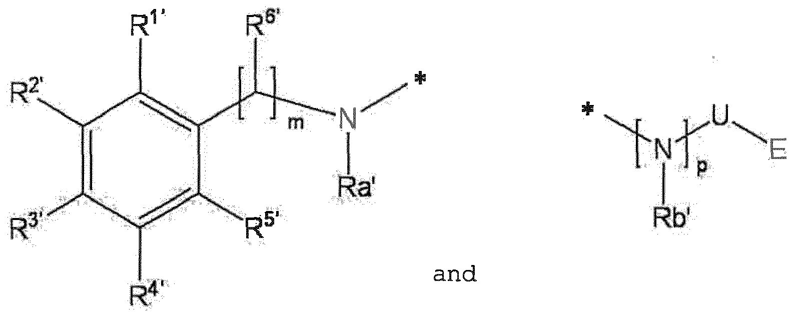 wherein * shows a binding position with a thiocarbonyl group, are not 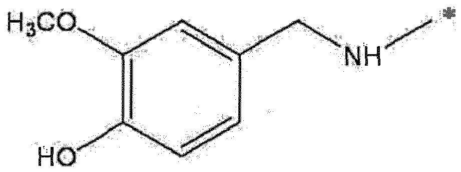 wherein * is as defined above,
or an edible salt thereof.

2. The salty taste enhancer for a food or drink according to claim 1, wherein, in the formula (I),
R¹' to R⁵' are each independently (i) a hydrogen atom, (ii) a hydroxyl group, (iii) a halogen atom, (iv) a carboxyl group, (v) a C₁₋₄ alkoxy-carbonyl group, (vi) a C₁₋₄ alkoxy group, (vii) an amino group, or (viii) a C₁₋₄ alkyl group,
R⁶' is (i) a hydrogen atom, (ii) a C₁₋₄ alkyl group optionally substituted by 1 to 3 substituents selected from a hydroxyl group and a C₁₋₄ alkoxy group, (iii) a carboxyl group, or (iv) a C₁₋₄ alkoxy-carbonyl group,
Ra' is a hydrogen atom, a hydroxyl group or a C₁₋₄ alkyl group,
Rb' is a hydrogen atom, a hydroxyl group or a C₁₋₄ alkyl group,
p is 1,
U is a single bond, -NH-CH₂-, -N=CR¹²' - wherein R¹²' is a hydrogen atom or a C₁₋₄ alkyl group, or a group represented by the following formula: 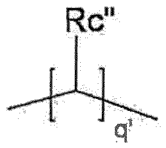 wherein
Rc'' is (i) a hydrogen atom, (ii) a hydroxyl group, (iii) a C₁₋₄ alkyl group optionally substituted by 1 to 3 substituents selected from a hydroxyl group and a C₁₋₄ alkoxy group, (iv) a carboxyl group, or (v) a C₁₋₄ alkoxy-carbonyl group, and
q' is 1 or 2, and
E is a phenyl group or an aromatic heterocyclic group, each of which is optionally substituted by 1 to 3 substituents selected from (a) a hydroxyl group, (b) a halogen atom, (c) a carboxyl group, (d) a C₁₋₄ alkoxy-carbonyl group, (e) a C₁₋₄ alkoxy group, (f) an amino group, and (g) a C₁₋₄ alkyl group.

3. The salty taste enhancer for a food or drink according to claim 1, wherein the compound represented by the formula (I) is 1-(2-phenylethyl)-3-((R)-1-phenylethyl)thiourea.

4. The salty taste enhancer for a food or drink according to claim 1, wherein the compound represented by the formula (I) is 1-((S)-2-hydroxy-1-phenylethyl)-3-(2-phenylethyl)thiourea.

5. A method of adjusting salty taste of a food or drink, which comprises mixing a compound represented by the formula (I) according to claim 1 or an edible salt thereof, with a food or drink.

6. A method of producing a food or drink, which comprises mixing a compound represented by the formula (I) according to claim 1 or an edible salt thereof, with a food or drink.

7. A food or drink having an enhanced salty taste, which comprises not less than an effective amount of a compound represented by the formula (I) according to claim 1 or an edible salt thereof.

8. A food or drink having an enhanced salty taste, which comprises effective amounts of a compound represented by the formula (I) according to claim 1 or an edible salt thereof and potassium chloride.

9. A compound represented by the following formula: 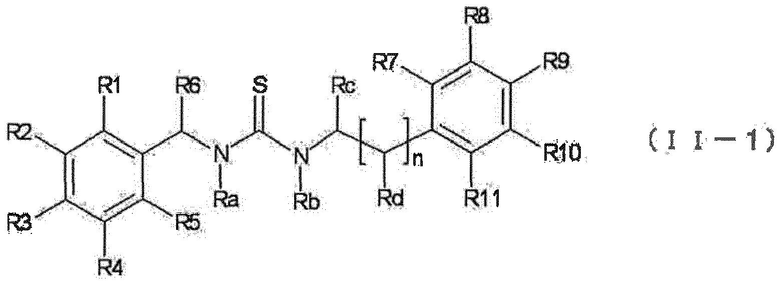 wherein
R¹ to R⁵ are each independently a hydrogen atom, a hydroxyl group, a C₁₋₄ alkoxy group, a halogen atom or an amino group,
R⁶ is (i) a hydrogen atom, (ii) a C₁₋₄ alkyl group optionally substituted by a hydroxyl group or a C₁₋₄ alkoxy group, (iii) a carboxyl group, or (vi) a C₁₋₄ alkoxy-carbonyl group,
Ra and Rb are each independently a hydrogen atom, a hydroxyl group or a methyl group,
Rc is (i) a hydrogen atom, (ii) a carboxyl group, (iii) a C₁₋₄ alkyl group optionally substituted by a hydroxyl group or a C₁₋₄ alkoxy group, or (iv) a C₁₋₄ alkoxy-carbonyl group;
Rd is (i) a hydrogen atom, (ii) a hydroxyl group, or (iii) a C₁₋₄ alkyl group optionally substituted by a hydroxyl group or a C₁₋₄ alkoxy group;
R⁷ to R¹¹ are each independently a hydrogen atom, a hydroxyl group, a carboxyl group, a halogen atom, a C₁₋₄ alkyl group or a C₁₋₄ alkoxy group; and
n is 0 or 1,
provided that
(1) when n is 0, then at least two of R¹ to R⁵ are each a hydroxyl group,
(2) when n is 1 and R⁶ is an unsubstituted C₁₋₄ alkyl group, then at least one of R¹ to R⁵ and R⁷ to R¹¹ is a hydroxyl group, and
(3) when n is 1, R⁶ is a hydrogen atom, Ra is a hydrogen atom or a methyl group, Rc is (i) a hydrogen atom, (ii) a carboxyl group, (iii) a C₁₋₄ alkyl group optionally substituted by a C₁₋₄ alkoxy group, or (iv) a C₁₋₄ alkoxy-carbonyl group, and
Rd is (i) a hydrogen atom, or (ii) a C₁₋₄ alkyl group optionally substituted by a hydroxyl group, then
(i) at least one of R¹ to R⁵ is a hydroxyl group,
(ii) when R³ is a hydroxyl group and R⁴ is a hydrogen atom, then R² is not a methoxy group,
(iii) R⁹ is not a halogen atom or a methoxy group, and
(iv) when Rb is a methyl group, then Rd is not a hydroxyl group,
or an edible salt thereof.

10. A compound represented by the following formula: 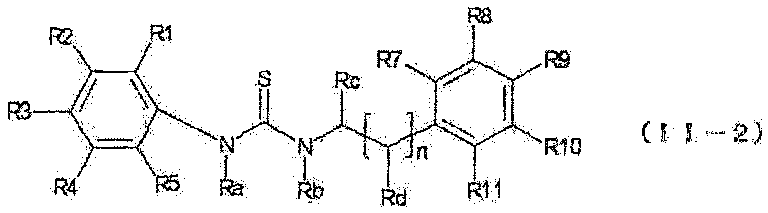 wherein
R¹ is a hydrogen atom, a hydroxyl group, a carboxyl group or a C₁₋₄ alkoxy-carbonyl group,
R² to R⁵ are each independently a hydrogen atom, a hydroxyl group, a C₁₋₄ alkoxy group or an amino group,
Ra and Rb are each independently a hydrogen atom, a hydroxyl group or a C₁₋₄ alkyl group,
Rc and Rd are each independently a hydrogen atom or a C₁₋₄ alkyl group,
R⁷ to R¹¹ are each independently a hydrogen atom, a hydroxyl group, a carboxyl group, a halogen atom, a C₁₋₄ alkyl group or a C₁₋₄ alkoxy group, and
n is 0 or 1,
provided that
(1) when n is 0, then
(i) R¹ is a hydroxyl group or a carboxyl group, and Rc is a C₁₋₄ alkyl group, or
(ii) at least two of R¹ to R⁵ are each a hydroxyl group, and
(2) when n is 1, then
(i) at least two of R¹ to R⁵ are each a hydroxyl group, or
(ii) when R¹ is a carboxyl group or a C₁₋₄ alkoxy-carbonyl group, then (a) Rb is a C₁₋₄ alkyl group, and (b) at least one of R² to R⁵ and R⁷ to R¹¹ is a hydroxyl group,
or an edible salt thereof.

11. A compound represented by the following formula: 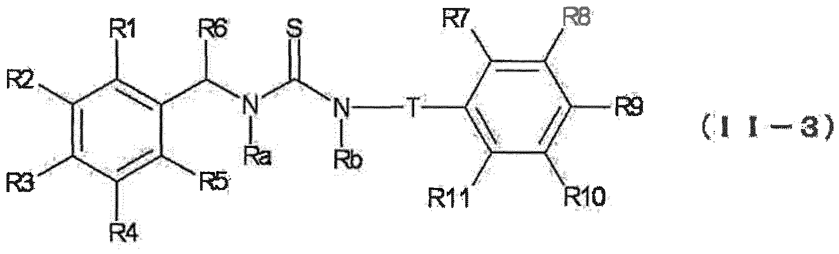 wherein
R¹ to R⁵ are each independently a hydrogen atom, a hydroxyl group, a C₁₋₄ alkoxy group or an amino group,
R⁶ is a C₁₋₄ alkyl group,
Ra and Rb are each independently a hydrogen atom, a hydroxyl group or a C₁₋₄ alkyl group,
T is -NH-CH₂- or -N=CR¹²- wherein R¹² is a hydrogen atom or a C₁₄ alkyl group, and
R⁷ to R¹¹ are each independently a hydrogen atom, a hydroxyl group, a carboxyl group, a halogen atom, a C₁₋₄ alkyl group, a C₁₋₄ alkoxy group or a C₁₋₄ alkoxy-carbonyl group,
provided that
(1) at least one of R⁷ to R¹¹ is not a hydrogen atom, and
(2) when T is -N=CH- and R⁷ is a hydroxyl group, then R¹¹ is not a hydrogen atom,
or an edible salt thereof.

12. A compound represented by the following formula: 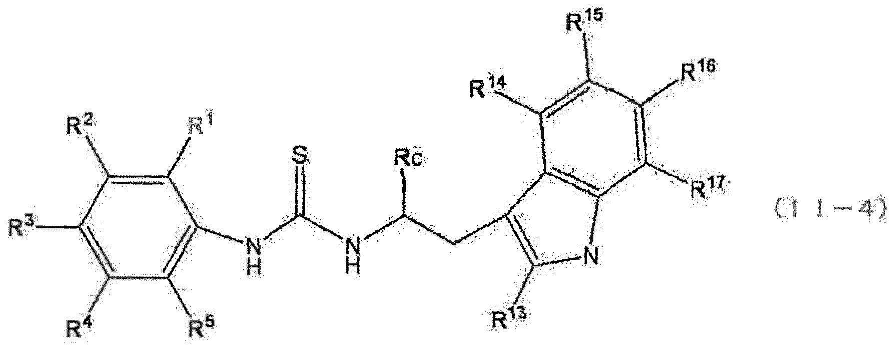 wherein
R¹ to R⁵ are each independently a hydrogen atom, a hydroxyl group, a halogen atom, a carboxyl group, a C₁₋₄ alkyl group, a C₁₋₄ alkoxy group or a C₁₋₄ alkoxy-carbonyl group,
Rc is a hydrogen atom, a carboxyl group, a C₁₋₄ alkyl group optionally substituted by 1 to 3 hydroxyl groups, or a C₁₋₄ alkoxy-carbonyl group, and
R¹³ to R¹⁷ are each independently a hydrogen atom, a hydroxyl group, a halogen atom, a C₁₋₄ alkyl group or a C₁₋₄ alkoxy group, provided that
(i) at least one of R¹ to R⁵ is a hydroxyl group, or
(ii) when R¹ is a C₁₋₄ alkoxy group, then R⁵ is not a hydrogen atom,
or an edible salt thereof.

13. A compound selected from the group consisting of
1-(3,4-dihydroxybenzyl)-3-(2-phenylethyl)thiourea:
N-(2-phenylethyl)-3,4-dihydro-6,7-dihydroxyisoquinoline-2(1H)-carbothioamide;
1-(3,4-dihydroxybenzyl)-1-methyl-3-(2-phenylethyl)thiourea;
1-(4-hydroxybenzyl)-3-(2-phenylethyl)thiourea;
1-(3-hydroxybenzyl)-3-(2-phenylethyl)thiourea;
1-(3,4-dihydroxybenzyl)-3-phenylthiourea;
1-(3,4-dihydroxybenzyl)-1-isobutyl-3-(2-phenylethyl)thiourea;
1-((S)-2-hydroxy-1-phenylethyl)-3-(2-phenylethyl)thiourea;
1-benzyl-3-[(R)-1-(hydroxymethyl)-2-phenylethyl]thiourea;
1-benzyl-3-(2-hydroxy-2-phenylethyl)thiourea;
1-benzyl-3-[(S)-1-(hydroxymethyl)-2-phenylethyl]thiourea;
1-[2-(2-hydroxyphenyl)ethyl]-3-benzylthiourea;
1-[2-(3-hydroxyphenyl)ethyl]-3-benzylthiourea;
1-benzyl-1-hydroxy-3-(2-phenylethyl)thiourea;
1-benzyl-3-((R)-2-hydroxy-2-phenylethyl)thiourea;
1-benzyl-3-((S)-2-hydroxy-2-phenylethyl)thiourea;
1-((S)-2-methoxy-1-phenylethyl)-3-(2-phenylethyl)thiourea;
1-benzyl-3-(2-methoxy-2-phenylethyl)thiourea;
1-(3-aminobenzyl)-3-(2-phenylethyl)thiourea;
1-{3-[(2-aminoacetyl)amino]benzyl}-3-(2-phenylethyl)thiourea;
1-[2-(2-hydroxyphenyl)ethyl]-3-((R)-1-phenylethyl)thiourea;
1-[3-(mesylamino)benzyl]-3-(2-phenylethyl)thiourea;
1-[2-(2-hydroxyphenyl)ethyl]-3-((R)-2,3-dihydro-1H-inden-1-yl)thiourea;
1-[2-(2-hydroxyphenyl)ethyl]-3-[(R)-1-(3-hydroxyphenyl)ethyl]thiourea;
1-(2-hydroxybenzyl)-4-((R)-1-phenylethyl)thiosemicarbazide;
1-(2,6-dihydroxyphenyl)-3-((R)-1-phenylethyl)thiourea;
2-{(E)-[4-((R)-1-phenylethyl)thiosemicarbazido]methylidene}benzoic acid;
1-[2-(2-hydroxyphenyl)ethyl]-3-((R)-1-phenylpropyl)thiourea;
1-[2-(2-hydroxyphenyl)ethyl]-3-[(R)-1-(3,4-dimethoxyphenyl)ethyl]thiourea;
(S)-2-[3-(2-phenylethyl)thioureido]-2-phenylacetic acid ;
(S)-2-{3-[2-(2-hydroxyphenyl)ethyl]thioureido}-2-phenylacetic acid ;
1-[2-(2-hydroxyphenyl)ethyl]-3-[(R)-1-(3,4-dihydroxyphenyl)ethyl]thiourea;
1-[2-(2-hydroxyphenyl)-2-hydroxyethyl]-3-[(R)-1-(3-hydroxyphenyl)ethyl]thiourea;
2-{3-[(R)-1-(3-hydroxyphenyl)ethyl]thioureido}-3-(2-hydroxyphenyl)propanoic acid;
(R)-2-{3-[(R)-1-(3-hydroxyphenyl)ethyl]thioureido}-3-phenylpropanoic acid;
(S)-2-{3-[(R)-1-(3-hydroxyphenyl)ethyl]thioureido}-3-phenylpropanoic acid;
(R)-2-{3-[(R)-1-(3-hydroxyphenyl)ethyl]thioureido}-3-(4-hydroxyphenyl)propanoic acid;
(S)-2-{3-[(R)-1-(3-hydroxyphenyl)ethyl-]thioureido}-3-(4-hydroxyphenyl)propanoic acid;
1-[2-(2,3-dihydroxyphenyl)-2-hydroxyethyl]-3-[(R)-1-(3-hydroxyphenyl)ethyl]thiourea;
1-[2-(2,5-dihydroxyphenyl)-2-hydroxyethyl]-3-[(R)-1-(3-hydroxyphenyl)ethyl]thiourea;
1-[2-(2-hydroxy-5-methylphenyl)-2-hydroxyethyl]-3-[(R)-1-(3-hydroxyphenyl)ethyl]thiourea;
1-[2-(2,4-dihydroxyphenyl)-2-hydroxyethyl]-3-[(R)-1-(3-hydroxyphenyl)ethyl]thiourea;
1-[2-(5-chloro-2-hydroxyphenyl)-2-hydroxyethyl]-3-[(R)-1-(3-hydroxyphenyl)ethyl]thiourea;
1-[2-(5-bromo-2-hydroxyphenyl)-2-hydroxyethyl]-3-[(R)-1-(3-hydroxyphenyl)ethyl]thiourea;
2-[3-(4-chlorophenyl)thioureido]benzoic acid;
2-{3-[(R)-1-(3-hydroxyphenyl)ethyl]thioureido}benzoic acid;
5-hydroxy-2-[3-methyl-3-(2-phenylethyl)thioureido]benzoic acid;
methyl 2-{3-methyl-3-[2-(2-hydroxyphenyl)ethyl]thioureido}benzoate;
methyl 2-{3-methyl-3-[2-(2-hydroxyphenyl)ethyl]thioureido}-5-hydroxybenzoate;
2-{3-methyl-3-[2-(2-hydroxyphenyl)ethyl]thioureido}benzoic acid;
2-{3-methyl-3-[2-(2-hydroxyphenyl)ethyl]thioureido}-5-hydroxybenzoic acid; and
2-(2-{3-[(R)-1-(3-hydroxyphenyl)ethyl]thioureido}ethyl)benzoic acid;
or an edible salt thereof.


SEQUENCE LISTING

<110>  AJINOMOTO CO., INC.

<120>  Salty Taste Enhancer

<130>  091823

<150>  JP 2011-049469
<151>  2011-03-07

<160>  3

<170>  PatentIn version 3.3

<210>  1
<211>  2409
<212>  DNA
<213>  Homo sapiens

<400>  1
atgagggcag tgctgtcaca gaagacaaca ccgctccctc gttacctgtg gcccggccac      60
ctcagcggcc caaggaggct cacctggtca tggtgcagtg accacaggac ccccacatgc     120
cgggagctgg gttcgcccca ccccaccccc tgcaccgggc cagcgagggg atggcccaga     180
agagggggag gaccatgtgg attcaccagt gctggacatg tgctctgtgg ctaccccctc     240
tgcctactct ctggcccgat acaggggtgt gggacaggcc tgggtgactc cagcatggct     300
ttcctctcca ggacgtcacc ggtggcagct gcttccttcc agagccggca ggaggccaga     360
ggctccatcc tgcttcagag ctgccagctg cccccgcaat ggctgagcac cgaagcatgg     420
acgggagaat ggaagcagcc acacgggggg gctctcacct ccagatcgcc tgggcctgtg     480
gctccccaga ggccctgcca cctgaaggga tggcagcaca gacccactca gcacaacgct     540
gcctgcaaac agggccaggc tgcagcccag acgcccccca ggccggggcc accatcagca     600
ccaccaccac cacccaagga ggggcaccag gaggggctgg tggagctgcc cgcctcgttc     660
cgggagctgc tcaccttctt ctgcaccaat gccaccatcc acggcgccat ccgcctggtc     720
tgctcccgcg ggaaccgcct caagacgacg tcctgggggc tgctgtccct gggagccctg     780
gtcgcgctct gctggcagct ggggctcctc tttgagcgtc actggcaccg cccggtcctc     840
atggccgtct ctgtgcactc ggagcgcaag ctgctcccgc tggtcaccct gtgtgacggg     900
aacccacgtc ggccgagtcc ggtcctccgc catctggagc tgctggacga gtttgccagg     960
gagaacattg actccctgta caacgtcaac ctcagcaaag gcagagccgc cctctccgcc    1020
actgtccccc gccacgagcc ccccttccac ctggaccggg agatccgtct gcagaggctg    1080
agccactcgg gcagccgggt cagagtgggg ttcagactgt gcaacagcac gggcggcgac    1140
tgcttttacc gaggctacac gtcaggcgtg gcggctgtcc aggactggta ccacttccac    1200
tatgtggata tcctggccct gctgcccgcg gcatgggagg acagccacgg gagccaggac    1260
ggccacttcg tcctctcctg cagttacgat ggcctggact gccaggcccg acagttccgg    1320
accttccacc accccaccta cggcagctgc tacacggtcg atggcgtctg gacagctcag    1380
cgccccggca tcacccacgg agtcggcctg gtcctcaggg ttgagcagca gcctcacctc    1440
cctctgctgt ccacgctggc cggcatcagg gtcatggttc acggccgtaa ccacacgccc    1500
ttcctggggc accacagctt cagcgtccgg ccagggacgg aggccaccat cagcatccga    1560
gaggacgagg tgcaccggct cgggagcccc tacggccact gcaccgccgg cggggaaggc    1620
gtggaggtgg agctgctaca caacacctcc tacaccaggc aggcctgcct ggtgtcctgc    1680
ttccagcagc tgatggtgga gacctgctcc tgtggctact acctccaccc tctgccggcg    1740
ggggctgagt actgcagctc tgcccggcac cctgcctggg gacactgctt ctaccgcctc    1800
taccaggacc tggagaccca ccggctcccc tgtacctccc gctgccccag gccctgcagg    1860
gagtctgcat tcaagctctc cactgggacc tccaggtggc cttccgccaa gtcagctgga    1920
tggactctgg ccacgctagg tgaacagggg ctgccgcatc agagccacag acagaggagc    1980
agcctggcca aaatcaacat cgtctaccag gagctcaact accgctcagt ggaggaggcg    2040
cccgtgtact cggtgccgca gctgctctcg gccatgggca gcctctgcag cctgtggttt    2100
ggggcctccg tcctctccct cctggagctc ctggagctgc tgctcgatgc ttctgccctc    2160
accctggtgc taggcggccg ccggctccgc agggcgtggt tctcctggcc cagagccagc    2220
cctgcctcag gggcgtccag catcaagcca gaggccagtc agatgccccc gcctgcaggc    2280
ggcacgtcag atgacccgga gcccagcggg cctcatctcc cacgggtgat gcttccaggg    2340
gttctggcgg gagtctcagc cgaagagagc tgggctgggc cccagcccct tgagactctg    2400
gacacctga                                                           2409

<210> 2
<211> 1923
<212> DNA
<213> Homo sapiens

<400> 2
atgcacgtga agaagtacct gctgaagggc ctgcatcggc tgcagaaggg ccccggctac 60
acgtacaagg agctgctggt gtggtactgc gacaacacca acacccacgg ccccaagcgc 120
atcatctgtg aggggcccaa gaagaaagcc atgtggttcc tgctcaccct gctcttcgcc 180
gccctcgtct gctggcagtg gggcatcttc atcaggacct acttgagctg ggaggtcagc 240
gtctccctct ccgtaggctt caagaccatg gacttccctg ccgtcaccat ctgcaatgct 300
agccccttca agtattccaa aatcaagcat ttgctgaagg acctggatga gctgatggaa 360
gctgtcctgg agagaatcct ggctcctgag ctaagccatg ccaatgccac caggaacctg 420
aacttctcca tctggaacca cacacccctg gtccttattg atgaacggaa cccccaccac 480
cccatggtcc ttgatctctt tggagacaac cacaatggct taacaagcag ctcagcatca 540
gaaaagatct gtaatgccca cgggtgcaaa atggccatga gactatgtag cctcaacagg 600
acccagtgta ccttccggaa cttcaccagt gctacccagg cattgacaga gtggtacatc 660
ctgcaggcca ccaacatctt tgcacaggtg ccacagcagg agctagtaga gatgagctac 720
cccggcgagc agatgatcct ggcctgccta ttcggagctg agccctgcaa ctaccggaac 780
ttcacgtcca tcttctaccc tcactatggc aactgttaca tcttcaactg gggcatgaca 840
gagaaggcac ttccttcggc caaccctgga actgaattcg gcctgaagtt gatcctggac 900
ataggccagg aagactacgt ccccttcctt gcgtccacgg ccggggtcag gctgatgctt 960
cacgagcaga ggtcataccc cttcatcaga gatgagggca tctacgccat gtcggggaca 1020
gagacgtcca tcggggtact cgtggacaag cttcagcgca tgggggagcc ctacagcccg 1080
tgcaccgtga atggttctga ggtccccgtc caaaacttct acagtgacta caacacgacc 1140
tactccatcc aggcctgtct tcgctcctgc ttccaagacc acatgatccg taactgcaac 1200
tgtggccact acctgtaccc actgccccgt ggggagaaat actgcaacaa ccgggacttc 1260
ccagactggg cccattgcta ctcagatcta cagatgagcg tggcgcagag agagacctgc 1320
attggcatgt gcaaggagtc ctgcaatgac acccagtaca agatgaccat ctccatggct 1380
gactggcctt ctgaggcctc cgaggactgg attttccacg tcttgtctca ggagcgggac 1440
caaagcacca atatcaccct gagcaggaag ggaattgtca agctcaacat ctacttccaa 1500
gaatttaact atcgcaccat tgaagaatca gcagccaata acatcgtctg gctgctctcg 1560
aatctgggtg gccagtttgg cttctggatg gggggctctg tgctgtgcct catcgagttt 1620
ggggagatca tcatcgactt tgtgtggatc accatcatca agctggtggc cttggccaag 1680
agcctacggc agcggcgagc ccaagccagc tacgctggcc caccgcccac cgtggccgag 1740
ctggtggagg cccacaccaa ctttggcttc cagcctgaca cggccccccg cagccccaac 1800
actgggccct accccagtga gcaggccctg cccatcccag gcaccccgcc ccccaactat 1860
gactccctgc gtctgcagcc gctggacgtc atcgagtctg acagtgaggg tgatgccatc 1920
taa 1923

<210> 3
<211> 1950
<212> DNA
<213> Homo sapiens

<400> 3
atggcacccg gagagaagat caaagccaaa atcaagaaga atctgcccgt gacgggccct 60
caggcgccga ccattaaaga gctgatgcgg tggtactgcc tcaacaccaa cacccatggc 120
tgtcgccgca tcgtggtgtc ccgcggccgt ctgcgccgcc tcctctggat cgggttcaca 180
ctgactgccg tggccctcat cctctggcag tgcgccctcc tcgtcttctc cttctatact 240
gtctcagttt ccatcaaagt ccacttccgg aagctggatt ttcctgcagt caccatctgc 300
aacatcaacc cctacaagta cagcaccgtt cgccaccttc tagctgactt ggaacaggag 360
accagagagg ccctgaagtc cctgtatggc tttccagagt cccggaagcg ccgagaggcg 420
gagtcctgga actccgtctc agagggaaag cagcctagat tctcccaccg gattccgctg 480
ctgatctttg atcaggatga gaagggcaag gccagggact tcttcacagg gaggaagcgg 540
aaagtcggcg gtagcatcat tcacaaggct tcaaatgtca tgcacatcga gtccaagcaa 600
gtggtgggat tccaactgtg ctcaaatgac acctccgact gtgccaccta caccttcagc 660
tcgggaatca atgccattca ggagtggtat aagctacact acatgaacat catggcacag 720
gtgcctctgg agaagaaaat caacatgagc tattctgctg aggagctgct ggtgacctgc 780
ttctttgatg gagtgtcctg tgatgccagg aatttcacgc ttttccacca cccgatgcat 840
gggaattgct atactttcaa caacagagaa aatgagacca ttctcagcac ctccatgggg 900

ggcagcgaat atgggctgca agtcattttg tacataaacg aagaggaata caacccattc 960
ctcgtgtcct ccactggagc taaggtgatc atccatcggc aggatgagta tcccttcgtc 1020
gaagatgtgg gaacagagat tgagacagca atggtcacct ctataggaat gcacctgaca 1080
gagtccttca agctgagtga gccctacagt cagtgcacgg aggacgggag tgacgtgcca 1140
atcaggaaca tctacaacgc tgcctactcg ctccagatct gccttcattc atgcttccag 1200
acaaagatgg tggagaaatg tgggtgtgcc cagtacagcc agcctctacc tcctgcagcc 1260
aactactgca actaccagca gcaccccaac tggatgtatt gttactacca actgcatcga 1320
gcctttgtcc aggaagagct gggctgccag tctgtgtgca aggaagcctg cagctttaaa 1380
gagtggacac taaccacaag cctggcacaa tggccatctg tggtttcgga gaagtggttg 1440
ctgcctgttc tcacttggga ccaaggccgg caagtaaaca aaaagctcaa caagacagac 1500
ttggccaaac tcttgatatt ctacaaagac ctgaaccaga gatccatcat ggagagccca 1560
gccaacagta ttgagatgct tctgtccaac ttcggtggcc agctgggcct gtggatgagc 1620
tgctctgttg tctgcgtcat cgagatcatc gaggtcttct tcattgactt cttctctatc 1680
attgcccgcc gccagtggca gaaagccaag gagtggtggg cctggaaaca ggctccccca 1740
tgtccagaag ctccccgtag cccacagggc caggacaatc cagccctgga tatagacgat 1800
gacctaccca ctttcaactc tgctttgcac ctgcctccag ccctaggaac ccaagtgccc 1860
ggcacaccgc cccccaaata caataccttg cgcttggaga gggccttttc caaccagctc 1920
acagataccc agatgctgga tgagctctga 1950